# EUROPEAN PATENT SPECIFICATION

(11) **EP 4 431 509 B1**
(45) Date of publication and mention of the grant of the patent: **21.01.2026**
(21) Application number: 22900556.6
(22) Date of filing: 30.11.2022
(51) Int. Cl.: C07D 471/04, C07D 497/14, A61K 31/55, A61P 31/04

(54) **NOVEL NON-FLUORINATED QUINOLONE COMPOUND AND APPLICATION THEREOF**
NEUE NICHTFLUORIERTE CHINOLONVERBINDUNG UND ANWENDUNG DAVON
NOUVEAU COMPOSÉ DE QUINOLONE NON FLUORÉE ET SON APPLICATION

(30) Priority: 30.11.2021 CN 202111444560; 21.07.2022 CN 202210864782
(43) Date of publication of application: 18.09.2024
(73) Proprietor: Guangzhou Baiyunshan Pharmaceutical Holdings Co., Ltd. Baiyunshan Pharmaceutical General Factory, Guangzhou, Guangdong 510515 (CN)
(72) Inventor: WANG, Jiansong, Guangzhou, Guangdong 510515 (CN); TANG, Dongdong, Shanghai 200131 (CN); LUO, Zhibo, Guangzhou, Guangdong 510515 (CN); HUANG, Zhigang, Shanghai 200131 (CN); HUANG, Haiwen, Guangzhou, Guangdong 510515 (CN); DING, Charles Z., Shanghai 200131 (CN); CHEN, Shuhui, Shanghai 200131 (CN)
(74) Representative: Nex & Phister Law & IP Aps
(86) International application number: PCT/CN2022/135584
(87) International publication number: WO 2023/098752

(56) References cited:
- WO-A1-2011/031740
- WO-A1-2011/031743
- WO-A1-2011/031744
- WO-A2-2009/137130
- CN-A- 101 497 612
- CN-A- 102 603 731
- SINGH NEHA ET AL: "Current Insights for the Management of Acne in the Modern Era", vol. 15, no. 1, 26 November 2020 (2020-11-26), NL, pages 3 - 29, XP093245828, ISSN: 1574-891X, Retrieved from the Internet <URL:https://eurekaselect.com/article/download/184345> DOI: 10.2174/1574891X15999200729192138

## Description

The present disclosure claims priorities to the following:
CN202111444560.2, November 30, 2021;
CN202210864782.8, July 21, 2022.

### TECHNICAL FIELD

The present disclosure relates to a novel non-fluorinated quinolone compound and use thereof, and in particular to a compound represented by formula (II) and a pharmaceutically acceptable salt thereof.

### BACKGROUND

Quinolone drugs were emerged in the 1960s, they have been continuously updated and improved after decades of development and are widely used in clinical practice. Due to the advantages such as broad antimicrobial spectrum, strong antibacterial activities, convenient administration, and no cross-resistance with other commonly used antibacterial drugs, quinolone drugs have rapidly popularized in clinical applications and become one of the commonly used priority drugs in the world nowadays. With the continuous deepening of modification and research on such drugs, it has been found that they also have many other biological activities, such as anti-tumor, antiviral, and anti-inflammatory effects, etc., which have been reported.

Acne is a skin disorder that affects over 85% of humans. Acne usually occurs on the face, neck, and upper trunk. Although the occurrence of acne is caused by multiple factors, symbiotic skin bacteria (such as *Propionibacterium acnes*) play a major role in the formation of acne lesions. It is an infection of sebaceous follicles, and oil glands in the skin. Meanwhile, *Propionibacterium acnes* can also activate Toll-like receptor 2 (TLR2) on innate immunocytes, the activization of TLR can trigger the expressions of various cytokines such as IL-6, IL-8 and IL-12, as well as chemokines that stimulate the recruitment of other host immune cells.

The most widely used antibiotics for treating acne are erythromycin and clindamycin, due to their long-term and extensive use, drug resistance is becoming increasingly severe, and there is an urgent need for new antibiotics for the treatment of acne. In recent years, multiple patents and articles have reported that quinolone drugs have good *propionibacterium acnes* resistant activity, as well as anti-inflammatory activity. Therefore, research and development of a new generation of quinolone drugs for the treatment of acne has important theoretical and economic values.

### SUMMARY

The present disclosure provides a compound represented by formula (II), a stereoisomer, or a pharmaceutically acceptable salt thereof, 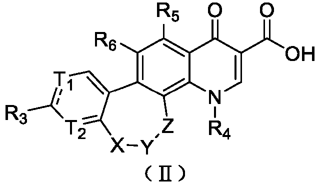 wherein, 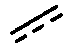
is selected from the group consisting of a single bond and a double bond;
T₁ is selected from the group consisting of N, NH, CR₁ and N⁺R₁(A⁻);
T₂ is selected from the group consisting of N and CR₂;
A⁻ is selected from the group consisting of F⁻, Cl⁻, Br⁻, I⁻, OH⁻ and HCO₃⁻;
X is selected from the group consisting of -C(R₇R₈)- and -C(R₇R₈)-C(R₇R₈)-;
Y is selected from the group consisting of -O-, -S-, -NH-, and -C(R₇R₈)-;
Z is -C(R₇R₈)-;
alternatively, -Y-Z- is -C(R₉)=C(R₉)-;
R₁ is selected from the group consisting of H, F, Cl, Br, I, -OH, -NH₂, -CN and -CH₃;
R₂ is selected from the group consisting of H, F, Cl, Br, I, -OH, -NH₂, -CN and C₁₋₃ alkyl, wherein the C₁₋₃ alkyl is optionally substituted by 1, 2 or 3 Rₐ;
R₃ is selected from the group consisting of H, F, Cl, Br, I, =O, -OH, -NH₂, -CN, -NHC(=NH)NH₂, C₁₋₃ alkyl, C₁₋₃ alkylamino and C₁₋₃ alkoxy, wherein the C₁₋₃ alkyl, C₁₋₃ alkylamino and C₁₋₃ alkoxy is independently optionally substituted by 1, 2 or 3 R_{b}, respectively;
alternatively, R₁ and R₃ together with the atoms to which they are attached form 5-6 membered heterocycloalkyl, 5-6 membered heterocycloalkenyl, or 5-6 membered heteroaryl, wherein the 5-6 membered heterocycloalkyl, 5-6 membered heterocycloalkenyl and 5-6 membered heteroaryl are independently optionally substituted by 1, 2, 3 or 4 R_{c}, respectively;
R₄ is selected from the group consisting of H, C₁₋₃ alkyl, C₃₋₆ cycloalkyl, phenyl and 5-6 membered heteroaryl, wherein the C₁₋₃ alkyl, C₃₋₆ cycloalkyl, phenyl and 5-6 membered heteroaryl is independently optionally substituted by 1, 2, 3 or 4 R_{d}, respectively;
R₅ is selected from the group consisting of H, F, Cl, Br, I, -OH, -NH₂ and -CN;
R₆ is selected from the group consisting of H, F, Cl, Br, I, -OH, -NH₂ and -CN;
R₇, R₈ and R₉ are each independently selected from the group consisting of H, F, Cl, Br, I, -OH, -NH₂ and -CN, respectively;
each Rₐ is independently selected from the group consisting of F, Cl, Br, I, -OH, -NH₂ and -CN, respectively;
each R_{b} is independently selected from the group consisting of F, Cl, Br, I, -OH, -NH₂, -CN, C₁₋₃ alkoxy, C₃₋₆ cycloalkyl, 3-6 membered heterocycloalkyl, 5-6 membered heteroaryl and phenyl, respectively, wherein the C₁₋₃ alkoxy, C₃₋₆ cycloalkyl, 3-6 membered heterocycloalkyl, 5-6 membered heteroaryl and phenyl are independently optionally substituted by 1, 2, 3 or 4 R, respectively;
each R_{c} is independently selected from the group consisting of F, Cl, Br, I, -OH, -NH₂, -CN and -CH₃, respectively;
each R_{d} is independently selected from the group consisting of F, Cl, Br, I, -OH, -NH₂ and -CN, respectively;
each R is independently selected from the group consisting of F, Cl, Br, I, -OH, -NH₂, -NO₂, -CN, - ONHC(=NH)NH₂ and C₁₋₃ alkyl, respectively; and
the "hetero" in the 5-6 membered heterocycloalkyl, 3-6 membered heterocycloalkyl, 5-6 membered heterocycloalkenyl and 5-6 membered heteroaryl means 1, 2, 3 or 4 heteroatoms or heteroatom groups independently selected from the group consisting of O, NH, S and N, respectively.

The present disclosure provides a compound represented by formula (II), a stereoisomer, or a pharmaceutically acceptable salt thereof, 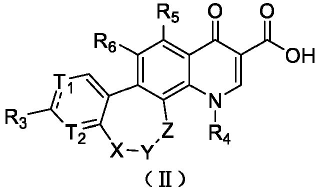 wherein, 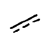
is selected from the group consisting of a single bond and a double bond;
T₁ is selected from the group consisting of N and CR₁;
T₂ is selected from the group consisting of N and CR₂;
X is selected from the group consisting of -C(R₇R₈)- and -C(R₇R₈)-C(R₇R₈)-;
Y is selected from the group consisting of -O-, -S-, -NH-, and -C(R₇R₈)-;
Z is -C(R₇R₈)-;
alternatively, -Y-Z- is -C(R₉)=C(R₉)-;
R₁ is selected from the group consisting of H, F, Cl, Br, I, -OH, -NH₂ and -CN;
R₂ is selected from the group consisting of H, F, Cl, Br, I, -OH, -NH₂, -CN and C₁₋₃ alkyl, wherein the C₁₋₃ alkyl is optionally substituted by 1, 2 or 3 Rₐ;
R₃ is selected from the group consisting of H, F, Cl, Br, I, =O, -OH, -NH₂, -CN, C₁₋₃ alkyl, C₁₋₃ alkylamino and C₁₋₃ alkoxy, wherein the C₁₋₃ alkyl, C₁₋₃ alkylamino and C₁₋₃ alkoxy are independently optionally substituted by 1, 2 or 3 R_{b}, respectively;
alternatively, R₁ and R₃ together with the atoms to which they are attached form 5-6 membered heterocycloalkyl, 5-6 membered heterocycloalkenyl, or 5-6 membered heteroaryl, wherein the 5-6 membered heterocycloalkyl, 5-6 membered heterocycloalkenyl and 5-6 membered heteroaryl are independently optionally substituted by 1, 2, 3 or 4 R_{c}, respectively;
R₄ is selected from the group consisting of H, C₁₋₃ alkyl, C₃₋₆ cycloalkyl, phenyl and 5-6 membered heteroaryl, wherein the C₁₋₃ alkyl, C₃₋₆ cycloalkyl, phenyl and 5-6 membered heteroaryl is independently optionally substituted by 1, 2, 3 or 4 R_{d}, respectively;
R₅ is selected from the group consisting of H, F, Cl, Br, I, -OH, -NH₂ and -CN;
R₆ is selected from the group consisting of H, F, Cl, Br, I, -OH, -NH₂ and -CN;
R₇, R₈ and R₉ are each independently selected from the group consisting of H, F, Cl, Br, I, -OH, -NH₂ and -CN, respectively;
Rₐ is independently selected from the group consisting of F, Cl, Br, I, -OH, -NH₂ and -CN, respectively;
R_{b} is independently selected from the group consisting of F, Cl, Br, I, -OH, -NH₂, -CN, C₃₋₆ cycloalkyl, 3-6 membered heterocycloalkyl and phenyl, respectively, wherein the C₃₋₆ cycloalkyl, 3-6 membered heterocycloalkyl and phenyl are independently optionally substituted by 1, 2, 3 or 4 R, respectively;
R_{c} is independently selected from the group consisting of F, Cl, Br, I, -OH, -NH₂ and -CN, respectively;
R_{d} is independently selected from the group consisting of F, Cl, Br, I, -OH, -NH₂ and -CN, respectively;
R is independently selected from the group consisting of F, Cl, Br, I, -OH, -NH₂ and -CN, respectively; and
the "hetero" in the 5-6 membered heterocycloalkyl, 3-6 membered heterocycloalkyl, 5-6 membered heterocycloalkenyl and 5-6 membered heteroaryl means 1, 2, 3 or 4 heteroatoms or heteroatom groups independently selected from the group consisting of -O-, -NH-, -S- and -N-, respectively.

The present disclosure provides a compound represented by formula (I), a stereoisomer, or a pharmaceutically acceptable salt thereof, 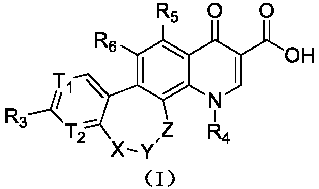 wherein,
T₁ is selected from the group consisting of N and CR₁;
T₂ is selected from the group consisting of N and CR₂;
X is selected from the group consisting of -C(R₇R₈)- and -C(R₇R₈)-C(R₇R₈)-;
Y is selected from the group consisting of -O- and -C(R₇R₈)-;
Z is -C(R₇R₈)-;
alternatively, -Y-Z- is -C(R₉)=C(R₉)-;
R₁ is selected from the group consisting of H, F, Cl, Br, I, -OH, -NH₂ and -CN;
R₂ is selected from the group consisting of H, F, Cl, Br, I, -OH, -NH₂, -CN and C₁₋₃ alkyl, wherein the C₁₋₃ alkyl is optionally substituted by 1, 2 or 3 Rₐ;
R₃ is selected from the group consisting of H, F, Cl, Br, I, -OH, -NH₂, -CN, C₁₋₃ alkylamino and C₁₋₃ alkoxy, wherein the C₁₋₃ alkylamino and C₁₋₃ alkoxy are independently optionally substituted by 1, 2 or 3 R_{b}, respectively;
alternatively, R₁ and R₃ together with the atoms to which they are attached form 5-6 membered heterocycloalkyl, 5-6 membered heterocycloalkenyl and 5-6 membered heteroaryl, wherein the 5-6 membered heterocycloalkenyl and 5-6 membered heteroaryl are independently optionally substituted by 1, 2, 3 or 4 R_{c}, respectively;
R₄ is selected from the group consisting of H, C₁₋₃ alkyl, C₃₋₆ cycloalkyl, phenyl and 5-6 membered heteroaryl, wherein the C₁₋₃ alkyl, C₃₋₆ cycloalkyl, phenyl and 5-6 membered heteroaryl are independently optionally substituted by 1, 2, 3 or 4 R_{d}, respectively;
R₅ is selected from the group consisting of H, F, Cl, Br, I, -OH, -NH₂ and -CN;
R₆ is selected from the group consisting of H, F, Cl, Br, I, -OH, -NH₂ and -CN;
R₇, R₈ and R₉ are each independently selected from the group consisting of H, F, Cl, Br, I, -OH, -NH₂ and -CN, respectively;
Rₐ is independently selected from the group consisting of F, Cl, Br, I, -OH, -NH₂ and -CN, respectively;
R_{b} is independently selected from the group consisting of F, Cl, Br, I, -OH, -NH₂ and -CN, respectively;
R_{c} is independently selected from the group consisting of F, Cl, Br, I, -OH, -NH₂ and -CN, respectively;
R_{d} is independently selected from the group consisting of F, Cl, Br, I, -OH, -NH₂ and -CN, respectively; and
the "hetero" in the 5-6 membered heterocycloalkyl, 5-6 membered heterocycloalkenyl and 5-6 membered heteroaryl means 1, 2, 3 or 4 heteroatoms or heteroatom groups independently selected from the group consisting of -O-, -NH-, -S- and -N-, respectively.

In some embodiments of the present disclosure, the above 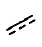 is a double bond, and other variables are as defined in the present disclosure.

In some embodiments of the present disclosure, the above T₁ is N, and other variables are as defined in the present disclosure.

In some embodiments of the present disclosure, the above T₁ is s CH, and other variables are as defined in the present disclosure.

In some embodiments of the present disclosure, the above T₁ is NH, and other variables are as defined in the present disclosure.

In some embodiments of the present disclosure, the above T₁ is N⁺R₁(A⁻), and other variables are as defined in the present disclosure.

In some embodiments of the present disclosure, the above T₁ is N⁺R₁(Cl⁻), and other variables are as defined in the present disclosure.

In some embodiments of the present disclosure, the above T₂ is N, and other variables are as defined in the present disclosure.

In some embodiments of the present disclosure, the above T₂ is CH, and other variables are as defined in the present disclosure.

In some embodiments of the present disclosure, the above A⁻ is selected from the group consisting of F⁻, Cl⁻, Br⁻and I⁻, and other variables are as defined in the present disclosure.

In some embodiments of the present disclosure, the above A⁻ is Cl⁻, and other variables are as defined in the present disclosure.

In some embodiments of the present disclosure, the above X is -CH₂-, and other variables are as defined in the present disclosure.

In some embodiments of the present disclosure, the above Y is -O-, and other variables are as defined in the present disclosure.

In some embodiments of the present disclosure, the above Y is -CH₂-, and other variables are as defined in the present disclosure.

In some embodiments of the present disclosure, the above Z is -CH₂-, and other variables are as defined in the present disclosure.

In some embodiments of the present disclosure, the above -Y-Z- is -CH=CH-, and other variables are as defined in the present disclosure.

In some embodiments of the present disclosure, the above R is each independently selected from the group consisting of -NO₂, -ONHC(=NH)NH₂ and -CH₃, respectively, and other variables are as defined in the present disclosure.

In some embodiments of the present disclosure, the above R_{b} is each independently selected from the group consisting of F, Cl, Br, -OH, -NH₂, -OCH₂CH₃, cyclobutyl, oxetanyl, oxacyclopentyl, azetidinyl, 5-membered heteroaryl and phenyl, respectively, wherein the -OCH₂CH₃, cyclobutyl, oxetanyl, oxacyclopentyl, azetidinyl, 5-membered heteroaryl and phenyl are independently optionally substituted by 1, 2, 3 or 4 R, respectively, and R and the other variables are as defined in the present disclosure.

In some embodiments of the present disclosure, the above R_{b} is each independently selected from the group consisting of F, Cl, Br, -OH, -NH₂, cyclobutyl, oxetanyl, azetidinyl and phenyl, respectively, wherein the cyclobutyl, oxetanyl, azetidinyl and phenyl are independently optionally substituted by 1, 2, 3 or 4 R, respectively, and R and the other variables are as defined in the present disclosure.

In some embodiments of the present disclosure, the above R_{b} is each independently selected from the group consisting of F, Cl, Br, -OH, -NH₂, -OCH₂CH₃, cyclobutyl, oxetanyl, 1,3-dioxolanyl, azetidinyl, imidazolyl and phenyl, respectively, wherein the -OCH₂CH₃, cyclobutyl, oxetanyl, 1,3-dioxolanyl, azetidinyl, imidazolyl and phenyl are independently optionally substituted by 1, 2, 3 or 4 R, respectively, and R and the other variables are as defined in the present disclosure.

In some embodiments of the present disclosure, the above R_{b} is each independently selected from the group consisting of F, Cl, Br, -OH, -NH₂, -OCH₂CH₃, 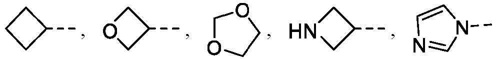 and 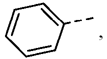 respectively, wherein the -OCH₂CH₃, 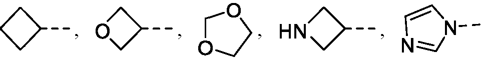 and 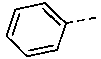 are independently optionally substituted by 1, 2, 3 or 4 R, respectively, and R and the other variables are as defined in the present disclosure.

In some embodiments of the present disclosure, the above R_{b} is each independently selected from the group consisting of -OH, -NH₂, 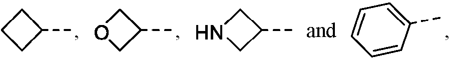 respectively, wherein the 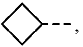 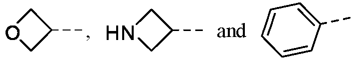 are independently optionally substituted by 1, 2, 3 or 4 R, respectively, and R and the other variables are as defined in the present disclosure.

In some embodiments of the present disclosure, the above R_{b} is each independently selected from the group consisting of F, -OH, -NH₂, 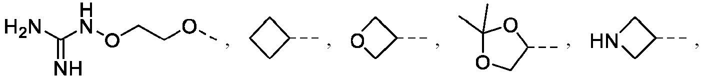 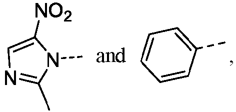 respectively, and other variables are as defined in the present disclosure.

In some embodiments of the present disclosure, the above R_{b} is each independently selected from the group consisting of -OH, -NH₂, 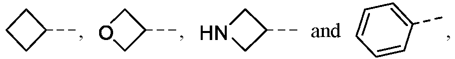 respectively, and other variables are as defined in the present disclosure.

In some embodiments of the present disclosure, the above R_{b} is each independently selected from the group consisting of F, Cl, Br and -OH, respectively, and other variables are as defined in the present disclosure.

In some embodiments of the present disclosure, the above R_{c} is each independently selected from -CH₃, respectively, and other variables are as defined in the present disclosure.

In some embodiments of the present disclosure, the above R_{d} is each independently selected from the group consisting of F, Cl, Br and -NH₂, respectively, and other variables are as defined in the present disclosure.

In some embodiments of the present disclosure, the above R_{d} is each independently selected from the group consisting of F and -NH₂, respectively, and other variables are as defined in the present disclosure.

In some embodiments of the present disclosure, the above R₁ is H, and other variables are as defined in the present disclosure.

In some embodiments of the present disclosure, the above R₂ is selected from the group consisting of H and - CH₃, and other variables are as defined in the present disclosure.

In some embodiments of the present disclosure, the above R₂ is H, and other variables are as defined in the present disclosure.

In some embodiments of the present disclosure, the above R₃ is selected from the group consisting of H, F, Cl, Br, =O, -OH, -NH₂, -CN, -NHC(=NH)NH₂, -CH₃, -NHCH₃, -N(CH₃)₂, -NHCH₂CH₃, -NHCH₂CH₂CH₃, - N(CH₃)CH₂CH₃ and -OCH₃, wherein the -NHC(=NH)NH₂, -CH₃, -NHCH₃, -N(CH₃)₂, -NHCH₂CH₃, - NHCH₂CH₂CH₃, -N(CH₃)CH₂CH₃ and -OCH₃ are independently optionally substituted by 1, 2, or 3 R_{b}, respectively, and R_{b} and other variables are as defined in the present disclosure.

In some embodiments of the present disclosure, the above R₃ is selected from the group consisting of H, F, Cl, Br, =O, -OH, -NH₂, -CN, -CH₃, -NHCH₃, -N(CH₃)₂, -NHCH₂CH₃, -NHCH₂CH₂CH₃ and -OCH₃, wherein the -CH₃, - NHCH₃, -N(CH₃)₂, -NHCH₂CH₃, -NHCH₂CH₂CH₃ and -OCH₃ are independently optionally substituted by 1, 2, or 3 R_{b}, respectively, and R_{b} and other variables are as defined in the present disclosure.

In some embodiments of the present disclosure, the above R₃ is selected from the group consisting of H, F, Cl, Br, -OH, -NH₂, -CN, -NHCH₃, -N(CH₃)₂, -NCH₂CH₃ and -OCH₃, wherein the NHCH₃, -N(CH₃)₂, -NCH₂CH₃ and - OCH₃ are independently optionally substituted by 1, 2, or 3 R_{b}, respectively, and R_{b} and other variables are as defined in the present disclosure.

In some embodiments of the present disclosure, the above R₃ is selected from the group consisting of H, =O, - NH₂, -NHC(=NH)NH₂, -CH₃, -NHCH₃, -N(CH₃)₂, -NCH₂CH₃, -NHCH₂CH₂CH₃, -NHCH₂CH₂OH, -NHCH₂CH₂NH₂, -OCH₃, 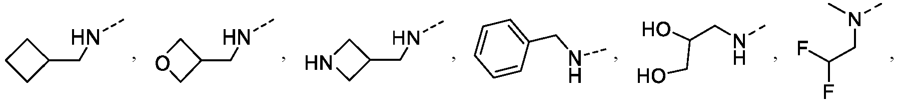 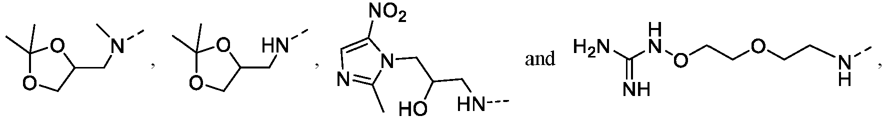 and other variables are as defined in the present disclosure.

In some embodiments of the present disclosure, the above R₃ is selected from the group consisting of H, =O, - NH₂, -CH₃, -NHCH₃, -N(CH₃)₂, -NCH₂CH₃, -NHCH₂CH₂CH₃, -NHCH₂CH₂OH, -NHCH₂CH₂NH₂, -OCH₃, 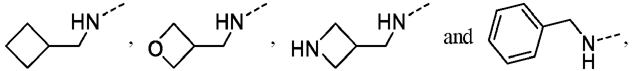 and other variables are as defined in the present disclosure.

In some embodiments of the present disclosure, the above R₃ is selected from the group consisting of -NH₂, - NHCH₃, -N(CH₃)₂, -NCH₂CH₃, -NCH₂CH₂OH and -OCH₃, and other variables are as defined in the present disclosure.

In some embodiments of the present disclosure, the above R₄ is selected from the group consisting of C₃₋₆ cycloalkyl, wherein the C₃₋₆ cycloalkyl is optionally substituted by 1, 2, 3 or 4 R_{d}, and other variables are as defined in the present disclosure.

In some embodiments of the present disclosure, the above R₄ is selected from the group consisting of H, -CH₂CH₃, cyclopropyl, phenyl and pyridyl, wherein the -CH₂CH₃, cyclopropyl, phenyl and pyridyl are independently optionally substituted by 1, 2, 3 or 4 R_{d}, respectively, and other variables are as defined in the present disclosure.

In some embodiments of the present disclosure, the above R₄ is selected from the group consisting of H, - CH₂CH₂F,

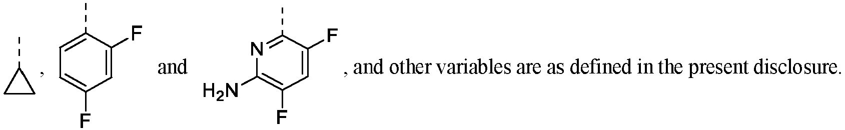

In some embodiments of the present disclosure, the above R₄ is 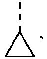 and other variables are as defined in the present disclosure.

In some embodiments of the present disclosure, the above R₅ and R₆ are independently selected from the group consisting of H and F, respectively, and other variables are as defined in the present disclosure.

In some embodiments of the present disclosure, the above R₆ is selected from the group consisting of H and F, and other variables are as defined in the present disclosure.

In some embodiments of the present disclosure, the above R₅ and R₆ are independently H, respectively, and other variables are as defined in the present disclosure.

In some embodiments of the present disclosure, the above R₇, R₈ and R₉ are each independently H, respectively, and other variables are as defined in the present disclosure.

In some embodiments of the present disclosure, the above R₁ and R₃ together with the atoms to which they are attached form a 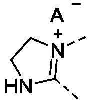 quaternary ammonium salt ring, wherein the 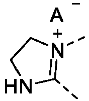 is independently optionally substituted by 1, 2, 3 or 4 R_{c}, respectively, and R_{c} and other variables are as defined in the present disclosure.

In some embodiments of the present disclosure, the above R₁ and R₃ together with the atoms to which they are attached form a 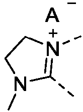 quaternary ammonium salt ring, and other variables are as defined in the present disclosure.

In some embodiments of the present disclosure, the above R₁ and R₃ together with the atoms to which they are attached form a 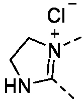 quaternary ammonium salt ring, wherein the 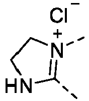 is independently optionally substituted by 1, 2, 3 or 4 R_{c}, respectively, and R_{c} and other variables are as defined in the present disclosure.

In some embodiments of the present disclosure, the above R₁ and R₃ together with the atoms to which they are attached form a 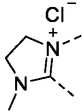 quaternary ammonium salt ring, and other variables are as defined in the present disclosure.

In some embodiments of the present disclosure, the above R₁ and R₃ together with the atoms to which they are attached form 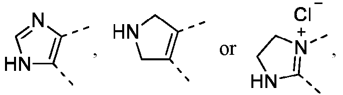 wherein the 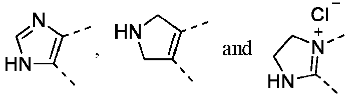 are independently optionally substituted by 1, 2, 3 or 4 R_{c}, respectively, and R_{c} and other variables are as defined in the present disclosure.

In some embodiments of the present disclosure, the above R₁ and R₃ together with the atoms to which they are attached form 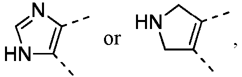 wherein the 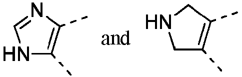 are independently optionally substituted by 1, 2, 3 or 4 R_{c}, respectively, and R_{c} and other variables are as defined in the present disclosure.

In some embodiments of the present disclosure, the above R₁ and R₃ together with the atoms to which they are attached form 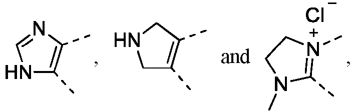 and other variables are as defined in the present disclosure.

In some embodiments of the present disclosure, the above R₁ and R₃ together with the atoms to which they are attached form 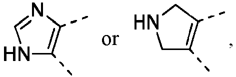 and other variables are as defined in the present disclosure.

In some embodiments of the present disclosure, the above compound has a structure represented by formula (II-1): 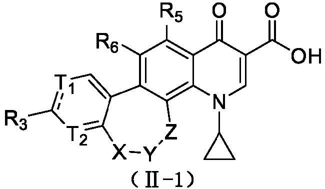 wherein, 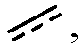 X, Y, Z, T₁, T₂, R₃, R₅ and R₆ are as defined in the present disclosure.

In some embodiments of the present disclosure, the above compound has a structure represented by formula (II-2): 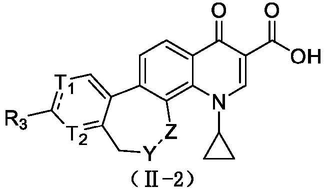 wherein, 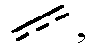 Y, Z, T₁, T₂ and R₃ are as defined in the present disclosure.

In some embodiments of the present disclosure, the above compounds have the structures represented by formulas (II-3), (II-4), (II-5) and (II-6): 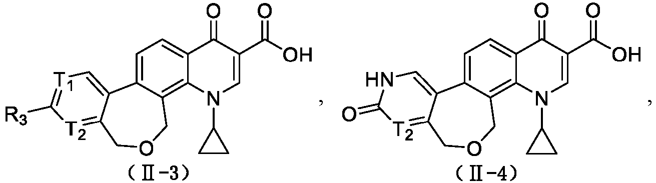 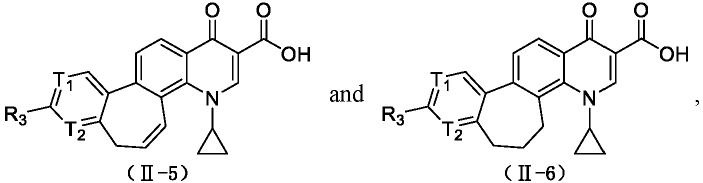 wherein, T₁, T₂ and R₃ are as defined in the present disclosure.

In some embodiments of the present disclosure, the above compounds have the structures represented by formulas (II-3A), (II-3B), (II-3C), (II-3D), (II-3E), (II-5A) and (II-6A): 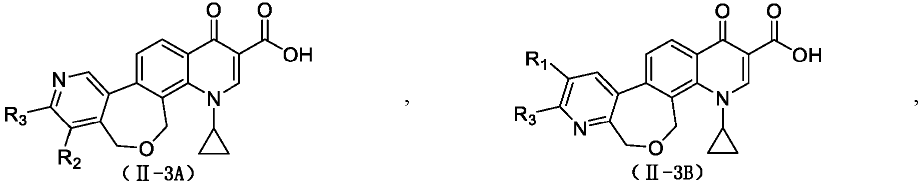 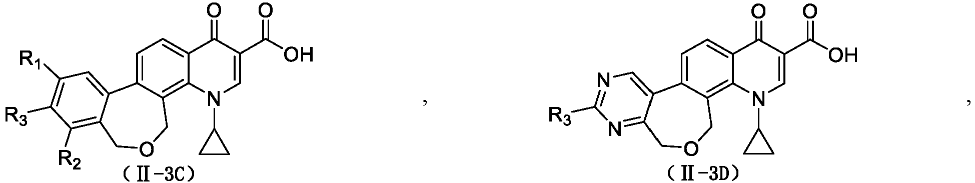 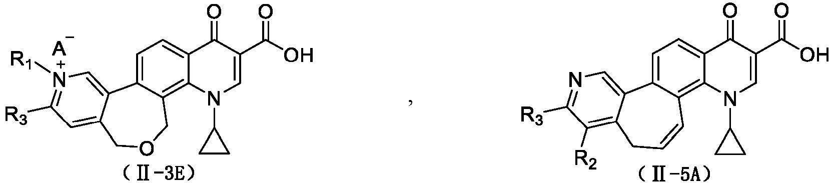 and 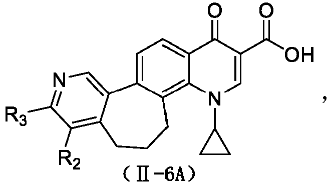 wherein, A⁻, R₁, R₂ and R₃ are as defined in the present disclosure.

Some embodiments of the present disclosure are derived from any combinations of the aforementioned variables.

The present disclosure further provides compounds represented by the following formulas, stereoisomers, or pharmaceutically acceptable salts thereof,

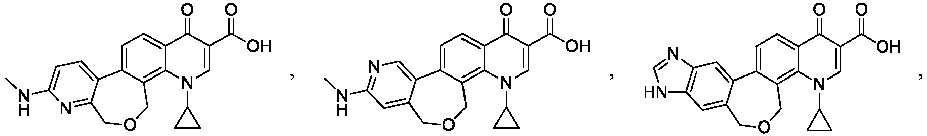

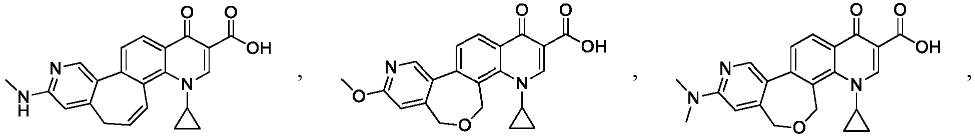

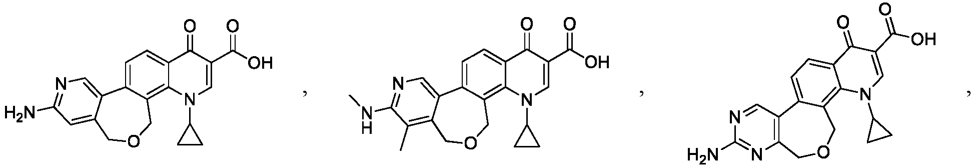

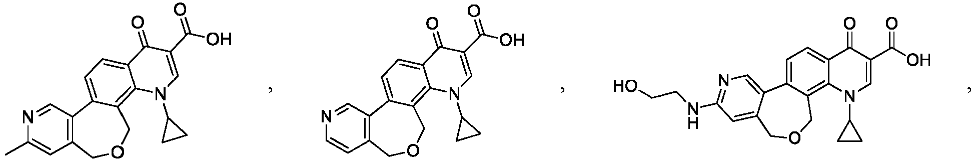

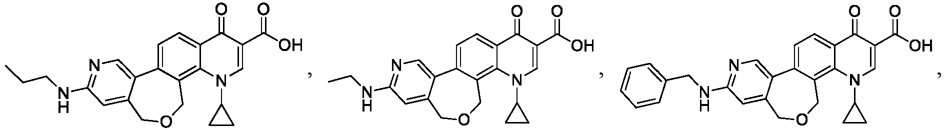

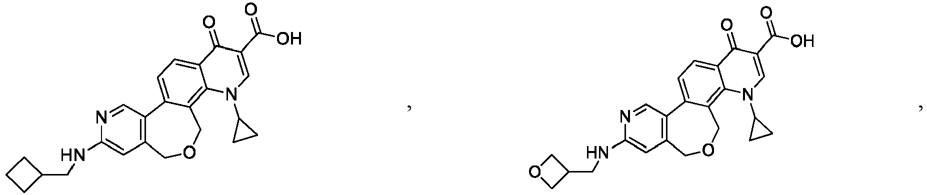

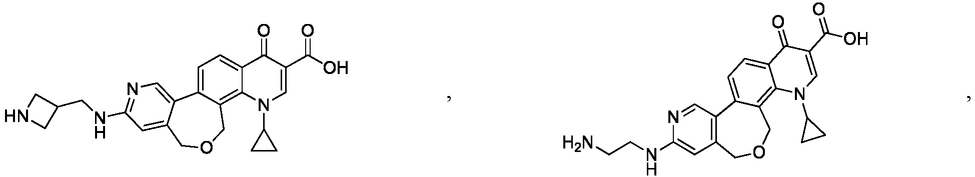

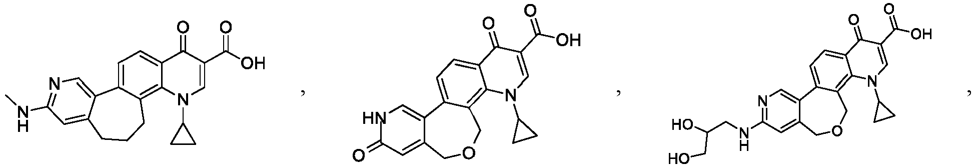

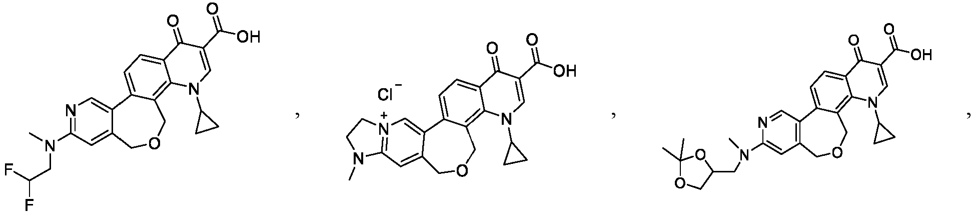

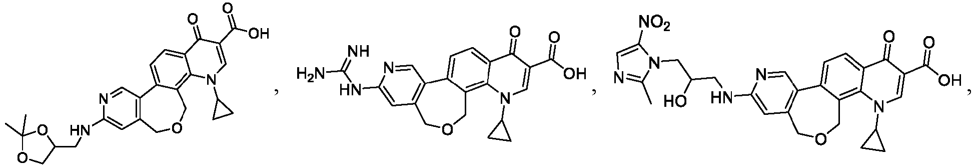

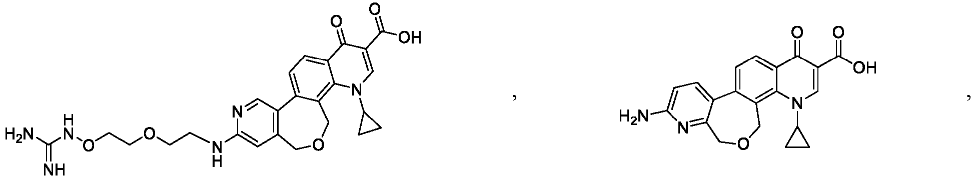

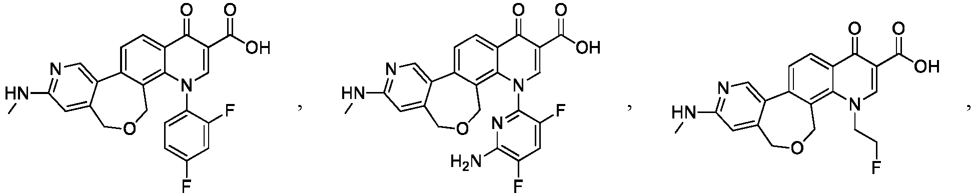

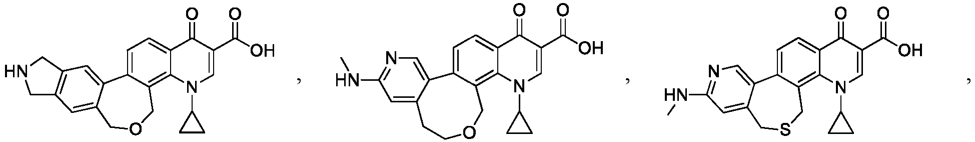

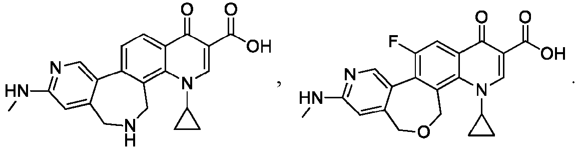

The present disclosure further provides compounds represented by the following formulas, stereoisomers, or pharmaceutically acceptable salts thereof,

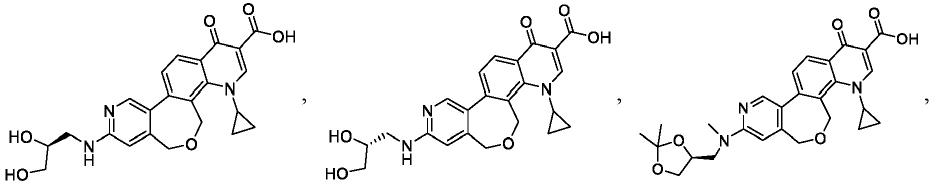

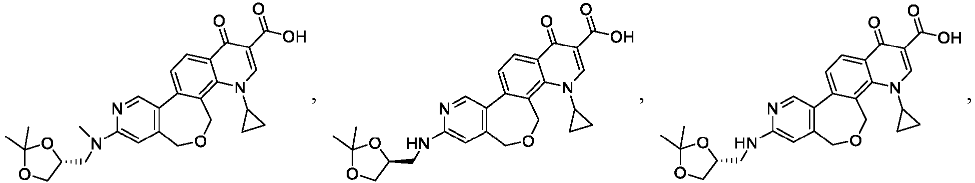

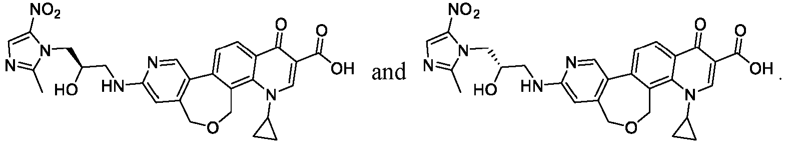

The present disclosure further provides use of the aforementioned compounds, stereoisomers, or pharmaceutically acceptable salts thereof in the preparation of antibacterial and anti-inflammatory drugs.

In some embodiments of the present disclosure, the above use is preferably use in the preparation of drugs for treating acne.

The present disclosure further provides use of the aforementioned compounds, stereoisomers, or a pharmaceutically acceptable salts thereof in the preparation of drugs for treating acne.

The present disclosure further provides the biological experimental testing methods for the aforementioned compounds.

### Experimental testing method 1: propionibacterium acnes-induced THP-1 cell inflammation model experiment

### Experimental purpose:

*Propionibacterium* acnes-induced THP-1 cell inflammation model is established.

### Experimental materials:

Strains: *Propionibacterium acnes ATCC 6919*; culture medium: brucella agar (containing 5% LHB+5 µg/ml Hemin+1 µg/ml VitaminK₁); cells: THP-1 ATCC TIB-202; culture medium: RPMI1640 (gibco 22400-089) +10% heat inactivated FBS+1×GlutaMAX (100×GlutaMAX: gibco 3050-061).

### Experimental protocols:

### a) Preparation of Inflammatory Stimulants

i. The glycerol tube containing *Propionibacterium acnes ATCC 6919* is inoculated in brucella agar (containing 5% LHB+5 µg/mL Hemin+1 µg/mL VitaminK₁), and then cultured in an anaerobic environment at 37°C for 48 hours.
ii. The plate cultures are collected and suspended in polybutylene succinate (PBS), and then the number of bacterial colonies is adjusted to 3×10⁹ cfu/mL. Then, the plate cultures are placed in a water bath at 80°C to inactivate for 30 minutes. Colony forming unit (CFU) counting is performed before and after bacteria inactivation.
iii. The inactivated bacterial suspension is subpackaged and stored in a refrigerator at -80°C for future use.

### b) Preparation of THP-1 cells

i. Thawing and culture of cells: the THP-1 cells cryopreserved in liquid nitrogen are thawed in RPMI1640 (containing 10% FBS and 1×glutamax). The thawed cells are subjected to one passage before used for the experiments.
ii. Cell inoculation: 2×10⁵ cells (190 µL, 1.1×10⁶ cfu/mL) are inoculated in a 96-well plate for future use.

### c) Induction and stimulation of cells

i. The cells are stimulated with 8 µL of heat inactivated *Propionibacterium acnes* (2 concentrations tested: 3×10⁹ cfu/mL (high concentration) and 6×10⁸ cfu/mL (low concentration) to induce inflammatory cytokines in two parallel experiments. After induction for 1 hour, the test plate is incubated at 37°C for 24 hours, 48 hours, and 72 hours, respectively. PBS is used as negative control. After incubation, the test plate is centrifuged at 4000 RPM for 5 minutes. The supernatant is collected and stored at -80°C for future use.

### d) Enzyme-linked immunosorbent assay (ELISA)

i. The level of cytokine IL-8 in the cell supernatant is measured according to the instructions of the Human CXCL8/IL-8 Quantikine ELISA Kit (R&D-D8000C).
ii. The level of cytokine IL-6 in the cell supernatant is measured according to the instructions of the Human IL-6 Quantikine ELISA Kit (R&D-D6050).

### Technical Effects

In the present disclosure, a compound of formula (I) and a pharmaceutically acceptable salt thereof are synthesized through a simple preparation method, so that a class of novel non-fluorinated quinolone antibiotics are obtained, which act on DNA gyrase and TLR-MD2 dual targets, and are used to treat acne caused by *Propionibacterium acnes,* thereby solving the problem of *Propionibacterium acnes* resistance.

### Definitions and Descriptions

Unless otherwise stated, the following terms and phrases used herein are intended to have the following meanings. A specific term or phrase should not be considered uncertain or unclear without a specific definition, but should be interpreted by ordinary meanings. A trade name herein refers to a corresponding product or active ingredient thereof.

The term "pharmaceutically acceptable" used herein refers to compounds, materials, compositions and/or dosage forms that are suitable for use in contact with human and animal tissues without excessive toxicity, irritation, allergic reactions or other problems or complications within the scope of reliable medical judgment, and are proportional to a reasonable benefit/risk ratio.

The term "pharmaceutically acceptable salt" refers to a salt of the compound of the present disclosure, which is prepared by the compound with a specific substituent found in the present disclosure and a relatively non-toxic acid or base. When the compounds of the present disclosure contain relatively acidic functional groups, an alkali addition salt can be obtained by contacting such compounds with a sufficient amount of alkali in a pure solution or suitable inert solvent. When the compounds of the present disclosure contain relatively alkalic functional groups, an acid addition salt can be obtained by contacting such compounds with a sufficient amount of acid in a pure solution or a suitable inert solvent. Some specific compounds in the present disclosure contain both alkalic and acidic functional groups, which can be converted into any alkali or acid addition salts. The present disclosure is also envisaged the N cationic quaternary ammonium salts formed by any compounds containing the N groups. N⁺R₁(A⁻) means a quaternary ammonium salt of which the heteroatom N is cation, and A⁻ is F, Cl⁻, Br, I⁻, OH⁻, HCO₃⁻, HSO₃⁻, HSO₄⁻, NO₃⁻, ClO⁻, BrO⁻, I₃⁻, ClO₃⁻, ClO₄⁻, HCOO⁻, CH₃COO⁻, H₂PO₄⁻, etc.

The pharmaceutically acceptable salts of the present disclosure can be synthesized from a parent compound containing an acidic or basic group by conventional chemical methods. In general cases, the preparation method of such salts is to react these compounds in the form of a free acid or base with a stoichiometric amount of appropriate base or acid in water or an organic solvent or a mixture of both.

In addition to the form of salt, the compounds provided by the present disclosure also have prodrug forms. The prodrugs of the compounds described herein are prone to chemical changes under physiological conditions, so as to be converted into the compounds of the present disclosure. In addition, the prodrugs can be converted *in vivo* into the compounds of the present disclosure through chemical or biochemical methods.

Some compounds of the present disclosure can exist in a non-solvated or solvated form, including a hydrate form. Generally, the solvated form is equivalent to the non-solvated form and is included within the scope of the present disclosure.

Unless otherwise specified, the terms "treat", "treating" and "treatment" are intended to refer to all processes that may slow down, interrupt, restrain, or prevent the progression of a disease, but may not necessarily mean that all symptoms have been completely eliminated.

The compounds of the present disclosure can exist in a specific geometric isomer or stereoisomer form. All such compounds are contemplated in the present disclosure, including *cis-* and *trans*-isomers, (-)- and (+)- enantiomers, (*R*)- and (*S*)-enantiomers, diastereoisomers, (*D*)-isomers, (*L*)-isomers, their racemic mixtures, and other mixtures, such as enantiomer- or diastereoisomer-enriched mixtures, all of which fall within the scope of the present disclosure. Additional asymmetric carbon atoms can exist in the substituents such as alkyl. All these isomers and their mixtures are included within the scope of the present disclosure.

Unless otherwise specified, the term "enantiomer" or "optical isomer" refer to stereoisomers that are in mirrorimage relationship of each other.

Unless otherwise specified, the term "*cis-trans* isomer" or "geometric isomer" is resulted from the inability of free rotation of a double bond or a single bond between ring-forming carbon atoms.

Unless otherwise specified, the term "diastereoisomer" refers to a stereoisomer where a molecule has two or more chiral centers and has a non-mirror image relationship between molecules.

Unless otherwise specified, "(+)" represents a dextroisomer, "(-)" represents a laevoisomer, and "(±)" represents a racemate.

Unless otherwise specified, a wedge solid line bond ( 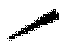 ) and a wedge dotted line bond ( 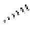 ) represent the absolute configuration of a stereocentre, a straight solid line bond ( 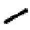 ) and a straight dotted line bond ( 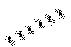 ) represent the relative configuration of a stereocentre, a wavy line ( 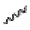 ) represents the wedge solid line bond ( 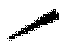 ) or the wedge dotted line bond ( 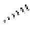 ), or a wavy line ( 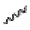 ) represents the straight solid line bond ( 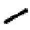 ) or the straight dotted line bond ( 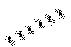 ).

The compounds of the present disclosure can exist in a specific form. Unless otherwise specified, the term "tautomer" or "tautomeric form" refers to a dynamic equilibrium of isomers with different functional groups at room temperature and their ability to quickly transform into each other. If a tautomer is possible (such as in solution), the chemical equilibrium of a tautomer can be achieved. For example, a proton tautomer (also known as prototropic tautomer) includes a mutual transformation through proton transfer, such as keto-enol isomerization and imine-enamine isomerization. A valence tautomer includes a mutual transformation through the recombination of some bonding electrons. Among them, the specific example of keto-enol tautomerization is the tautomerization between the two tautomers of pentane-2,4-dione and 4-hydroxypenta-3-en-2-one.

Unless otherwise specified, the term "rich in an isomer", "isomer-enriched", "rich in an enantiomer", or "enantiomer-enriched" refers to the content of an isomer or enantiomer being less than 100%, and the content of the isomer or enantiomer being greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "isomeric excess" or "enantiomeric excess" refers to the difference in relative percentages between two isomers or two enantiomers. For example, if the content of one isomer or enantiomer is 90% and the content of the other isomer or enantiomer is 10%, the isomeric excess or enantiomeric excess (ee value) is 80%.

Optically active (*R*)- and (*S*)- isomers as well as *D-* and *L-* isomers can be prepared through chiral synthesis, chiral reagents, or other conventional techniques. In order to obtain one enantiomer of a certain compound in the present disclosure, it can be prepared through asymmetric synthesis or derivatization with a chiral auxiliary reagent, in which the obtained diastereomeric mixture is separated and the auxiliary groups are split to provide the pure desired enantiomer. Alternatively, when a molecule contains a basic functional group (such as an amino group) or an acidic functional group (such as carboxyl), it forms a salt of diastereoisomer with an appropriate optically active acid or base, which is then subjected to resolution of diastereoisomer using conventional methods well-known in the art, and then the pure enantiomer is recovered. In addition, the separation of enantiomers and diastereoisomers is usually achieved through chromatography, which uses chiral stationary phases and optionally combines with chemical derivatization methods (such as generation of carbamate from amines).

The compound of the present disclosure can contain non-natural ratios of atomic isotopes on one or more atoms that make up the compound. For example, the compound can be labeled with a radioactive isotope, such as tritium (³H), iodine-125 (¹²⁵I), or C-14 (¹⁴C). For example, a deuterated drug can be formed by replacing hydrogen with deuterium. The bond between deuterium and carbon is stronger than the bond between ordinary hydrogen and carbon, compared to an un deuterated drug, the deuterated drug has the advantages of reduced toxic side effects, increased drug stability, enhanced therapeutic effect, and extended biological half-life period, etc. The transformations of all isotopic compositions of the compound in the present disclosure, whether radioactive or not, are included within the scope of the present disclosure.

The term "optional" or "optionally" refers to a situation where the subsequently described events or conditions may occur but are not necessarily, and this description includes a situation where said events or conditions may occur and may not occur.

The term "substituted" refers to the substitution of any one or more hydrogen atoms on a specific atom by a substituent, and the substituent can include variants of deuterium and hydrogen, as long as the specific atom is in a normal valence state and the substituted compound is stable. When the substituent is oxygen (i.e., =O), it means that two hydrogen atoms are substituted. For example, when R₃ in the present disclosure is =O, 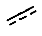 in the structural unit 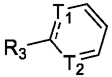 is a single bond, and the structural unit

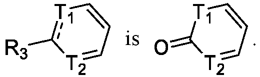

The term "optionally substituted" refers to a situation where a group may be substituted or may not be substituted, and unless otherwise specified, the type and number of the substituents can be arbitrary on a chemically achievable basis.

When any variable (such as R) appears more than one time in the composition or structure of a compound, its definition in each case is independent. Therefore, for example, if a group is substituted by 0-2 R, said group can be optionally substituted by at most two R, and t R is independently selected in each case. In addition, a combination of a substituent and/or a variant thereof is allowed only if such combination can produce a stable compound.

When the number of a connecting group is 0, such as -(CRR)₀-, it indicates that the connecting group is a single bond.

When one of the variables is selected from a single bond, it indicates that the two groups are connected by it directly. For example, when L in A-L-Z represents a single bond, it indicates that the structure is actually A-Z.

When a substituent is absent, it indicates that the substituent does not exist, for example, when X in A-X is absent, it indicates that the structure is actually A.

When the atom by which the listed substituents are connected to a group to be substituted is not indicated, the substituents can be bonded through any atoms thereof. For example, pyridyl as a substituent can be connected to a group to be substituted through any carbon atom on the pyridine ring.

When the connecting direction is not indicated for the listed connecting groups, the connecting direction is arbitrary. For example, if the connecting group L in 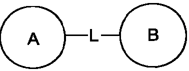 is -M-W-, the -M-W- can connect ring A and ring B in the same direction as a reading order from left to right to form 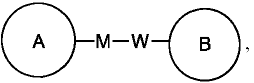 or connect ring A and ring B in an opposite direction to the reading order from left to right to form 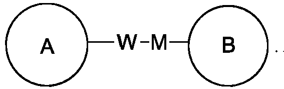 A combination of the connecting group, substituent and/or variant thereof is allowed only if such combination can produce a stable compound. Unless otherwise specified, when a certain group has one or more connectable sites, any one or more sites of the group can be connected to other groups through a chemical bond. If the connecting way of the chemical bond is unoriented and there are H atoms at the connectable sites, when the chemical bond is connected, the number of H atoms at that site will correspondingly decrease with the number of the connected chemical bonds and become the groups having the corresponding valence number. The chemical bonds connecting said site to other groups can be represented by a straight solid line bond ( 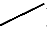 ), a straight dotted line bond ( 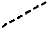 ), or a wavy line ( 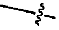 ). For example, the straight solid line bond in -OCH₃ represents the connection to other groups via the oxygen atom in this group; the straight dotted line bond in 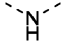 represents the connection to other groups via the two ends of the nitrogen atom in this group: and the wavy line in 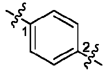 represents the connection to other groups via the carbon atoms at positions 1 and 2 of phenyl. 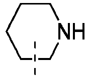 indicates that any connectable sites on the piperidyl can be connected to other groups via one chemical bond, including at least four connecting ways 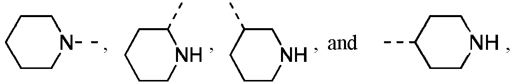 even if a H atom is shown on -N-, 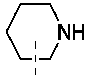 still includes the group in the connecting way of 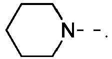 However, when one chemical bond is connected, the H at that site will correspondingly decrease by one to become the corresponding monovalent piperidyl.

Unless otherwise specified, the term "alkyl" itself or as part of another substituent represents a linear or branched saturated hydrocarbon group. The alkyl can be C₁₋₆ alkyl or C₁₋₃ alkyl. The alkyl is optionally substituted by one or more of the following groups: oxo, hydroxyl, amino, nitro, halogen, cyano, alkenyl, alkynyl, alkoxy, haloalkoxy, alkylamino, dialkylamino, haloalkylamino, halodialkylamino, cycloalkyl, cycloalkyloxy, heterocyclyl, heterocyclyloxy, heterocycloalkyl, heterocycloalkyloxy, heteroaryl, heteroaryloxy, aryl or aryloxy.

Unless otherwise specified, the term "C₁₋₃ alkyl" is used to refer to a linear or branched saturated hydrocarbon group consisting of 1 to 3 carbon atoms. The C₁₋₃ alkyl includes C₁₋₂ and C₂₋₃ alkyl, etc., which may be monovalent (such as methyl), divalent (such as methylene), or multivalent (such as methenyl). The examples of C₁₋₃ alkyl include but are not limited to methyl (Me), ethyl (Et), propyl (including n-propyl and isopropyl), etc.

Unless otherwise specified, the term "alkoxy" refers to those alkyl groups connected to the rest of the molecule through an oxygen atom. The alkoxy can be C₁₋₆ alkoxy or C₁₋₃ alkoxy. The alkoxy is optionally substituted by one or more of the following groups: oxo, hydroxyl, amino, nitro, halogen, cyano, alkenyl, alkynyl, alkoxy, haloalkoxy, alkylamino, dialkylamino, haloalkylamino, halodialkylamino, cycloalkyl, cycloalkyloxy, heterocyclyl, heterocyclyloxy, heterocycloalkyl, heterocycloalkyloxy, heteroaryl, heteroaryloxy, aryl or aryloxy.

Unless otherwise specified, the term "C₁₋₃ alkoxy" refers to alkyl groups containing 1 to 3 carbon atoms which are connected to the rest of the molecule through an oxygen atom. The C₁₋₃ alkoxy includes C₁₋₂, C₂₋₃, C₃ and C₂ alkoxy, etc. The examples of C₁₋₃ alkoxy include but are not limited to methoxy, ethoxy, propoxy (including n-propoxy and isopropoxy), etc.

Unless otherwise specified, the term "C₁₋₃ alkylamino" refers to alkyl groups containing 1 to 3 carbon atoms which are connected to the rest of the molecule through an amino group. The C₁₋₃ alkylamino includes C₁₋₂, C₃ and C₂ alkylamino, etc. The examples of C₁₋₃ alkylamino include but are not limited to -NHCH₃, -N(CH₃)₂, -NHCH₂CH₃, - N(CH₃)CH₂CH₃, -NHCH₂CH₂CH₃, and -NHCH₂(CH₃)₂, etc.

Unless otherwise specified, the term "halo" or "halogen" itself or as a part of another substituent represents fluorine, chlorine, bromine, or iodine atoms.

The term "cycloalkyl" refers to a carbon ring that is completely saturated and can exist as a single ring, a bridged ring, or a spirocyclic ring. Unless otherwise indicated, the carbon ring is generally a 3-12 membered ring. The non-limiting examples of cycloalkyl include but are not limited to cyclopropyl, cyclobutyl, cyclopentyl, cyclohexyl, norbornyl (bicyclo[2.2.1]heptyl), bicyclo[2.2.2]octyl, adamantyl, etc. The cycloalkyl can be C₃₋₆ cycloalkyl. The cycloalkyl is optionally substituted by one or more of the following groups: oxo, hydroxyl, amino, nitro, halogen, cyano, alkyl, alkenyl, alkynyl, alkoxy, haloalkoxy, alkylamino, dialkylamino, haloalkylamino, halodialkylamino, carboxyl, -C(O)O- alkyl, -OC(O)- alkyl, -C(O)NH₂, -C(O)NH- alkyl, -C(O)N( alkyl)₂, -NHC(O)- alkyl, -C(O)- alkyl, -S(O)- alkyl, -S(O)₂- alkyl, -S(O)₂NH₂, -S(O)₂NH- alkyl, -S(O)₂N(alkyl)₂, cycloalkyl, cycloalkyloxy, heterocyclyl, heterocyclyloxy, heterocycloalkyl, heterocycloalkyloxy, heteroaryl, heteroaryloxy, aryl or aryloxy.

Unless otherwise specified, "C₃₋₆ cycloalkyl" represents a saturated cyclic hydrocarbon group composed of 3 to 6 carbon atoms, which is a single ring and a double ring system, the C₃₋₆ cycloalkyl includes C₃₋₅, C₄₋₅ and C₅₋₆ cycloalkyl, etc. and it can be monovalent, divalent, or multivalent. The examples of C₃₋₆ cycloalkyl include but are not limited to cyclopropyl, cyclobutyl, cyclopentyl, and cyclohexyl, etc.

The term "heterocyclyl" refers to a non-aromatic ring that is completely saturated or partially unsaturated (but not completely unsaturated heteroaromatic group) and can exist as a single ring, a bridged ring, or a spirocyclic ring. Unless otherwise indicated, the heterocyclyl ring is generally a 3-12 membered ring containing 1 to 3, preferably 1 or 2 heteroatoms independently selected from the group consisting of sulfur, oxygen, and/or nitrogen. The non-limiting examples of heterocyclyl include but are not limited to oxiranyl, tetrahydrofuryl, dihydrofuryl, pyrrolidinyl, N-methylpyrrolidinyl, dihydropyrrolyl, piperidyl, piperazinyl, pyrazolidinyl, 4H-pyranyl, morpholinyl, thiomorpholinyl, tetrahydrothienyl, etc. The heterocyclyl is optionally substituted by one or more of the following groups: oxo, hydroxyl, amino, nitro, halogen, cyano, alkyl, alkenyl, alkynyl, alkoxy, haloalkoxy, alkylamino, dialkylamino, haloalkylamino, halodialkylamino, carboxyl, -C(O)O-alkyl, -OC(O)-alkyl, -C(O)NH₂, -C(O)NH-alkyl, -C(O)N(alkyl)₂, -NHC(O)-alkyl, -C(O)-alkyl, -S(O)-alkyl, -S(O)₂-alkyl, -S(O)₂NH₂. -S(O)₂NH-alkyl, -S(O)₂N(alkyl)₂, cycloalkyl, cycloalkyloxy, heterocyclyl, heterocyclyloxy, heterocycloalkyl, heterocycloalkyloxy, heteroaryl, heteroaryloxy, aryl or aryloxy.

The term "heterocycloalkyl" refers to a cyclic group that is completely saturated and can exist as a single ring, a bridged ring, or a spirocyclic ring. Unless otherwise indicated, the heterocyclyl ring is generally a 4-12 membered ring containing 1 to 3, preferably 1 or 2 heteroatoms independently selected from the group consisting of sulfur, oxygen, and/or nitrogen. The non-limiting examples of 4-membered heterocycloalkyl include but are not limited to azetidinyl, oxetanyl, thietanyl; the examples of 5-membered heterocycloalkyl include but are not limited to tetrahydrofuryl, tetrahydrothienyl, pyrrolidinyl, isoxazolidinyl, oxazolidinyl, isothiazolidinyl, thiazolidinyl, imidazolidinyl, tetrahydropyrazolyl; the examples of 6-membered heterocycloalkyl include but are not limited to piperidyl, tetrahydropyranyl, tetrahydrothianyl, morpholinyl, piperazinyl, 1,4-thioxanyl, 1,4-dioxanyl, thiomorpholinyl, 1,3-dithianyl, 1,4-dithianyl; and the examples of 7-membered heterocycloalkyl include but are not limited to azepanyl, oxepanyl, thiepanyl. The heterocycloalkyl can be 4-6-membered heterocycloalkyl, or it can be a monocyclic heterocycloalkyl with 5 or 6 ring atoms. The heterocycloalkyl is optionally substituted by one or more of the following groups: oxo, hydroxyl, amino, nitro, halogen, cyano, alkyl, alkenyl, alkynyl, alkoxy, haloalkoxy, alkylamino, dialkylamino, haloalkylamino, halodialkylamino, carboxyl, -C(O)O-alkyl, -OC(O)-alkyl, -C(O)NH₂, - C(O)NH-alkyl, -C(O)N(alkyl)₂, -NHC(O)-alkyl, -C(O)-alkyl, -S(O)-alkyl, -S(O)₂-alkyl, -S(O)₂NH₂, -S(O)₂NH-alkyl, -S(O)₂N(alkyl)₂, cycloalkyl, cycloalkyloxy, heterocyclyl, heterocyclyloxy, heterocycloalkyl, heterocycloalkyloxy, heteroaryl, heteroaryloxy, aryl or aryloxy.

Unless otherwise specified, the term "3-6 membered heterocycloalkyl" represents a saturated cyclic group composed of 3 to 6 ring atoms, respectively, either in itself or in combination with other terms, 1, 2, 3, or 4 ring atoms of which are heteroatoms independently selected from the group consisting of O, S, and N, while the rests are carbon atoms, with nitrogen atoms being optionally quaternized and carbon, nitrogen, and sulfur heteroatoms being optionally oxidized (i.e., C(O), NO and S(O)ₚ, where p is 1 or 2). It includes a single ring and a double ring system, where the double ring system includes a spiro ring, a fused ring, and a bridged ring. In addition, in terms of "3-6 membered heterocycloalkyl", the heteroatoms can occupy the connecting position between the heterocycloalkyl and the rest of the molecule. The 3-6 membered heterocycloalkyl includes 4-6 membered, 5-6 membered, 4-membered, 5-membered and 6-membered heterocycloalkyl, etc. The examples of 3-6 membered heterocycloalkyl include but are not limited to azetidinyl, oxetanyl, thietanyl, pyrrolidinyl, pyrazolidinyl, imidazolidinyl, tetrahydrothienyl (including tetrahydrothiophen-2-yl and tetrahydrothiophen-3-yl, etc.), tetrahydrofuryl (including tetrahydrofuran-2-yl, etc.), tetrahydropyranyl, piperidyl (including 1-piperidyl, 2-piperidyl and 3-piperidyl, etc.), piperazinyl (including 1-piperazinyl and 2-piperazinyl, etc.), morpholinyl (including 3-morpholinyl and 4-morpholinyl, etc.), dioxanyl, dithanyl, isoxazolidinyl, isothiazolidinyl, 1,2-oxazinyl, 1,2- thiazinyl or hexahydropyridazinyl, etc.

Unless otherwise specified, the term "5-6 membered heterocycloalkyl" represents a saturated cyclic group composed of 5 to 6 ring atoms, respectively, either in itself or in combination with other terms, 1, 2, 3, or 4 ring atoms of which are heteroatoms independently selected from the group consisting of O, S, and N, while the rests are carbon atoms, with nitrogen atoms being optionally quaternized and carbon, nitrogen, and sulfur atoms being optionally oxidized (i.e., C(O), NO and S(O)ₚ, where p is 1 or 2). It includes a single ring and a double ring system, where the double ring system includes a spiro ring, a fused ring, and a bridged ring. In addition, in terms of "5-6 membered heterocycloalkyl", the heteroatoms can occupy the connecting position between the heterocycloalkyl and the rest of the molecule. The 5-6 membered heterocycloalkyl includes 5-membered and 6-membered heterocycloalkyl. The examples of 5-6 membered heterocycloalkyl include but are not limited to pyrrolidinyl, pyrazolidinyl, imidazolidinyl, tetrahydrothienyl (including tetrahydrothiophen-2-yl and tetrahydrothiophen-3-yl, etc.), tetrahydrofuryl (including tetrahydrofuran-2-yl, etc.), tetrahydropyranyl, piperidyl (including 1-piperidyl, 2-piperidyl and 3-piperidyl, etc.), piperazinyl (including 1-piperazinyl and 2-piperazinyl, etc.), morpholinyl (including 3-morpholinyl and 4-morpholinyl, etc.), dioxanyl, dithanyl, isoxazolidinyl, isothiazolidinyl, 1,2-oxazinyl, 1,2- thiazinyl hexahydropyridazinyl, etc.

Unless otherwise specified, the term "5-6 membered heterocycloalkenyl" represents a partially unsaturated cyclic group consisting of 5 to 6 ring atoms containing at least one carbon-carbon double bond, respectively, either in itself or in combination with other terms, 1, 2, 3, or 4 ring atoms of which are heteroatoms independently selected from the group consisting of O, S, and N, while the rest are carbon atoms, with nitrogen atoms being optionally quaternized and carbon, nitrogen, and sulfur atoms being optionally oxidized (i.e., C(O), NO and S(O)ₚ, where p is 1 or 2). It includes a single ring and a double ring system, where the double ring system includes a spiro ring, a fused ring, and a bridged ring, and any ring in this system is nonaromatic. In addition, in terms of "5-6 membered heterocycloalkenyl", the heteroatoms can occupy the connecting position between the heterocycloalkenyl and the rest of the molecule. The 5-6 membered heterocycloalkenyl includes 5-membered and 6-membered heterocycloalkenyl, etc. The examples of 5-6 membered heterocycloalkenyl include but are not limited to 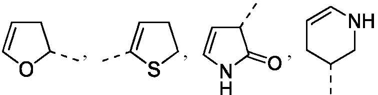 or

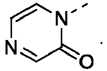

The term "heteroaryl" refers to a single ring or a fused polycyclic system which contains at least one ring atom selected from the group consisting of N, O, and S, with the rest of the ring atom being C, and has at least one aromatic ring. The preferred heteroaryl has a single 4-8 tmembered ring, especially a 5-8 membered ring, or multiple fused rings containing 6 to 14, especially 6 to 10 ring atoms. The non-limiting examples of heteroaryl include but are not limited to pyrrolyl, furyl, thienyl, imidazolyl, oxazolyl, pyrazolyl, pyridyl, pyrimidinyl, pyrazinyl, quinolyl, isoquinolyl, tetrazolyl, triazolyl, triazinyl, benzofuryl, benzothienyl, indolyl, isoindolyl, etc. The heteroaryl can be 5-6 membered heteroaryl. The heteroaryl is optionally substituted by one or more of the following groups: hydroxyl, amino, nitro, halogen, cyano, alkyl, alkenyl, alkynyl, alkoxy, haloalkoxy, alkylamino, dialkylamino, haloalkylamino, halodialkylamino, carboxyl, -C(O)O- alkyl, -OC(O)-alkyl, -C(O)NH₂, -C(O)NH-alkyl, -C(O)N(alkyl)₂, -NHC(O)-alkyl, -C(O)-alkyl, -S(O)-alkyl, -S(O)₂-alkyl, -S(O)₂NH₂, -S(O)₂NH- alkyl, -S(O)₂N(alkyl)₂, cycloalkyl, cycloalkyloxy, heterocyclyl, heterocyclyloxy, heterocycloalkyl, heterocycloalkyloxy, heteroaryl, heteroaryloxy, aryl or aryloxy.

Unless otherwise specified, the terms "5-6 membered heteroaryl ring" and "5-6 -membered heteroaryl" in the present disclosure can be used interchangeably, the term "5-6 membered heteroaryl" represents a single ring group with conjugated π electron systems consisting of 5 to 6 ring atoms, 1, 2, 3, or 4 ring atoms of which are heteroatoms independently selected from the group consisting of O, S, and N, while the rests are carbon atoms. Where, the nitrogen atoms are optionally quaternized and carbon, nitrogen, and sulfur atoms are optionally oxidized (i.e., C(O), NO and S(O)ₚ, where p is 1 or 2). The 5-6membered heteroaryl can be connected to the rest of the molecule through heteroatoms or carbon atoms. The 5-6membered heteroaryl includes 5-membered and 6-membered heteroaryl. The examples of the 5-6 membered heteroaryl include but are not limited to pyrrolyl (including *N*-pyrrolyl, 2-pyrrolyl and 3-pyrrolyl, etc.), pyrazolyl (including 2-pyrazolyl and 3-pyrazolyl, etc.), imidazolyl (including *N*-imidazolyl, 2-imidazolyl, 4-imidazolyl and 5-imidazolyl, etc.), oxazolyl (including 2-oxazolyl, 4-oxazolyl and 5-oxazolyl, etc.), triazolyl (1*H*-1,2,3-triazolyl, 2*H*-1,2,3-triazolyl, 1*H*-1,2,4-triazolyl and 4*H*-1,2,4-triazolyl, etc.), tetrazolyl, isoxazolyl (3-isoxazolyl, 4-isoxazolyl and 5-isoxazolyl, etc.), thiazolyl (including 2-thiazolyl, 4-thiazolyl and 5-thiazolyl, etc.), furyl (including 2-furyl and 3-furyl, etc.), thienyl (including 2-thienyl and 3-thienyl, etc.), pyridyl (including 2-pyridyl, 3-pyridyl and 4-pyridyl, etc.), pyrazinyl or pyrimidinyl (including 2-pyrimidinyl and 4-pyrimidinyl, etc.).

Unless otherwise specified, the terms "5-membered heteroaryl ring" and "5-membered heteroaryl" in the present disclosure can be used interchangeably, the term "5-membered heteroaryl" represents a single ring group with conjugated π electron systems consisting of 5 ring atoms, 1, 2, 3, or 4 ring atoms of which are heteroatoms independently selected from the group consisting of O, S, and N, while the rest are carbon atoms. Where, the nitrogen atoms are optionally quaternized and carbon, nitrogen, and sulfur atoms are optionally oxidized (i.e. C(O), NO and S(O)ₚ, where p is 1 or 2). The 5-membered heteroaryl can be connected to the rest of the molecule through heteroatoms or carbon atoms. The examples of the 5-membered heteroaryl include but are not limited to pyrrolyl (including *N-*pyrrolyl, 2-pyrrolyl and 3-pyrrolyl, etc.), pyrazolyl (including 2-pyrazolyl and 3-pyrazolyl, etc.), imidazolyl (including *N*-imidazolyl, 2-imidazolyl, 4-imidazolyl and 5-imidazolyl, etc.), oxazolyl (including 2-oxazolyl, 4-oxazolyl and 5-oxazolyl, etc.), triazolyl (1*H*-1,2,3-triazolyl, 2*H*-1,2,3-triazolyl, 1*H*-1,2,4-triazolyl and 4*H*-1,2,4-triazolyl, etc.), tetrazolyl, isoxazolyl (3-isoxazolyl, 4-isoxazolyl and 5-isoxazolyl, etc.), thiazolyl (including 2-thiazolyl, 4-thiazolyl and 5-thiazolyl, etc.), furyl (including 2-furyl and 3-furyl, etc.), thienyl (including 2-thienyl and 3-thienyl, etc.).

Unless otherwise specified, Cₙ₋ₙ₊ₘ or Cₙ-Cₙ₊ₘ includes any specific case of n to n+m carbons, for example C₁₋₁₂ includes C₁, C₂, C₃, C₄, C₅, C₆, C₇, C₈, C₉, C₁₀, C₁₁, and C₁₂, also includes any range from n to n+m, e.g. C₁₋₁₂ includes C₁₋₃, C₁₋₆, C₁₋₉, C₃₋₆, C₃₋₉, C₃₋₁₂, C₆₋₉, C₆₋₁₂, and C₉₋₁₂, etc.. Similarly, n- to n+m-membered indicates that the number of atoms on the ring is n to n+m, e.g. 3-12 membered ring includes 3-membered ring, 4-membered ring, 5-membered ring, 6-membered ring, 7-membered ring, 8-membered ring, 9-membered ring, 10-membered ring, 11-membered ring, and 12-membered ring, also includes any range from n to n+m, e.g. 3-12 membered ring includes 3-6 membered ring, 3-9 membered ring, 5-6 membered ring, 5-7 membered ring, 6-7membered ring, 6-8 membered ring, and 6-10 membered ring, etc.

The term "leaving group" refers to a functional group or atom that can be substituted by another functional group or atom through a substitution reaction (such as a nucleophilic substitution reaction). For example, the representative leaving groups include triflate; chlorine, bromine, iodine; sulfonate group, such as methanesulfonate, tosylate, brosylate, p-toluenesulfonate, etc; acyloxy group, such as acetoxyl, trifluoroacetoxyl, etc.

The term "protecting group" includes but is not limited to "amino protecting group", "hydroxyl protecting group" or "sulfydryl protecting group". The term "amino protecting group" refers to a protecting group suitable for preventing the side reactions on the nitrogen site of an amino group. Representative amino protection groups include but are not limited to: formyl; acyl, such as alkanoyl (such as acetyl, trichloroacetyl, or trifluoroacetyl); alkoxycarbonyl, such as tert-butoxycarbonyl (Boc); arylmethoxycarbonyl, such as carbobenzoxy (Cbz) and 9-fluorene methoxycarbonyl (Fmoc); arylmethyl, such as benzyl (Bn), trityl (Tr), 1,1-di(4'-methoxyphenyl)methyl; silyl, such as trimethylsilyl (TMS), 2-(trimethylsilyl)ethoxymethyl (SEM), and tert-butyldimethylsilyl (TBS), etc. The term "hydroxyl protecting group" refers to a protecting group suitable for preventing the side reactions of a hydroxyl group. Representative hydroxyl protecting groups include but are not limited to: alkyl, such as methyl, ethyl, and tert-butyl; acyl, such as alkanoyl (such as acetyl); arylmethyl, such as benzyl (Bn), p-methoxybenzyl (PMB), 9-fluorenylmethyl (Fm), and diphenylmethyl (benzhydryl, DPM); silyl, such as trimethylsilyl (TMS) and tert-butyl dimethylsilyl (TBS), etc.

The compound of the present disclosure can be prepared through various synthesis methods well-known to those skilled in the art, including the specific embodiments listed below, the embodiments formed by combining the specific embodiments with other chemical synthesis methods, and equivalent substitutions well-known to those skilled in the art, wherein the preferred embodiments include but are not limited to the examples of the present disclosure.

The structures of the compounds in the present disclosure can be confirmed by conventional methods well-known to those skilled in the art. If the present disclosure involves the absolute configuration of a compound, the absolute configuration can be confirmed by conventional technical means in the art. For example, the single crystal X-ray diffraction (SXRD) is to collect diffraction intensity data from the cultured single crystals using a Bruker D8 venture diffractometer, with CuKα radiation as light source, scanning mode: φ/ω scanning, after the relevant data are collected, the crystal structure is further resolved by a direct method (Shelxs97) to confirm the absolute configuration.

The solvents used in the present disclosure are commercially available.

The present disclosure uses the following abbreviations: DMAP represents 4-dimethylaminopyridine; DMSO represents dimethylsulfoxide; CFU represents colony forming unit; DCM represents dichloromethane; DMF represents N,N-dimethylformamide; DME represents ethylene glycol dimethyl ether; TFA represents trifluoroacetic acid; Boc₂O represents di-tert-butyl dicarbonate; NaOH represents sodium hydroxide; NaH represents sodium hydride; NBS represents N-bromosuccinimide; TBSCl represents tert-butyl dimethylsilyl chloride; HPLC represents high-performance liquid chromatography; LCMS represents liquid chromatography-tandem mass spectrometry; SBE-β-CD represents sulfobutylether-β-cyclodextrin; and PBS represents phosphate buffer.

Compounds are named according to conventional nomenclature principles in the art or using ChemDraw^{®} Software, and the commercially available compounds are named according to the suppliers' catalogue.

### DETAILED DESCRIPTION

The present disclosure will be described below in detail by way of examples, but it does not imply any adverse limitations on the present disclosure. The compound of the present disclosure can be prepared through various synthesis methods well-known to those skilled in the art, including the specific embodiments listed below, the embodiments formed by combining the specific embodiments with other chemical synthesis methods, and equivalent substitutions well-known to those skilled in the art, wherein the preferred embodiments include but are not limited to the examples of the present disclosure.

### Example 1

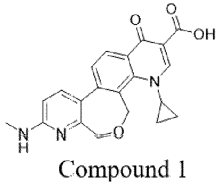

### Synthetic route:

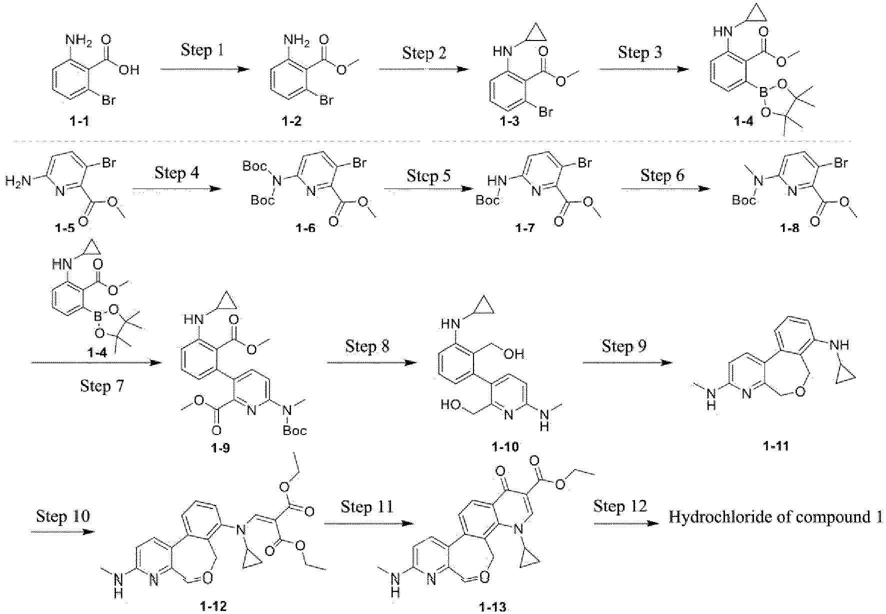

### Step 1: Preparation of Compound 1-2

Compound 1-1 (5.00 g, 23.14 mmol) was dissolved in sulfoxide chloride (65.13 g, 547.43 mmol, 39.71 mL). The mixture was stirred under reflux for 2 hours. The mixture concentrated under reduced pressure to remove sulfoxide chloride, then methanol (50 mL) was added, and the mixture was stirred at 65°C for 1 hour. The mixture was concentrated under reduced pressure to remove methanol, and then the reaction mixture was quenched with an aqueous sodium carbonate solution (50 mL). The reaction mixture was extracted with ethyl acetate (50 mL × 2), and the separated organic layer was washed with a saturated brine solution (30 mL), dried over anhydrous sodium sulfate, filtered, and concentrated under reduced pressure to obtain Compound 1-2. LCMS (ESI) m/z: 229.9/231.9 (M+1).

### Step 2: Preparation of Compound 1-3

Compound 1-2 (15 g, 65.20 mmol), cyclopropylboronic acid (11.20 g, 130.4 mmol), copper acetate (23.68 g, 130.40 mmol), 2, 2'-dipyridine (20.37 g, 130.40 mmol) and sodium carbonate (20.73 g, 195.60 mmol) were mixed in dichloromethane (240 mL), the reaction was replaced with O₂ three times, and then the mixture was stirred in an oxygen atmosphere at 15-25°C for 12 hours. The reaction mixture was filtered to obtain filtrate. The filtrate was added with water (200 mL), and then extracted with dichloromethane (100 mL × 2). The separated organic layer was washed with a saturated brine solution (100 mL), dried over anhydrous sodium sulfate, filtered, and concentrated under reduced pressure, and the resulting residue was purified by silica gel column chromatography (petroleum ether/ethyl acetate=1/0 to 10/1) to obtain Compound 1-3. LCMS (ESI) m/z: 270.0/272.0 (M+1).

### Step 3: Preparation of Compound 1-4

Compound 1-3 (1.8 g, 6.66 mmol), (diphenylphosphinoferrocene) dichloropalladium (487.59 mg, 666.37 µmol), bis(pinacolato) diboron (2.03 g, 8.00 mmol) and potassium acetate (1.96 g, 19.99 mmol) were mixed in dioxane (30 mL), the reaction was replaced with N₂ three times, then the mixture was stirred in a nitrogen atmosphere at 100°C for 4 hours. The reaction mixture was quenched with water (20 mL) and extracted with ethyl acetate (20 mL × 3), and the separated organic layer was washed with a saturated brine solution (20 mL), dried over anhydrous sodium sulfate, filtered, and concentrated under reduced pressure to obtain Compound 1-4. LCMS (ESI) m/z: 318.2 (M+1).

### Step 4: Preparation of Compound 1-6

Compound 1-5 (6.00 g, 25.97 mmol) was dissolved in dichloromethane (120 mL), and then (Boc)₂O (14.17 g, 64.92 mmol) and DMAP (317.26 mg, 2.60 mmol) were added. Then, the mixture was stirred at 15-25°C for 2 hours. The reaction solution was washed with an aqueous saturated sodium bicarbonate solution (50 mL) and a saturated brine solution (50 mL), respectively, dried over anhydrous sodium sulfate, filtered, and concentrated under reduced pressure to obtain Compound 1-6. LCMS (ESI) m/z: 431.0/433.0 (M+1).

### Step 5: Preparation of Compound 1-7

Potassium carbonate (9.61 g, 69.56 mmol) was added to a solution of Compound 1-6 (10.00 g, 23.19 mmol) in methanol (45 mL). The mixture was stirred at 15-25°C for 2 hours. The mixture was concentrated under reduced pressure to remove methanol, and then the reaction mixture was quenched with water (50 mL). The reaction mixture was extracted with ethyl acetate (50 mL × 2), and the separated organic layer was washed with a saturated brine solution (30 mL), dried over anhydrous sodium sulfate, filtered, and concentrated under reduced pressure to obtain the residue. The residue was purified through silica gel column chromatography (petroleum ether/ethyl acetate = 10/0 to 2/1) to obtain Compound 1-7. ¹H NMR (400 MHz, CDCl₃) δ = 8.02 - 7.95 (m, 1H) 7.93 - 7.86 (m, 1H) 7.41 (br s, 1H), 3.97 (s, 3H), 1.51 (s, 9H).

### Step 6: Preparation of Compound 1-8

Compound 1-7 (5.70 g, 17.21 mmol) was dissolved in acetone (85 mL), and then iodomethane (2.93 g, 20.65 mmol) and cesium carbonate (16.85 g, 51.64 mmol) were added. Then, the mixture was stirred at 15-25°C for 3 hours. The reaction mixture was quenched with water (50 mL) and extracted with ethyl acetate (50 mL × 2), and the separated organic layer was washed with a saturated brine solution (30 mL), dried over anhydrous sodium sulfate, filtered, and concentrated under reduced pressure to obtain Compound 1-8. LCMS (ESI) m/z: 344.9/446.9 (M+1).

### Step 7: Preparation of Compound 1-9

Compound 1-8 (1.8 g, 5.21 mmol), Compound 1-4 (2.1 g, 6.62 mmol), sodium carbonate (1.11 g, 10.43 mmol), (diphenylphosphinoferrocene) dichloropalladium (381.55 mg, 521.45 µmol), 2-dicyclohexylphosphino-2,6-dimethoxybiphenyl (214.07 mg, 521.45 µmol) and palladium acetate (58.54 mg, 260.73 µmol) were mixed in tetrahydrofuran (30 mL) and water (6 mL), the reaction was replaced with N₂ three times, and then the mixture was stirred in a nitrogen atmosphere at 60°C to 65°C for 10 hours. The reaction mixture was quenched with water (50 mL) and extracted with ethyl acetate (30 mL × 3), and the separated organic layer was washed with a saturated brine solution (20 mL), dried over anhydrous sodium sulfate, filtered, and concentrated under reduced pressure to obtain the residue. The residue was purified through silica gel column chromatography (petroleum ether/ethyl acetate = 20/1 to 5/1) to obtain Compound 1-9. LCMS (ESI) m/z: 456.1 (M+1).

### Step 8: Preparation of Compound 1-10

At 0°C, Compound 1-9 (0.50 g, 1.10 mmol) was dissolved in tetrahydrofuran (20 mL), and then lithium aluminum hydride (166.63 mg, 4.39 mmol) was added. Then, the mixture was stirred at 0°C for 2 hours. The reaction mixture was quenched with water (1 mL), dried over anhydrous sodium sulfate, filtered, and concentrated under reduced pressure to obtain Compound 1-10. LCMS (ESI) m/z: 300.2 (M+1).

### Step 9: Preparation of Compound 1-11

Compound 1-10 (0.30 g, 1.00 mmol) was dissolved in water (10 mL), and then concentrated sulfuric acid (18.4 g, 183.85 mmol) was added. Then, the mixture was stirred at 60°C for 12 hours. The reaction mixture was quenched with an aqueous saturated sodium carbonate solution (100 mL) and extracted with ethyl acetate (30 mL × 3), and the separated organic layer was washed with a saturated brine solution (20 mL), dried over anhydrous sodium sulfate, filtered, and concentrated under reduced pressure to obtain Compound 1-11. LCMS (ESI) m/z: 282.1 (M+1).

### Step 10: Preparation of Compound 1-12

Compound 1-11 (0.26 g, 924.11 µmol) and diethyl 2-(ethoxymethylene) malonate (0.20 g, 924.11 µmol) were dissolved in toluene (5 mL), and the reaction was replaced with nitrogen three times. The mixture was stirred at 110°C for 10 hours. The reaction mixture was concentrated under reduced pressure to obtain Compound 1-12. LCMS (ESI) m/z: 452.1 (M+1).

### Step 11: Preparation of Compound 1-13

Compound 1-12 (0.12 g, 265.77 µmol) was dissolved in polyphosphoric acid (1.00 g). Then, the mixture was stirred at 90°C for 2 hours. The reaction mixture was quenched with water (100 mL), the pH of the mixture was adjusted to 8-9 with potassium carbonate, then the mixture was extracted with dichloromethane (20 mL × 4), and the separated organic layer was washed with saturated brine solution (30 mL), dried over anhydrous sodium sulfate, filtered, and concentrated under reduced pressure to obtain Compound 1-13. LCMS (ESI) m/z: 406.1 (M+1).

### Step 12: Preparation of hydrochloride of Compound 1

Compound 1-13 (0.05 g, 123.32 µmol) was dissolved in tetrahydrofuran (1 mL), ethanol (1 mL) and water (0.5 mL), and then sodium hydroxide (14.80 mg, 369.96 µmol) was added. Then, the mixture was stirred at 15-25°C for 2 hours. The reaction mixture was adjusted to pH=5-6 with 1 M hydrochloric acid, filtered and concentrated under reduced pressure to obtain the residue. The residue was purified through preparative HPLC (chromatographic column: YMC Triart 30 × 150 mm × 7 µm; mobile phase: 0.05% of aqueous hydrochloric acid solution and acetonitrile; gradient: acetonitrile 12%-32%) to obtain hydrochloride of Compound 1. ¹H NMR (400 MHz, CD₃OD) δ = 9.08 (s, 1H), 8.64 (d, *J* = 8.4 Hz, 1H), 8.39 - 8.28 (m, 1H), 7.89 (d, *J* = 8.5 Hz, 1H), 7.26 (d, *J =* 9.4 Hz, 1H), 4.89 - 4.88 (m, 4H), 4.51 - 4.39 (m, 1H),3.18 (s, 3H), 1.50 - 1.44 (m, 2H), 1.09 - 1.03 (m, 2H). LCMS (ESI) m/z: 378.1 (M+1).

### Example 2

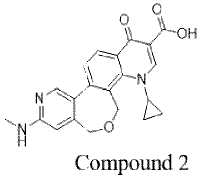

### Synthetic route:

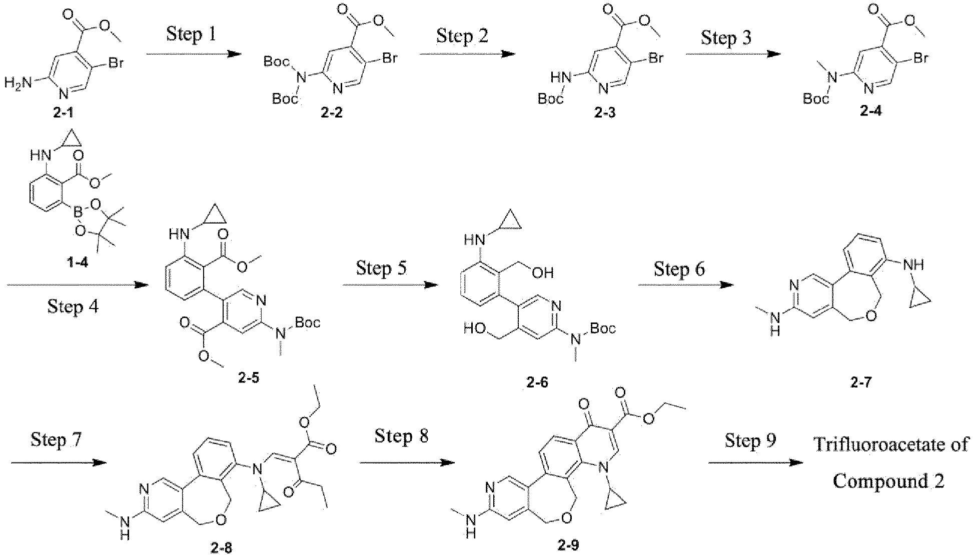

### Step 1: Preparation of Compound 2-2

Compound 2-1 (1.00 g, 4.33 mmol) was dissolved in dichloromethane (20 mL), then Boc₂O (2.36 g, 10.82 mmol) and DMAP (52.88 mg, 432.81 µmol) were added, and the mixture was stirred at 20-25°C for 2 hours. The reaction solution was washed with saturated sodium bicarbonate (50 mL) and saturated brine solution (50 mL), successively, dried over anhydrous sodium sulfate, filtered, and concentrated under reduced pressure to obtain Compound 2-2. LCMS (ESI) m/z: 374.9 (M-56+1).

### Step 2: Preparation of Compound 2-3

Potassium carbonate (1.44 g, 10.43 mmol) was added to a solution of Compound 2-2 (1.50 g, 3.48 mmol) in methanol (20 mL). The mixture was stirred at 20-25°C for 2 hours. The crude product was stirred in a mixed solvent (petroleum ether/ethyl acetate=1/1) and filtered to obtain Compound 2-3. ¹H NMR (400 MHz, CDCl₃) δ = 8.47 (s, 1H), 8.33 (s, 1H), 7.68 (br s, 1H), 3.98 (s, 3H), 1.56 (s, 9H). LCMS (ESI) m/z: 275.0 (M-56+1).

### Step 3: Preparation of Compound 2-4

Cesium carbonate (15.64 g, 48.01 mmol) and iodomethane (2.73 g, 19.21 mmol) were added to a solution of Compound 2-3 (5.3 g, 16.00 mmol) in acetone (80 mL). The mixture was stirred at 20-25°C for 3 hours, filtered and concentrated under reduced pressure to obtain a crude product. The crude product was purified through silica gel column chromatography (petroleum ether/ethyl acetate = 300/1 to 100/1) to obtain Compound 2-4. LCMS (ESI) m/z: 289.0 (M-56+1).

### Step 4: Preparation of Compound 2-5

Compound 1-4 (91.89 mg, 289.70 µmol), 2-dicyclohexylphosphino-2,6-dimethoxybiphenyl (23.79 mg, 57.94 µmol), sodium carbonate (61.41 mg, 579.39 µmol), and palladium acetate (6.50 mg, 28.97 µmol) were added to a solution of Compound 2-4 (0.1 g, 289.70 µmol) in tetrahydrofuran (2 mL) and water (0.4 mL), and then the reaction was replaced with nitrogen three times. Then the mixture was warmed to 70°C and stirred for 22 hours, filtered and concentrated under reduced pressure to obtain a crude product. The crude product was purified by thin-layer chromatography (petroleum ether/ethyl acetate=5/1) to obtain Compound 2-5. LCMS (ESI) m/z:456.1 (M+1).

### Step 5: Preparation of Compound 2-6

At 0°C, lithium aluminum hydride (316.63 mg, 8.34 mmol) was added to a solution of Compound 2-5 (950 mg, 2.09 mmol) in tetrahydrofuran (20 mL), and then the mixture was stirred at 0-10°C for 2 hours. The reaction solution was quenched with water (0.33 mL), 15% sodium hydroxide (0.33 mL) and water (1 mL), dried over anhydrous sodium sulfate, filtered, and concentrated to obtain Compound 2-6. LCMS (ESI) m/z: 344.2 (M-56+1).

### Step 6: Preparation of Compound 2-7

Compound 2-6 (500 mg, 1.67 mmol) was dissolved in a 50% aqueous sulfuric acid solution (10 mL), then warmed to 60 °C and stirred for 12 hours. The reaction solution was diluted with water (10 mL), adjusted to pH=9 with sodium carbonate, and extracted with ethyl acetate (20 mL). The reaction solution was purified by thin-layer chromatography (petroleum ether/ethyl acetate=1/1) to obtain Compound 2-7. LCMS (ESI) m/z: 282.2 (M+1).

### Step 7: Preparation of Compound 2-8

At 25°C, Compound 2-7(90.00 mg, 319.88 µmol) and diethyl 2-(ethoxymethylene) malonate (83.00 mg, 383.86 µmol) were dissolved in toluene (5 mL), warmed to 110°C, and stirred for 14 hours. The reaction mixture was concentrated to obtain Compound 2-8. LCMS (ESI)m/z: 452.3 (M+1).

### Step 8: Preparation of trifluoroacetate of Compound 2-9

Compound 2-8 (0.15 g, 332.22 µmol) was dissolved in polyphosphoric acid (88.59 µmol) solution, warmed to 90°C, and stirred 2 hours. The mixture was filtered, and the filtrate was purified through preparative HPLC (chromatographic column: Shim-pack C18 150*25 mm*10 µm; mobile phase: 0.05% aqueous trifluoroacetic acid solution-acetonitrile; gradient: acetonitrile: 12%-42%) to obtain trifluoroacetate of Compound 2-9. LCMS (ESI) m/z: 406.3 (M+1).

### Step 9: Preparation of trifluoroacetate of Compound 2

At 25°C, sodium hydroxide (2.96 mg, 73.99 µmol) was added to a solution of trifluoroacetate of Compound 2-9 (6 mg, 14.80 µmol) in methanol (0.3 mL) and water (0.1 mL). The mixture was stirred at 25°C for 2 hours and filtered, and the filtrate was purified through preparative HPLC (chromatographic column: 3_Phenomenex Luna C18 75*30 mm*3 µm; mobile phase: 0.05% aqueous trifluoroacetic acid solution-acetonitrile; gradient: acetonitrile: 12%-42%) to obtain trifluoroacetate of Compound 2. ¹H NMR (400 MHz, CD₃OD+D₂O) δ = 9.15 (s, 1H), 8.59 (d, *J =* 8.4 Hz, 1H), 8.31 (s, 1H), 7.89 (d, *J =* 8.4 Hz, 1H), 7.23 (s, 1H), 3.37 (s, 1H), 3.32 (s, 4H), 3.14 (s, 3H), 2.72 (s, 1H), 2.09 (s, 2H), 1.58 - 1.44 (m, 2H), 1.06 (br d, *J* = 2.3 Hz, 2H). LCMS (ESI) m/z:378.2 (M+1).

### Example 3

### Synthetic route:

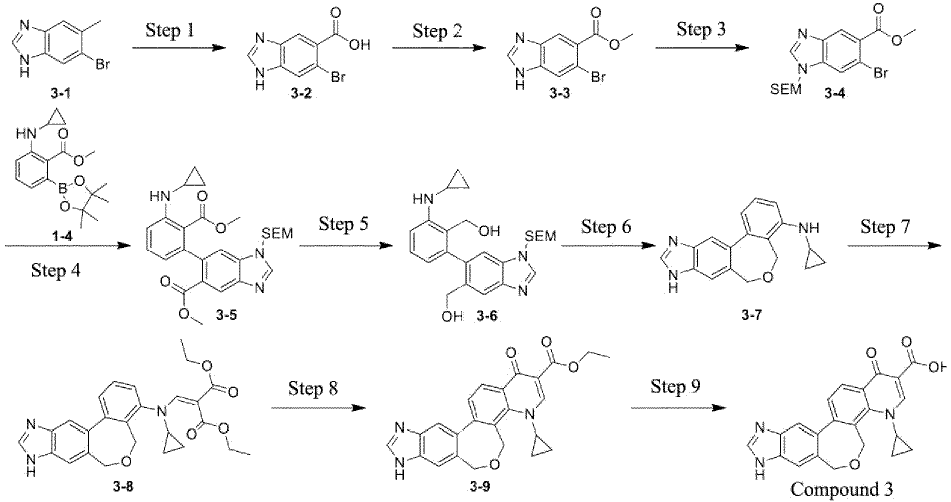

### Step 1: Preparation of Compound 3-2

Compound 3-1 (5.00 g, 23.69 mmol) was dissolved in tert-butanol (60 mL) and water (60 mL), and then potassium permanganate (18.72 g, 118.45 mmol) was added. Then, the mixture was stirred at 80°C for 6 hours. The reaction solution was cooled to room temperature, filtered, and washed with water (20 mL) and methyl tert-butyl ether (50 mL), respectively, an aqueous phase was separated and adjusted to pH=4 with 1 M hydrochloric acid, and then concentrated under reduced pressure to obtain Compound 3-2. LCMS (ESI) m/z: 241.0/243.0 (M+1).

### Step 2: Preparation of Compound 3-3

Sulfoxide chloride (4.94 g, 41.49 mmol) was added to a solution of Compound 3-2 (5 g, 20.74 mmol) in methanol (100 mL). The mixture was stirred at 70°C for 12 hours. Then the reaction mixture was quenched with water (30 mL), and concentrated under reduced pressure to remove methanol. The reaction mixture was extracted with ethyl acetate (50 mL), and the separated organic layer was washed with water (50 mL) and a saturated brine solution (50 mL), respectively, dried over anhydrous sodium sulfate, filtered, and concentrated under reduced pressure to obtain Compound 3-3. LCMS (ESI) m/z: 255.1/257.1 (M+1).

### Step 3: Preparation of Compound 3-4

Compound 3-3 (1.00 g, 3.92 mmol) was dissolved in DMF (20 mL), sodium hydride (235.21 mg, 5.88 mmol, purity: 60%) was added at 0°C, and the mixture was stirred at 0°C for 1 hour. Then, 2-(trimethylsilyl) ethoxymethyl chloride (980.45 mg, 5.88 mmol) was added, and the mixture was stirred at 20-30°C for 2 hours. The reaction mixture was quenched with water (20 mL) and extracted with ethyl acetate (20 mL × 2), and the separated organic layer was washed with a saturated brine solution (20 mL), dried over anhydrous sodium sulfate, filtered, and concentrated under reduced pressure to obtain the residue. The residue was purified through silica gel column chromatography (petroleum ether/ethyl acetate = 10/1 to 1/1) to obtain Compound 3-4. LCMS (ESI) m/z: 515.1/517.1 (M+1).

### Step 4: Preparation of Compound 3-5

Compound 3-4 (0.19 g, 493.09 µmol), Compound 1-4 (187.68 mg, 591.70 µmol), sodium carbonate (130.66 mg, 1.23 µmol) and (diphenylphosphinoferrocene) dichloropalladium (36.08 mg, 49.31 µmol) were mixed in dioxane (4 mL) and water (1 mL), and then the mixture was stirred in a microwave condition at 115°C for 1 hour. The reaction mixture was quenched with water (30 mL) and extracted with ethyl acetate (30 mL × 2), and the separated organic layer was washed with brine (20 mL), dried over anhydrous sodium sulfate, filtered, and concentrated under reduced pressure to obtain the residue. The residue was purified through silica gel column chromatography (petroleum ether/ethyl acetate = 10/1 to 1/1) to obtain Compound 3-5. LCMS (ESI) m/z: 496.3 (M+1).

### Step 5: Preparation of Compound 3-6

At 0°C, compound 3-5 (0.55 g, 1.11 mmol) was dissolved in tetrahydrofuran (50 mL), and then lithium aluminum hydride (168.45 mg, 4.44 mmol) was added. Then, the mixture was stirred at 0°C for 2 hours. The reaction mixture was quenched with water (4 mL), dried over anhydrous sodium sulfate, filtered, and concentrated under reduced pressure to obtain Compound 3-6. LCMS (ESI) m/z: 440.2 (M+1).

### Step 6: Preparation of Compound 3-7

Compound 3-6 (0.45 g, 1.02 mmol) was dissolved in water (5 mL), and then concentrated sulfuric acid (9.2 g, 91.93 mmol) was added. Then, the mixture was stirred at 60°C for 12 hours. The reaction mixture was quenched with an aqueous sodium carbonate solution (100 mL) and extracted with ethyl acetate (30 mL × 3), and the separated organic layer was washed with a saturated brine solution (20 mL), dried over anhydrous sodium sulfate, filtered, and concentrated under reduced pressure to obtain Compound 3-7. LCMS (ESI) m/z: 292.0 (M+1).

### Step 7: Preparation of Compound 3-8

Compound 3-7 (0.2 g, 686.47 µmol) and diethyl 2-(ethoxymethylene) malonate (178.12 mg, 823.76 µmol) was dissolved in N-methylpyrrolidone (4 mL), and the reaction was degassed and replaced with nitrogen three times. The mixture was stirred at 110°C for 16 hours. The reaction mixture was quenched with water (30 mL) and extracted with ethyl acetate (30 mL × 2), and the separated organic layer was washed with a saturated brine solution (20 mL), dried over anhydrous sodium sulfate, filtered, and concentrated under reduced pressure to obtain the residue. The residue was purified through silica gel column chromatography (petroleum ether/ethyl acetate = 10/1 to 0/1) to obtain Compound 3-8. LCMS (ESI) m/z: 462.1 (M+1).

### Step 8: Preparation of Compound 3-9

Compound 3-8 (0.12 g, 260.02 µmol) was dissolved in polyphosphoric acid (5.00 g). Then, the mixture was stirred at 80°C for 4 hours. The reaction mixture was quenched with an aqueous saturated sodium carbonate solution (100 mL) and extracted with ethyl acetate (30 mL × 2), and the separated organic layer was washed with a saturated brine solution (20 mL), dried over anhydrous sodium sulfate, filtered, and concentrated under reduced pressure to obtain Compound 3-9. LCMS (ESI) m/z: 416.3 (M+1).

### Step 9: Preparation of Compound 3

Compound 3-9 (0.02 g, 48.14 µmol) was dissolved in methanol (3 mL) and water (1 mL), and then lithium hydroxide monohydrate (4.04 mg, 96.28 µmol) was added. Then, the mixture was stirred at 20-30°C for 1 hour. The reaction mixture was adjusted to pH=5-6 with 1 M hydrochloric acid, then concentrated under reduced pressure to obtain the residue. The residue was purified through preparative HPLC (chromatographic column: Phenomenex Synergi C18 150 × 25mm × 10 µm; mobile phase: 0.225% aqueous formic acid solution-acetonitrile; gradient: acetonitrile: 8%-38%) to obtain Compound 3. ¹H NMR (400 MHz, DMSO-*d*₆) δ = 8.91 (s, 1H), 8.57 - 8.46 (m, 1H), 8.40 (s, 1H), 8.09 - 7.76 (m, 3H), 4.67 - 4.46 (m, 4H), 3.98 - 3.98 (m, 1H), 1.39 - 1.29 (m, 2H), 1.08 - 1.01 (m, 2H). LCMS (ESI) m/z: 388.1 (M+1).

### Example 4

### Synthetic route:

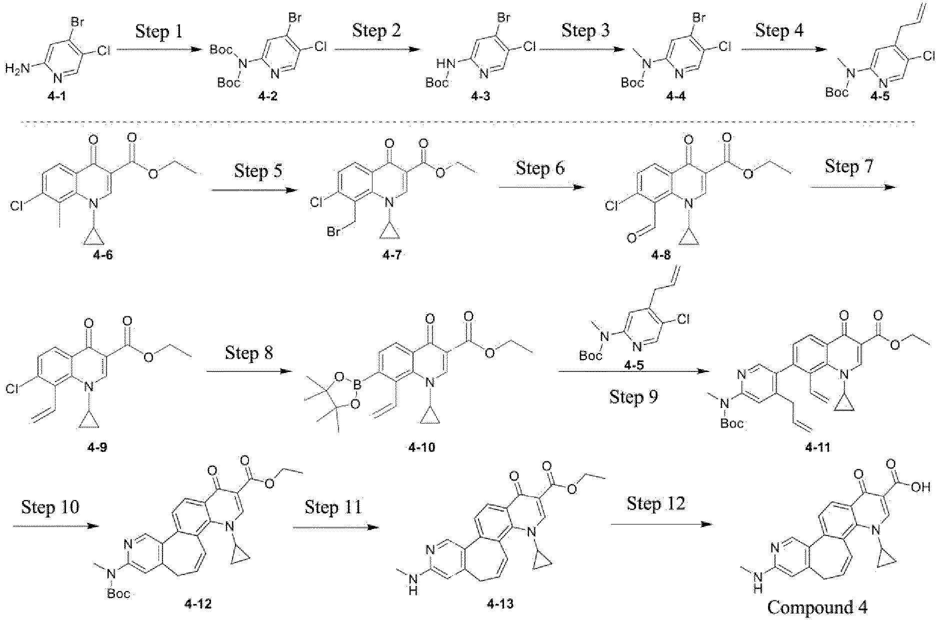

### Step 1: Preparation of Compound 4-2

Compound 4-1 (10.00 g, 48.20 mmol) was dissolved in dichloromethane (200 mL), and then (Boc)₂O (26.30 g, 120.51 mmol, 27.69 mL) and DMAP (588.90 mg, 4.82 mmol) were added thereto. Then, the mixture was stirred at 15-25°C for 2 hours. The reaction solution was washed with an aqueous saturated sodium bicarbonate solution (50 mL) and a saturated brine solution (50 mL), respectively, dried over anhydrous sodium sulfate, filtered, and concentrated under reduced pressure to obtain Compound 4-2. LCMS (ESI) m/z: 250.9/252.9 (M-100-56+1).

### Step 2: Preparation of Compound 4-3

Potassium carbonate (20.34 g, 147.17 mmol) was added to a solution of Compound 4-2 (20.00 g, 49.06 mmol) in methanol (100 mL). The mixture was stirred at 20-30°C for 2 hours. The mixture was concentrated under reduced pressure to remove methanol, then the reaction mixture was quenched with water (100 mL) and ethyl acetate (50 mL) and then filtered to collect the filter cake 1. The filtrate was extracted with ethyl acetate (30 mL × 2), the separated organic layer was washed with a saturated brine solution (30 mL), dried over anhydrous sodium sulfate, filtered, and concentrated under reduced pressure to obtain a crude product. The crude product was added into ethyl acetate (15 mL), stirred, and then filtered to collect the filter cake 2. The two filter cakes were combined, and dried to obtain Compound 4-3. LCMS (ESI) m/z: 250.9/252.9 (M-56+1).

### Step 3: Preparation of Compound 4-4

Compound 4-3 (13.2 g, 42.92 mmol) was dissolved in DMF (100 mL), and then iodomethane (7.31g, 51.50 mmol) and cesium carbonate (41.95 g, 128.75 mmol) were added. Then, the mixture was stirred at 20-30°C for 3 hours. The reaction mixture was quenched with water (200 mL) and extracted with ethyl acetate (100 mL × 2), and the separated organic layer was washed with a saturated brine solution (50 mL), dried over anhydrous sodium sulfate, filtered, and concentrated under reduced pressure to obtain Compound 4-4. LCMS (ESI) m/z: 264.9/266.9 (M-56+1).

### Step 4: Preparation of Compound 4-5

Compound 4-4 (4.94 g, 14.93 mmol), allyltributylstannane (4 g, 12.44 mmol), cesium fluoride (4.53 g, 29.85 mmol) and tetrakis(triphenylphosphine) palladium (1.15 g, 995.03 µmol) were mixed in dioxane (40 mL), the reaction was replaced with N₂ three times, and then the mixture was stirred in a nitrogen atmosphere at 70°C for 5 hours. The reaction mixture was quenched with an aqueous potassium fluoride solution (50 mL), stirred for 1 hour and then filtered. The filtrate was extracted with ethyl acetate (50 mL × 3), and the separated organic layer was washed with a saturated brine solution (50 mL), dried over anhydrous sodium sulfate, filtered, and concentrated under reduced pressure to obtain the residue. The residue was purified through silica gel column chromatography (petroleum ether/ethyl acetate = 1/0 to 10/1) to obtain Compound 4-5. LCMS (ESI) m/z: 227.1/229.1 (M-56+1).

### Step 5: Preparation of Compound 4-7

Compound 4-6 (1.00 g, 3.27 mmol) was dissolved in chloroform (10 mL), N-bromosuccinimide (698.53 mg, 3.92 mmol) and azobisisobutyronitrile (53.71 mg, 0.33 mmol) were added, and the mixture was stirred at 80°C for 16 hours. Then the reaction solution was concentrated under reduced pressure to obtain the residue. The residue was purified through silica gel column chromatography (petroleum ether/ethyl acetate = 1/1 to 1/2) to obtain Compound 4-7. ¹H NMR (400 MHz, CD₃OD) δ ppm= 8.90 (s, 1 H), 8.36 (d, *J =* 8.75 Hz, 1 H), 7.61 (d, *J =* 8.76 Hz, 1 H), 4.32 - 4.41 (m, 3 H), 2.71 (s, 2 H), 1.40 (t, *J =* 7.07 Hz, 7 H).

### Step 6: Preparation of Compound 4-8

Compound 4-7 (2.2 g, 5.72 mmol) was dissolved in acetonitrile (60 mL), and then 4 Å molecular sieve (4.4 g) and N-methylmorpholine-oxide (1.34 g, 11.44 mmol) were added. The mixture was stirred at 20-30°C for 2 hours. The reaction mixture was quenched with water (100 mL) and extracted with ethyl acetate (50 mL × 2), and the separated organic layer was washed with a saturated brine solution (50 mL), dried over anhydrous sodium sulfate, filtered, and concentrated under reduced pressure to obtain Compound 4-8. LCMS (ESI) m/z: 320.1 (M+1).

### Step 7: Preparation of Compound 4-9

Methyl triphenyl phosphonium bromide (2.85 g, 7.98 mmol) was dissolved in tetrahydrofuran (80 mL), then potassium tert-butoxide (1 M, 7.98 mL) was added at 0°C, and the mixture was stirred at 15-25°C for 0.5 hours. Then, Compound 4-8 (1.7 g, 5.32 mmol) was added, and the mixture was stirred at 15-25°C for 2 hours. The reaction mixture was quenched with water (200 mL) and extracted with ethyl acetate (50 mL × 3), and the separated organic layer was washed with a saturated brine solution (50 mL), dried over anhydrous sodium sulfate, filtered, and concentrated under reduced pressure to obtain the residue. The residue was purified through silica gel column chromatography (petroleum ether/ethyl acetate = 10/1 to 1/1) to obtain Compound 4-9. LCMS (ESI) m/z: 318.1 (M+1).

### Step 8: Preparation of Compound 4-10

Compound 4-9 (1.5 g, 4.72 mmol), 2-di-tert-butylphosphino-2',4',6'-triisopropylbiphenyl (225.03 mg, 472.05 µmol), tricyclohexyl phosphine (132.38 mg, 472.05 µmol), bis(pinacolato) diboron (1.80 g, 7.08 mmol), potassium acetate (926.53 mg, 9.44 mmol) and tris(dibenzylideneacetone) dipalladium (432.26 mg, 472.05 µmol) were mixed in dioxane (10 mL), the reaction was replaced with N₂ three times, and then the mixture was stirred in a nitrogen atmosphere at 100°C for 2 hours. The reaction mixture was quenched with water (50 mL) and extracted with ethyl acetate (30 mL × 3), and the separated organic layer was washed with a saturated brine solution (20 mL), dried over anhydrous sodium sulfate, filtered, and concentrated under reduced pressure to obtain the residue. The residue was purified through silica gel column chromatography (petroleum ether/ethyl acetate = 10/1 to 1/1) to obtain Compound 4-10. LCMS (ESI) m/z: 410.3 (M+1).

### Step 9: Preparation of Compound 4-11

Compound 4-10 (0.5 g, 1.22 mmol), Compound 4-5 (345.44 mg, 1.12 mmol), sodium carbonate (337.68 g, 2.44 mmol), 2-di-tert-butylphosphino-2',4',6'-triisopropylbiphenyl (58.24 mg, 122.17 µmol), tricyclohexyl phosphine (34.26 mg, 122.17 µmol) and tris(dibenzylideneacetone) dipalladium (111.87 mg, 122.17 µmol) were mixed in dioxane (10 mL) and water (2 mL), the reaction was replaced with N₂ three times, and then the mixture was stirred in a nitrogen atmosphere at 100°C for 1.5 hours. The reaction mixture was quenched with water (50 mL) and extracted with ethyl acetate (30 mL × 3), and the separated organic layer was washed with a saturated brine solution (20 mL), dried over anhydrous sodium sulfate, filtered, and concentrated under reduced pressure to obtain the residue. The residue was purified through preparative HPLC (chromatographic column: Phenomenex luna C18 150 × 40 mm × 15 µm; mobile phase: 0.225% aqueous formic acid solution-acetonitrile; gradient: acetonitrile: 57%-87%) to obtain Compound 4-11. LCMS (ESI) m/z: 530.2 (M+1). ¹H NMR (400 MHz, CDCl₃) δ = 8.70 (s, 1H), 8.47 (d, *J =* 8.3 Hz, 1H), 8.13 - 8.06 (m, 1H), 7.66 (s, 1H), 7.22 (d, *J =* 8.0 Hz, 1H), 6.92 - 6.81 (m, 1H), 5.75 (tdd, *J =* 6.6, 10.2, 16.9 Hz, 1H), 5.34 - 5.26 (m, 1H), 5.09 - 4.83 (m, 1H), 4.43 (q, *J =* 7.0 Hz, 2H), 3.84 - 3.74 (m, 1H), 3.45 (s, 3H), 3.18 - 3.00 (m, 2H), 1.58 - 1.55 (m, 9H), 1.44 (t, *J=* 7.1 Hz, 3H), 1.30 - 1.24 (m, 3H), 1.15 - 1.08 (m, 2H), 0.91 - 0.84 (m, 2H).

### Step 10: Preparation of Compound 4-12

Compound 4-11 (0.04 g, 75.53 µmol) was dissolved in dichloromethane (2 mL), and then Hoveyda-Grubbs 2^{nd} generation catalyst (4.73 mg, 7.55 µmol) was added. Then, the mixture was stirred at 40°C for 12 hours. The reaction mixture was concentrated under reduced pressure to obtain the residue. The residue was purified through silica gel column chromatography (petroleum ether/ethyl acetate = 10/1 to 0/1) to obtain Compound 4-12. LCMS (ESI) m/z: 502.3 (M+1).

### Step 11: Preparation of Compound 4-13

Compound 4-12 (0.05 g, 99.69 µmol) was dissolved in dioxane (0.5 mL), and then hydrochloric acid/dioxane solution (4 M, 0.5 mL) was added. Then, the mixture was stirred at 10-20°C for 2 hours. The reaction mixture was concentrated under reduced pressure to obtain Compound 4-13. LCMS (ESI) m/z: 402.2 (M+1).

### Step 12: Preparation of Compound 4

Compound 4-13 (0.04 g, 99.64 µmol) was dissolved in methanol (1 mL) and water (0.2 mL), and then sodium hydroxide (7.97 mg, 199.27 µmol) was added. Then, the mixture was stirred at 10-20°C for 2 hours. The reaction mixture was adjusted to pH=5-6 with acetic acid, then concentrated under reduced pressure to obtain the residue. The residue was purified through preparative HPLC (chromatographic column: Phenomenex luna C18 150 × 25 mm × 10 µm; mobile phase: 0.225% aqueous formic acid solution-acetonitrile; gradient: acetonitrile: 4%-34%) to obtain Compound 4. ¹H NMR (400 MHz, DMSO-*d*₆) δ = 15.09 (s, 1H), 8.89 (s, 1H), 8.38 (s, 1H), 8.28 (d, *J =* 8.8 Hz, 1H), 7.93 (d, *J =* 8.8 Hz, 1H), 7.08 (d, *J =* 9.8 Hz, 1H), 6.86 (br d, *J* = 4.9 Hz, 1H), 6.39 (s, 1H), 6.16 (td, *J=* 7.1, 9.8 Hz, 1H), 4.37 (br t, *J* = 4.4 Hz, 1H), 3.45 - 3.39 (m, 2H), 2.83 (d, *J* = 4.9 Hz, 3H), 1.28 - 0.77 (m, 4H). LCMS (ESI) m/z: 374.1 (M+1).

### Example 5

### Synthetic route:

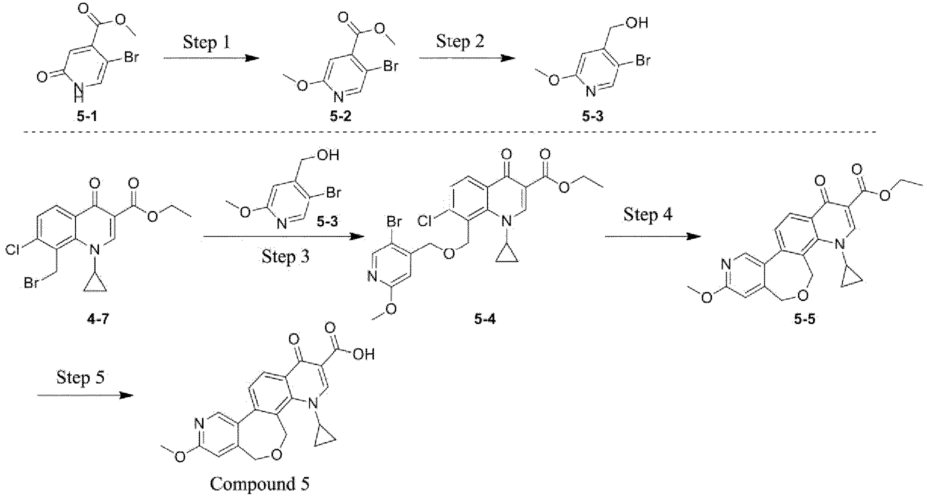

### Step 1: Preparation of Compound 5-2

Silver carbonate (5.41 g, 19.61 mmol) was added to a solution of Compound 5-1 (3.50 g, 15.08 mmol) and iodomethane (3.21 g, 22.63 mmol, 1.41 mL) in toluene (50 mL). The mixture was stirred at 100°C for 1.5 hours. Then the mixture was cooled to room temperature, diluted with ethyl acetate (50 mL) and filtered, the mother liquor was concentrated to obtain the residue. The residue was purified through silica gel column chromatography (petroleum ether/ethyl acetate = 20/1 to 10/1) to obtain Compound 5-2. LCMS (ESI) m/z: 246/248 (M+1); ¹H NMR (400 MHz, CDCl₃) δ= 8.37 (s, 1 H), 7.10 (s, 1 H), 3.97 (s, 3 H), 3.95 (s, 3 H).

### Step 2: Preparation of Compound 5-3

At 0°C, lithium aluminum hydride (466.61 mg, 12.29 mmol) was added in batches to a solution of Compound 5-2 (2.75 g, 11.18 mmol) in tetrahydrofuran (40 mL). The mixture was stirred at 0°C for 0.5 hours. At 0°C, the reaction mixture was quenched with sodium sulfate decahydrate (5.00 g) and then filtered, the obtained mother liquor was concentrated under reduced pressure to obtain the residue. The residue was purified through silica gel column chromatography (petroleum ether/ethyl acetate = 5/1 to 3/1) to obtain Compound 5-3. LCMS (ESI) m/z: 218/220 (M+1).

### Step 3: Preparation of Compound 5-4

At 0°C, sodium hydride (238.46 mg, 5.96 mmol, purity:60%), followed by Compound 4-7 (1.76 g, 4.59 mmol), was added to a solution of Compound 5-3 (1.00 g, 4.59 mmol) in tetrahydrofuran (15 mL), and then the mixture was stirred at 0°C for 3.5 hours. The reaction was quenched with an aqueous saturated ammonium chloride solution (15 mL) and then extracted with ethyl acetate (15 mL × 2). The combined organic layer was washed with brine (15 mL), dried over anhydrous sodium sulfate, filtered, and concentrated under reduced pressure to obtain the residue. The residue was purified through silica gel column chromatography (petroleum ether/ethyl acetate = 2/1 to 1/1) to obtain Compound 5-4. LCMS (ESI) m/z: 521/523 (M+1).

### Step 4: Preparation of Compound 5-5

Compound 5-4 (0.1 g, 0.19 mmol), bis(pinacolato) diboron (97.33 mg, 0.38 mmol), potassium acetate (37.62 g, 0.38 mmol), bis(triphenylphosphinoferrocene) dichloropalladium (14.02 mg, 19.16 µmol) were mixed in ethylene glycol dimethyl ether (2 mL), the reaction was replaced with N₂ three times, and then the mixture was stirred in a nitrogen atmosphere at 80°C for 16 hours. Bis(triphenylphosphinoferrocene)dichloropalladium (14.02 mg, 19.16 µmol), sodium carbonate (40.62 mg, 0.38 mmol) and water (0.2 mL) continued to be added into the reaction mixture, and then the mixture was stirred in a nitrogen atmosphere at 80°C for 20 hours. The reaction mixture was concentrated under reduced pressure to remove the solvent and obtain the residue. The residue was purified through silica gel column chromatography (petroleum ether/ethyl acetate = 1/1 to 0/1) to obtain Compound 5-5. LCMS (ESI) m/z: 407 (M+1).

### Step 5: Preparation of Compound 5

At 20°C, lithium hydroxide monohydrate (10.32 mg, 246.04 µmol) was added to a solution of Compound 5-5 (25 mg, 61.51 µmol) in methanol (1 mL) and water (0.25 mL). The mixture was stirred at 20°C for 3 hours and concentrated under reduced pressure, and the resulting residue was purified through preparative HPLC (chromatographic column: Phenomenex C1875× 30mm× 3µm; mobile phase: 0.225% aqueous formic acid solution-acetonitrile; gradient: acetonitrile: 32%-62%) to obtain Compound 5. LCMS (ESI) m/z: 309 (M+1); ¹H NMR (400 MHz, DMSO-*d*₆) δ= 8.88 - 8.97 (m, 1 H), 8.61 - 8.68 (m, 1 H), 8.44 - 8.53 (m, 1 H), 7.90 - 8.01 (m, 1 H), 7.10 - 7.17 (m, 1 H), 4.66 - 4.78 (m, 2 H), 4.44 - 4.59 (m, 3 H), 3.95 - 4.02 (m, 3 H), 1.30 - 1.38 (m, 2 H), 1.04 (br d, *J =* 8.19 Hz, 2H).

### Example 6

### Synthetic route:

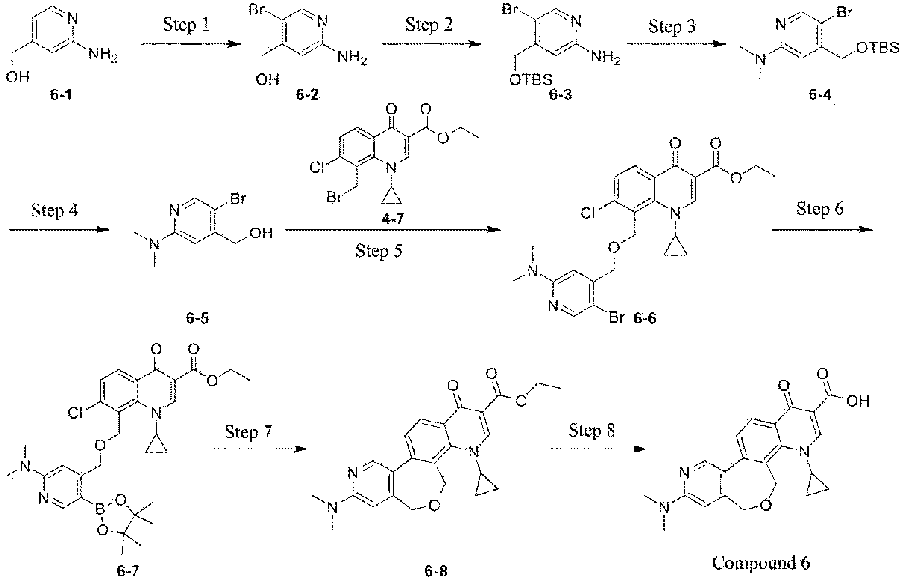

### Step 1: Preparation of Compound 6-2

Compound 6-1 (1.72 g, 13.86 mmol) was dissolved in acetonitrile (17.2 mL), then NBS (2.47 g, 13.86 mmol) was added at 0-20°C, and the mixture was stirred in a nitrogen atmosphere for 3 hours. The reaction solution was quenched with water (20 mL), and then diluted with ethyl acetate (20 mL). The diluted reaction solution was extracted with ethyl acetate (20 mL × 2), the combined organic phase was washed with a saturated brine solution (10 mL× 2), dried over anhydrous sodium sulfate, filtered, and concentrated under reduced pressure, and the residue was purified by silica gel column chromatography (petroleum ether/ethyl acetate=1/0 to 1/1) to obtain Compound 6-2. LCMS (ESI) m/z: 203.05 (M+1). ¹H NMR (400 MHz, DMSO-*d*₆) δ = 7.87 (s, 1H), 6.67 (s, 1H), 6.11 (s, 2H), 5.46 (t, *J =* 5.6 Hz, 1H), 4.35 (d, *J =* 5.6 Hz, 2H).

### Step 2: Preparation of Compound 6-3

Compound 6-2 (30 g, 147.76 mmol) and imidazole (15.09 g, 221.64 mmol) were dissolved in DMF (250 mL) solution, then TBSCl (33.40 g, 221.64 mmol, 27.16 mL) was dissolved in DMF (50 mL), which was slowly dropped into the reaction solution at 25°C within 20 minutes, and stirred at 25°C for 3 hours. The reaction solution was diluted with water (100 mL) and then extracted with ethyl acetate (50 mL × 2), and the combined organic phase was washed with saturated brine solution (50 mL × 2), dried over anhydrous sodium sulfate, filtered and concentrated under reduced pressure to obtain the crude product. The crude product was stirred in petroleum ether (100 mL) and then filtered to collect the filter cake. The filter cake was dried to obtain Compound 6-3. LCMS (ESI) m/z: 319.1 (M+1).

### Step 3: Preparation of Compound 6-4

NaH (1.51 g, 37.82 mmol) and iodomethane (6.71 g, 47.27 mmol, 2.94 mL) were added successively to a solution of Compound 6-3 (3 g, 9.45 mmol) in DMF (30 mL). The mixture was stirred at 0°C for 5 hours. The reaction solution was quenched with a saturated ammonium chloride solution (50 mL), then diluted with water (20 mL) and extracted with ethyl acetate (20 mL × 2), and the combined organic phase was washed with a saturated brine solution (20 mL × 2), dried over anhydrous sodium sulfate, filtered and concentrated under reduced pressure to obtain the crude product. The crude product was purified through silica gel column chromatography (petroleum ether/ethyl acetate = 100/1) to obtain compound 6-4. LCMS (ESI) m/z: 346.9 (M+1).

### Step 4: Preparation of Compound 6-5

Triethylamine hydrofluoride (4.48 g, 27.80 mmol, 4.53 mL) was added to a solution of Compound 6-4 (1.92 g, 5.56 mmol) in dichloromethane (20 mL), and then the mixture was stirred in a nitrogen atmosphere at 25°C for 2 hours. The reaction solution was diluted with water (5 mL) and then extracted with ethyl acetate (5 mL × 2), the combined organic phase was washed with a saturated brine solution (5 mL × 2), dried over anhydrous sodium sulfate, filtered and concentrated under reduced pressure to obtain a crude product. The crude product was purified through silica gel column chromatography (petroleum ether/ethyl acetate = 100/1 to 5/1) to obtain Compound 6-5. ¹H NMR (400 MHz, DMSO-*d*₆) δ = 8.09 (s, 1H), 6.83 (s, 1H), 4.44 (s, 2H), 3.05 (s, 6H).

### Step 5: Preparation of Compound 6-6

At 0°C, sodium hydride (328.88 mg, 8.22 mmol, purity:60%) and Compound 4-7 (1.58 g, 4.11 mmol) were successively added to a solution of Compound 6-5 (0.95 g, 4.11 mmol) in DMF (10 mL), and then the mixture was stirred for 6 hours. The reaction solution was quenched with a saturated ammonium chloride solution (10 mL), then diluted with water (10 mL) and extracted with ethyl acetate (10 mL × 2), and the combined organic phase was washed with saturated brine solution (10 mL), dried over anhydrous sodium sulfate, filtered and concentrated under reduced pressure to obtain a crude product. The crude product was purified through silica gel column chromatography (petroleum ether/ethyl acetate = 100/1 to 1/2) to obtain Compound 6-6. ¹H NMR (400 MHz, DMSO-*d*₆) δ = 8.60 (s, 1H), 8.14 (d, *J =* 8.6 Hz, 1H), 8.02 (s, 1H), 7.57 (d, *J =* 8.7 Hz, 1H), 6.51 (s, 1H), 5.39 (s, 2H), 4.44 (s, 2H), 4.24 (q, *J =* 7.1 Hz, 3H), 2.94 (s, 6H), 1.29 (t, *J =* 7.1 Hz, 3H), 1.17 (br d, *J =* 7.1 Hz, 3H), 0.90 - 0.85 (m, 2H).

### Step 6: Preparation of Compound 6-7

At room temperature, Compound 6-6 (0.5 g, 934.88 µmol), bis(pinacolato) diboron (712.20 mg, 2.80 mmol), potassium pivalate (327.73 mg, 2.34 mmol), and tetrakis(triphenylphosphine) palladium (108.03 mg, 93.49 µmol) were dissolved in ethylene glycol dimethyl ether (5 mL), and then the mixture was warmed to 90°C, and stirred for 18 hours. The reaction solution was diluted with water (10 mL) and then extracted with ethyl acetate (20 mL). The obtained organic phase was washed with a saturated brine solution (10 mL), dried over anhydrous sodium sulfate, filtered and concentrated under reduced pressure. The crude product was purified through silica gel column chromatography (petroleum ether/ethyl acetate = 100/1 to 1/1) to obtain Compound 6-7. LCMS (ESI) m/z: 582.3 (M+1).

### Step 7: Preparation of trifluoroacetate of Compound 6-8

Compound 6-7 (390 mg, 670.23 µmol), potassium carbonate (231.58 mg, 1.68 mmol) and tetrakis(triphenylphosphine) palladium (24.71 mg, 67.02 µmol), 2-dicyclohexylphosphino-2,4,6-triisopropylbiphenyl (63.90 mg, 134.05 µmol) were dissolved in water (0.4 mL) and ethylene glycol dimethyl ether (4 mL), and then the mixture was warmed to 90°C and stirred for 15 hours. The reaction solution was diluted with water (5 mL) and then extracted with ethyl acetate (5 mL × 2), and the combined organic phase was washed with a saturated brine solution (5 mL), dried over anhydrous sodium sulfate, filtered and concentrated to obtain a crude product. The crude product was purified through preparative HPLC (chromatographic column: Phenomenex Synergi Polar-RP 100*25mm*4µm; mobile phase: 0.05% aqueous trifluoroacetic acid solution-acetonitrile; gradient: acetonitrile: 24%-44%) to obtain trifluoroacetate of Compound 6-8. LCMS (ESI) m/z: 420.2 (M+1).

### Step 8: Preparation of Compound 6 and hydrochloride of Compound 6

Method 1: at 0°C, sodium hydroxide (14.30 mg, 357.59 µmol) was added to a mixed solution of Compound 6-8 (30 mg, 71.52 µmol) in methanol (0.6 mL) and water (0.3 mL), and then the mixture was stirred in a nitrogen atmosphere at 20°C for 15 hours. The mixture was filtered, and the filtrate was purified through preparative HPLC (chromatographic column: Waters Xbridge 150*25mm* 5µm; mobile phase: 10 mM aqueous ammonium bicarbonate solution- acetonitrile; gradient: acetonitrile: 24%-54%) to obtain Compound 6. LCMS (ESI) m/z: 392.1 (M+1). ¹H NMR (400 MHz, DMSO-*d*₆) δ = 8.90 (s, 1H), 8.47 (d, *J =* 8.4 Hz, 1H), 8.41 (s, 1H), 7.89 (d, *J =* 8.4 Hz, 1H), 7.51 (s, 1H), 4.77 (br s, 2H), 4.56 - 4.52 (m, 2H), 3.91 - 4.02 (m, 1H), 3.15 (s, 6H), 1.22-1.45 (m, 4H).

Method 2: at 0°C, an aqueous solution (10 mL) of sodium hydroxide (600.76 mg, 15.02 mmol) was added to Compound 6-8 (0.63 g, 1.50 mmol) in methanol (0.6 mL), and then the mixture was stirred at 20-30°C for 15 hours and filtered, and the filter cake was collected to obtain a crude product. At room temperature, the above crude product was added to a mixed solution of hydrochloric acid (12 M, 122.63 µL) and water (4 mL), and stirred for 16 hours. The reaction solution was filtered, and then the filter cake was collected and dried to obtain hydrochloride of Compound 6. LCMS (ESI) m/z: 392.1 (M+1).

### Example 7

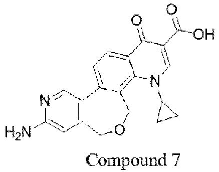

### Synthetic route:

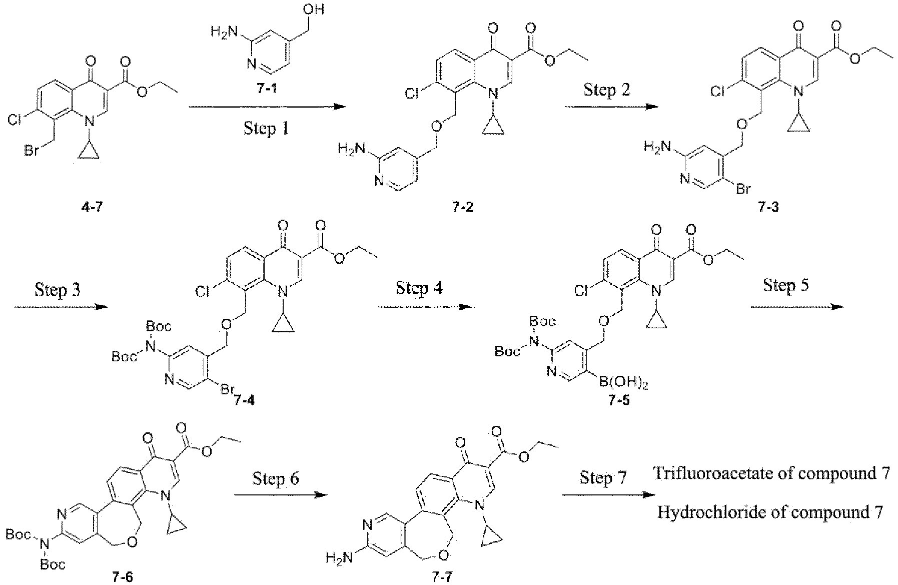

### Step 1: Preparation of Compound 7-2

At 0°C, sodium hydride (322.22 mg, 8.06 mmol) was added to a solution of Compound 7-1 (0.50 g, 4.03 mmol) in DMF (5 mL), 10 minutes later, Compound 4-7 was added to the reaction solution and stirred for 6 hours. The reaction solution was quenched with a saturated ammonium chloride solution (10 mL) and then extracted with ethyl acetate (10 mL), an organic layer was washed with a saturated brine solution (10 mL), dried over anhydrous sodium sulfate, filtered and concentrated under reduced pressure to obtain a crude product. The crude product was purified through silica gel column chromatography (dichloromethane/methanol = 100/3) to obtain Compound 7-2. LCMS (ESI) m/z: 428.2 (M+1).

### Step 2: Preparation of Compound 7-3

At 0°C, bromosuccinimide (0.61 g, 3.41 mmol) was added to a solution of Compound 7-2 (1.46 g, 3.41 mmol) in dichloromethane (20 mL), and the mixture was stirred at 0°C for 2 hours. The reaction solution was concentrated, diluted with water (10 mL), and then extracted with dichloromethane (10 mL × 2). The combined organic phase was washed with a saturated brine solution (10 mL × 2), dried over anhydrous sodium sulfate, filtered and concentrated under reduced pressure to obtain a crude product. The crude product was purified through silica gel column chromatography (petroleum ether/ethyl acetate = 1/0 to 100/3) to obtain Compound 7-3. LCMS (ESI) m/z: 508.1 (M+1).

### Step 3: Preparation of Compound 7-4

Boc₂O (0.42 mg, 1.93 mmol, 0.44 mL) and 4-dimethylaminopyridine (9.42 mg, 77.12 µmol) were added to a solution of Compound 7-3 (390.85 mg, 771.24 µmol) in dichloromethane (6 mL) and stirred at 20-25°C for 2 hours. The reaction solution was diluted with water (5 mL) and then extracted with dichloromethane (5 mL × 2), and the combined organic phase was washed with a saturated brine solution (5 mL × 2), dried over anhydrous sodium sulfate, filtered and concentrated under reduced pressure to obtain Compound 7-4. LCMS (ESI) m/z:708.1 (M+1).

### Step 4: Preparation of Compound 7-5

Bis(pinacolato) diboron(35.92 mg, 141.44 µmol), potassium pivalate (24.79 mg, 176.80 µmol) and tetrakis(triphenylphosphine) palladium (2.61 mg, 7.07 µmol) were added successively to a solution of Compound 7-4 (0.05 g, 70.72 µmol) in ethylene glycol dimethyl ether (1 mL), and stirred at 90°C for 15 hours. The resulting reaction solution containing the crude Compound 7-5 was directly used for the next step. LCMS (ESI) m/z: 672.3 (M+1).

### Step 5: Preparation of Compound 7-6

The crude Compound 7-5 (47.52 mg, 70.72 µmol), potassium carbonate (24.44 mg, 176.80 µmol) and tetrakis(triphenylphosphine) palladium (2.61mg, 7.07 µmol), 2-dicyclohexylphosphino-2,4,6-triisopropylbiphenyl (6.74 mg, 14.14 µmol) were dissolved in water (0.1 mL) and ethylene glycol dimethyl ether (1 mL), and the reaction was replaced with nitrogen for three times, then warmed to 90°C and stirred for 15 hours. The reaction solution was filtered, concentrated under reduced pressure to obtain Compound 7-6. LCMS (ESI) m/z: 592.2 (M+1).

### Step 6: Preparation of trifluoroacetate of Compound 7-7 and Compound 7-7

Trifluoroacetic acid (821.20 mg, 7.20 mmol, 533.25 µL) was added to a solution of Compound 7-6 (41.84 mg, 70.72 µmol) in dichloromethane (1 mL), and then the mixture was stirred for 2 hours. Multi-batch post-processing, Method 1: the reaction solution was concentrated under reduced pressure to obtain trifluoroacetate of Compound 7-7; Method 2: the reaction solution was concentrated under reduced pressure to obtain a crude product, the crude product was adjusted to pH=8 with saturated sodium carbonate solution, and extracted with dichloromethane, and organic phases were combined, and concentrated under reduced pressure to obtain Compound 7-7. LCMS (ESI) m/z: 392.1 (M+1).

### Step 7: Preparation of trifluoroacetate of Compound 7 and hydrochloride of Compound 7

Method 1: at 0°C, sodium hydroxide (26.52 mg, 662.97 µmol) was added to a solution of crude Compound 7-7 (25.95 mg, 66.30 µmol) in methanol (1 mL) and water (0.5 mL), and then the mixture was stirred in a nitrogen atmosphere at 20°C for 15 hours. The mixture was concentrated under reduced pressure to obtain a crude product, and then the crude product was purified through preparative HPLC (chromatographic column: Phenomenex Synergi Polar-RP 100*25mm*4µm; mobile phase: 0.05% aqueous trifluoroacetic acid solution-acetonitrile; gradient: acetonitrile: 17%-37%) to obtain trifluoroacetate of Compound 7. LCMS (ESI) m/z: 364.1 (M+1). ¹H NMR (400 MHz, DMSO-*d*₆) δ = 8.90 (s, 1H), 8.47 (d, *J=* 8.4 Hz, 1H), 8.41 (s, 1H), 7.89 (d, *J= 8.4* Hz, 1H), 6.91 (s, 1H), 4.77 (br s, 2H), 4.51 (s, 3H), 1.32 (br d, *J* = 6.4 Hz, 2H), 1.02 (br s, 2H).

Method 2: at room temperature, an aqueous solution (5 mL) of sodium hydroxide (102.18 mg, 2.55 mmol) was added to a solution of crude Compound 7-7 (1 g, 2.55 mmol) in methanol (10 mL), and then the mixture was stirred at 20-50°C for 36 hours. The mixture was filtered, and the filter cake was collected to obtain a crude product. Hydrochloric acid (12 M, 2 mL) was added to the aqueous solution (4 mL) of the above crude product, and the mixture was stirred for 16 hours. The reaction solution was filtered, and then the filter cake was collected, and dried to obtain hydrochloride of Compound 7. LCMS (ESI) m/z: 364.1 (M+1). ¹H NMR (400 MHz, DMSO-*d*₆) δ = 8.91 (s, 1H), 8.49 (d, J = 8.3 Hz, 1H), 8.42 (s, 1H), 7.89 (d, J = 8.4 Hz, 1H), 7.05 (s, 1H), 4.81 (s, 2H), 4.55 (s, 2H), 4.52 - 4.43 (m, 1H), 1.36 - 1.28 (m, 2H), 1.07 - 0.99 (m, 2H).

### Example 8

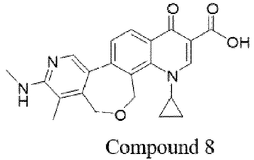

### Synthetic route:

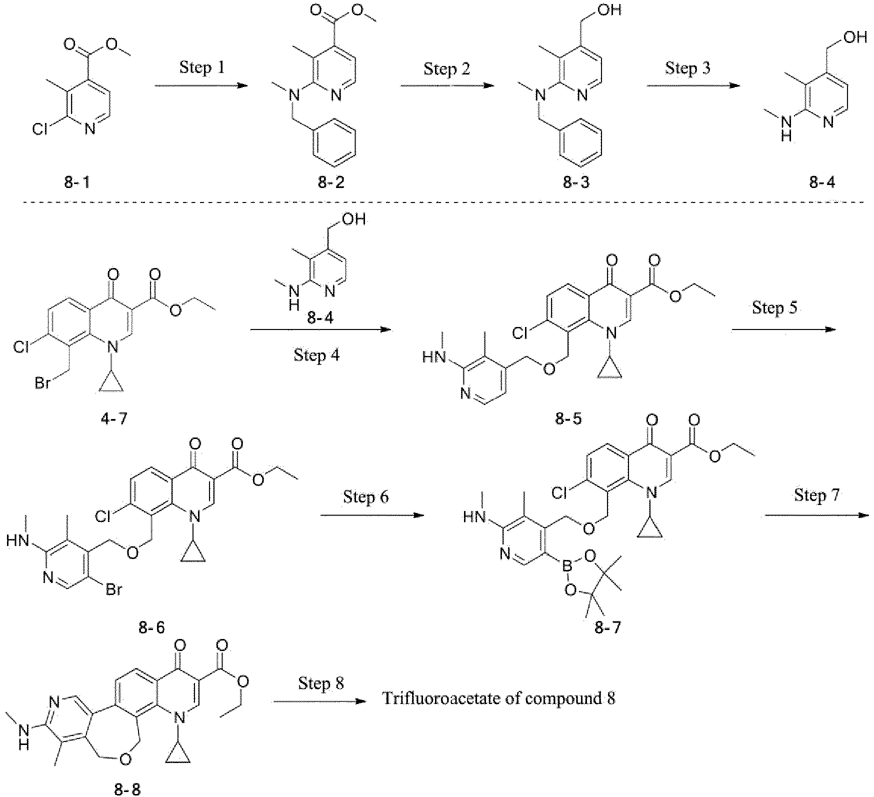

### Step 1: Preparation of Compound 8-2

At 20°C, Compound 8-1 (1.00 g, 5.39 mmol) and N-methylbenzylamine (979.32 mg, 8.08 mmol, 1.04 mL) were dissolved in dioxane (20 mL), then cesium carbonate (5.27 g, 16.16 mmol) and [(tri-tert-butylphosphine)-2-(2-aminobiphenyl) palladium (II) chloride (276.07 mg, 538.77 µmol) were added, the mixture was stirred at 90°C for 16 hours, and the compound was filtered, concentrated and passed through silica gel column chromatography (petroleum ether/ethyl acetate = 50/1 to 30/1) to obtain Compound 8-2. LCMS (ESI) m/z: 271.2 (M+1). ¹H NMR (400 MHz, CDCl₃) δ = 8.15 (d, *J* = 5.0 Hz, 1H), 7.32 - 7.23 (m, 4H). 7.21 - 7.12 (m, 2H), 4.27 (s, 2H), 3.84 (s, 3H), 2.67 (s, 3H), 2.42 (s, 3H).

### Step 2: Preparation of Compound 8-3

Compound 8-2 (200 mg, 739.85 µmol) was dissolved in a tetrahydrofuran (3 mL) solution, and then lithium aluminum hydride (33.70 mg, 887.82 µmol) was added at 0°C. The mixture was stirred at 0-25°C for 2 hours, water (0.034 mL) and a 15% aqueous sodium hydroxide solution (0.034 mL) were added to the reaction solution, and then the reaction was quenched with water (0.10 mL). The reaction solution was added with anhydrous magnesium sulfate, filtered and concentrated to obtain a crude product, and the crude product was purified through silica gel column chromatography (petroleum ether/ethyl acetate = 3/1) to obtain Compound 8-3. LCMS (ESI) m/z:243.1 (M+1). ¹H NMR (400 MHz, DMSO-*d*₆) δ = 8.09 (d, *J* = 5.0 Hz, 1H), 7.33 (d, *J=* 4.6 Hz, 4H), 7.28 - 720 (m, 1H), 7.08 (d, *J* = 5.0 Hz, 1H), 5.29 (t, *J* = 5.4 Hz, 1H), 4.50 (d, *J* = 5.4 Hz, 2H), 4.22 (s, 2H), 2.63 (s, 3H), 2.19 (s, 3H).

### Step 3: Preparation of Compound 8-4

Compound 8-3 (800 mg, 3.30 mmol) was dissolved in a mixed solvent of ethanol (3 mL) and acetic acid (3 mL), then 80 mg of Pd/C(purity: 10%) was added, a reaction flask was covered with a hydrogen balloon and replaced for three times so that the reaction system was filled with hydrogen, the materials were reacted at 15-25°C for 3 hours while stirring. The reaction solution was filtered and concentrated, water (50 mL) was added, and the reaction solution was extracted with ethyl acetate (25 mL×2). The obtained was washed with water (15 mL × 2), then dried over anhydrous sodium sulfate and filtered, and the filtrate was concentrated under reduced pressure to obtain crude Compound 8-4. LCMS (ESI) m/z: 153.1 (M+1)

### Step 4: Preparation of Compound 8-5

Compound 8-4 (156 mg, 1.03 mmol) was dissolved in DMF (1 mL), NaH (82.00 mg, 2.05 mmol, purity:60%) was added at 0-5°C, and the mixture was stirred for 0.5 hours, then Compound 4-7(413.99 mg, 1.08 mmol) was added, the materials were reacted at 0-5°C for 3 hours while stirring. The reaction was quenched with an aqueous saturated ammonium chloride solution (1 mL), then water (50 mL) was added, and the reaction solution was extracted with ethyl acetate (25 mL × 2. The obtained organic phase was washed with a saturated brine solution (25 mL × 2), filtered, dried over anhydrous sodium sulfate, and the filtrate was concentrated under reduced pressure to obtain Compound 8-5. LCMS (ESI) m/z: 456.1 (M+1).

### Step 6: Preparation of trifluoroacetate of Compound 8-6

Compound 8-5 (330 mg, 723.79 µmol) was dissolved in DCM (3 mL), N-bromosuccinimide (128.82 mg, 723.79 µmol) was added at 0°C, and the mixture was stirred at 0-5°C for 1 hour. The mixture was concentrated to obtain a crude product. The crude product was purified through preparative HPLC (chromatographic column: Phenomenex Synergi Polar-RP 100*25 mm*4 µm; mobile phase: 0.05% aqueous trifluoroacetic acid solution- acetonitrile; gradient: acetonitrile: 32%-52%) to obtain trifluoroacetate of Compound 8-6. LCMS (ESI) m/z: 536.0 (M+1).

### Step 8: Preparation of Compound 8-7

The trifluoroacetate (90 mg, 168.28 µmol) of Compound 8-6 and bis(pinacolato) diboron (85.46 mg, 336.56 µmol) were dissolved in ethylene glycol dimethyl ether (5 mL), then potassium pivalate (47.19 mg, 336.56 µmol) and tetrakis(triphenylphosphine) palladium (19.45 mg, 16.83 µmol) were added, the reaction was replaced with nitrogen for three times, and the mixture was stirred at 90°C for 12 hours to obtain a reaction solution containing crude Compound 8-7, and the reaction solution was directly used for the next step. LCMS (ESI) m/z: 582.5 (M+1).

### Step 7: Preparation of Compound 8-8

The reaction solution containing crude Compound 8-7 (97.9 mg, 168.24 µmol) was added to a solution of potassium carbonate (46.51 mg, 336.49 µmol), tetrakis(triphenylphosphine) palladium (19.44 mg, 16.82 µmol) and 2-dicyclohexylphosphino-2,4,6-triisopropylbiphenyl (16.04 mg, 33.65 µmol) in ethylene glycol dimethyl ether (2 mL) and water (0.2 mL) , and then the materials were reacted at 90°C for 2 hours. The mixture was concentrated, diluted with a saturated brine solution (30 mL) and extracted with ethyl acetate (30 mL × 2). The obtained organic phase was washed with a saturated brine solution (10 mL × 2), dried over anhydrous sodium sulfate, filtered and concentrated under reduced pressure to obtain crude Compound 8-8. LCMS (ESI) m/z: 420.2 (M+1).

### Step 8: Preparation of trifluoroacetate of Compound 8

Compound 8-8 (95 mg, 226.48 µmol) was dissolved in methanol (3 mL), then water (1 mL) and NaOH (54.35 mg, 1.36 mmol) were added, and the materials were reacted at 15-20°C for 12 hours while stirring. The reaction solution was filtered, concentrated to obtain a crude product. The crude product was purified through preparative HPLC (chromatographic column: 3_Phenomenex Luna C18 75*30mm*3µm; mobile phase: 0.05% aqueous trifluoroacetic acid solution- acetonitrile; gradient: acetonitrile: 13%-33%) to obtain trifluoroacetate of Compound 8. LCMS (ESI) m/z: 392.1 (M+1). ¹H NMR (400 MHz, CD₃OD) δ = 9.09 (s, 1H), 8.64 (d, *J* = 8.3 Hz, 1H), 8.15 (s, 1H), 7.85 (d, *J* = 8.3 Hz, 1H), 4.99 - 4.95 (s, 2H), 4.80 - 4.75 (s, 2H), 4.45 (tt, *J* = 3.5, 7.0 Hz, 1H), 3.21 (s, 3H), 2.42 (s, 3H), 1.52 - 1.45 (m, 2H), 1.12 - 1.03 (m, 2H).

### Example 9

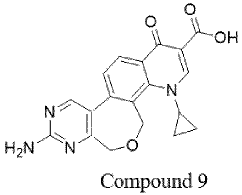

### Synthetic route:

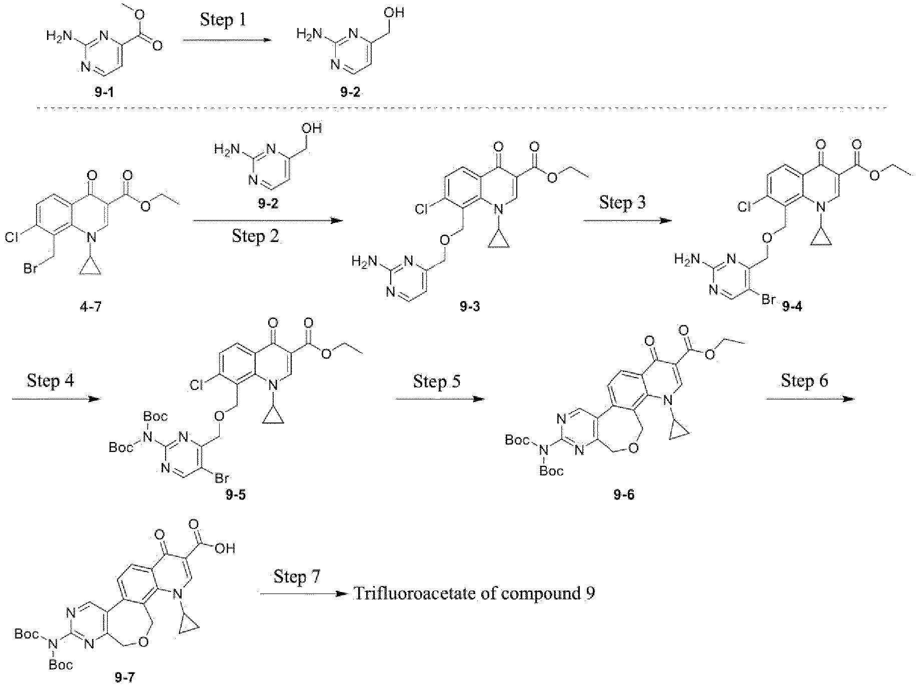

### Step 1: Preparation of Compound 9-2

Compound 9-1 (2 g, 13.06 mmol) was added to a mixed solvent of ethanol (125 mL) and dichloromethane (20 mL), then NaBH₄ (1.51 g, 39.97 mmol) was added and the mixture was stirred at 15-20°C for 12 hours. The reaction solution was filtered, the filtrate was concentrated, acetone (20 mL) was added under an ice bath, then 2M of dilute hydrochloric acid was added until no bubbles were generated, a saturated sodium bicarbonate solution was added until pH = 8, the reaction solution was filtered, the filtrate was concentrated, ethanol (50 mL) was added, and the mixture was stirred at 15-20°C for 0.5 hours, the reaction solution was filtered, and the filtrate was concentrated to obtain Compound 9-2. ¹H NMR (400 MHz, DMSO-d₆) δ = 8.20 (d, *J* = 4.9 Hz, 1H), 6.66 (d, *J=* 5.0 Hz, 1H), 6.50 (br s, 2H), 5.75 - 5.00 (br s, 1H), 4.30(s, 2H).

### Step 2: Preparation of Compound 9-3

Compound 9-2 (0.87 g, 4.17 mmol) was dissolved in DMF (10 mL), NaH (667.47 mg, 16.69 mmol) was added at 0°C, the mixture was stirred for 0.5 hours, then Compound 4-7 (1.60 g, 4.17 mmol) was added, and the resulting mixture was stirred at 0-5°C for 3 hours. The reaction was quenched with a saturated brine solution (100 mL), and the obtained reaction solution was dried over sodium sulfate and filtered, and the filtrate was concentrated to obtain Compound 9-3. LCMS (ESI) m/z: 429.1 (M+1).

### Step 3: Preparation of Compound 9-4

Compound 9-3 (0.55 g, 1.28 mmol) was dissolved in DCM (5 mL), NBS (228.25 mg, 1.28 mmol) was added at 0°C, and the mixture was reacted for 1 hour while stirring. The crude product was purified through silica gel flash column chromatography (dichloromethane/methanol = 50/1) to obtain Compound 9-4. LCMS (ESI) m/z: 508.9 (M+1).

### Step 4: Preparation of Compound 9-5

Compound 9-4 (0.8 g, 1.58 mmol), Boc₂O (1.20 g, 5.51 mmol, 1.27 mL), and DMAP (19.25 mg, 157.55 µmol) were dissolved in DCM (3 mL), and reacted at 10-25°C for 12 hours while stirring. The reaction solution was concentrated, and purified through silica gel flash column chromatography (dichloromethane/methanol = 50/1) to obtain Compound 9-5. LCMS (ESI) m/z: 709.2 (M+1).

### Step 5: Preparation of Compound 9-6

Compound 9-5 (100 mg, 141.24 µmol), bis(pinacolato) diboron (71.73 mg, 282.49 µmol), tetrakis(triphenylphosphine)palladium (16.32 mg, 14.12 µmol), and potassium pivalate (39.61 mg, 282.49 µmol) were added into DME (2 mL), the mixture was stirred at 90°C for 12 hours and cooled to room temperature, then potassium carbonate (39.02 mg, 282.35 µmol), tetrakis(triphenylphosphine) palladium (16.32 mg, 14.12 µmol), 2-dicyclohexylphosphino-2,4,6-triisopropylbiphenyl (13.46 mg, 28.24 µmol), and H₂O (0.2 mL) were added, and the materials were reacted at 90°C for 2 hours. The reaction solution was concentrated to obtain Compound 9-6. LCMS (ESI) m/z: 593.3 (M+1).

### Step 6: Preparation of Compound 9-7

Compound 9-6 (83 mg, 140.05 µmol) was added to a mixed solvent of MeOH (1 mL) and H₂O (3 mL), sodium hydroxide (33.61 mg, 840.31 µmol) was added, and the materials were reacted at 15-20°C for 12 hours while stirring. The reaction solution was concentrated to obtain Compound 9-7. LCMS (ESI) m/z: 565.4 (M+1).

### Step 7: Preparation of trifluoroacetate of Compound 9

Compound 9-7 (79 mg, 139.93 µmol) was added to a mixed solvent of DCM (1 mL) and TFA (770.00 mg, 6.75 mmol) to react at 15-20°C for 6 hours while stirring. The reaction solution was concentrated, the crude product was dissolved in a mixed solvent of DMF (5 mL) and TFA (200 µL), and separated and purified through preparative HPLC (chromatographic column: Phenomenex Synergi Polar-RP 100*25mm*4µm; mobile phase: [0.05% aqueous trifluoroacetic acid solution- acetonitrile ; gradient: acetonitrile : 30%-50%), MeOH (1 mL) was added, the reaction solution was stirred at 15-20°C for 1 hour and filtered, and the filter cake was washed with MeOH (0.5 mL) and dried under reduced pressure to obtain trifluoroacetate of Compound 9. LCMS (ESI) m/z: 365.1 (M+1).

### Example 10

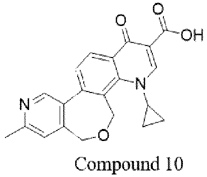

### Synthetic route:

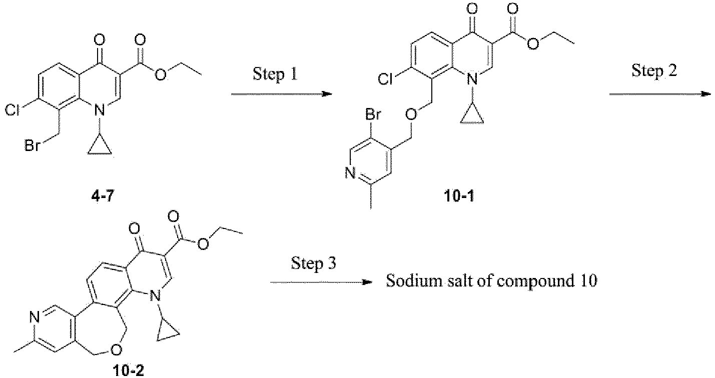

### Step 1: Preparation of trifluoroacetate of Compound 10-1

At 0°C, 5-bromo-2-methylpyridine-4-methanol (525.28 mg, 2.60 mmol) was dissolved in DMF (10 mL), NaH (415.96 mg, 10.40 mmol, purity:60%) was slowly added, and the materials were reacted for 0.5 hour; then, Compound 4-7 (1 g, 2.60 mmol) was added at 0°C, and the mixture was stirred at 0°C for 1 hour. At 0°C, the reaction solution was quenched with an aqueous saturated ammonium chloride solution (20 mL), then diluted with water (20 mL) and extracted with ethyl acetate (90 mL). The obtained organic layer was washed with a saturated brine solution (60 mL), dried over anhydrous sodium sulfate, filtered and concentrated under reduced pressure to obtain a crude product. The crude product was purified through preparative HPLC (chromatographic column: Phenomenex Luna 150× 40 mm × 15 µm; mobile phase: 0.05% aqueous trifluoroacetic acid solution and acetonitrile; the proportion of acetonitrile in the mobile phase ranged from 21% to 51%) to obtain trifluoroacetate of Compound 10-1. LCMS (ESI) m/z: 507.1 (M+1). 1H NMR (400 MHz, CD₃OD) δ ppm 0.81 - 0.92 (m, 2 H) 1.17 (br d, J=6.24 Hz, 3 H) 1.26 (t, J=7.09 Hz, 3 H) 2.42 (s, 3 H) 4.22 (d, J=7.09 Hz, 3 H) 4.54 (s, 2 H) 5.49 (s, 2 H) 7.35 (s, 1 H) 7.42 (d, J=8.68 Hz, 1 H) 8.13 (d, J=8.68 Hz, 1 H) 8.47 (s, 1 H) 8.71 (s, 1 H).

### Step 2: Preparation of trifluoroacetate of Compound 10-2

Trifluoroacetate (252 mg, 498.23 µmol) of Compound 10-1, bis(pinacolato) diboron (253.04 mg, 996.47 µmol), potassium pivalate (139.72 mg, 996.47 µmol) and triphenylphosphine palladium (36.74 mg, 99.65 µmol) were mixed in DMF (3 mL), the reaction was degassed and replaced with N₂ three times, then the mixture was stirred in a nitrogen atmosphere at 120°C for 16 hours. The reaction solution was filtered, and the filtrate was purified through preparative HPLC (chromatographic column: 3_Phenomenex Luna 75 × 30 mm × 3 µm; mobile phase: 0.05% aqueous trifluoroacetic acid solution- acetonitrile; gradient: acetonitrile: 15%-35%) to obtain trifluoroacetate of Compound 10-2. LCMS (ESI) m/z:391.2 (M+1).¹H NMR (400 MHz, DMSO-*d*₆) δ ppm 0.94 (br s, 2 H) 1.25 - 1.33 (m, 5 H) 2.65 (s, 3 H) 4.20 - 4.39 (m, 3 H) 4.57 (s, 2 H) 4.66 (s, 2 H) 7.65 (s, 1 H) 7.83 (d, J=8.31 Hz, 1 H) 8.38 (d, J=8.19 Hz, 1 H) 8.67 (s, 1 H) 8.94 (s, 1 H).

### Step 3: Preparation of sodium salt of Compound 10

At room temperature, a solution of sodium hydroxide (51.22 mg, 1.28 mmol) in water (0.3 mL) was added to a solution of trifluoroacetate of Compound10-2 (50 mg, 128.06 µmol) in anhydrous methanol (0.6 mL). The mixture was stirred at 20°C for 3 hours. The mixture was concentrated under reduced pressure to obtain a crude product. DMF (1 mL) was added to the crude product, and the solution was stirred for 1 hour and filtered, and the filter cake was collected to obtain a sodium salt of Compound 10. LCMS (ESI) m/z: 363.1 (M+1). ¹H NMR (400 MHz, DMSO-*d*₆) δ ppm 1.04 (br s, 2 H) 1.34 (br d, J=6.85 Hz, 3 H) 2.62 (s, 3 H) 4.48 - 4.59 (m, 3 H) 4.69 (br s, 2 H) 7.54 (s, 1 H) 8.01 (d, J=8.31 Hz, 1 H) 8.52 (d, J=8.31 Hz, 1 H) 8.91 (d, J=10.03 Hz, 2 H) 14.84 (s, 1 H).

### Example 11

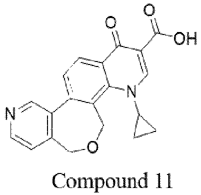

### Synthetic route:

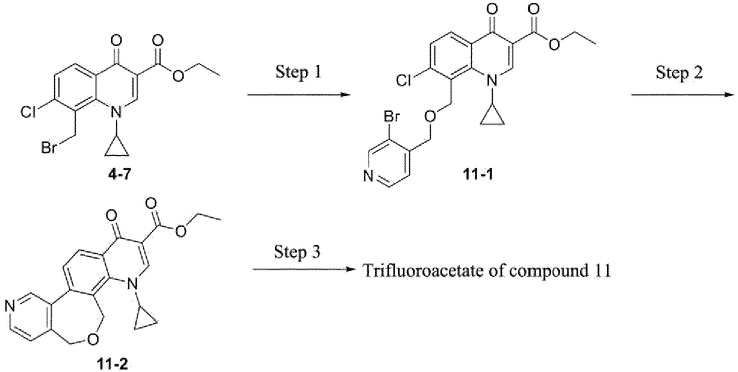

### Step 1: Preparation of trifluoroacetate of Compound 11-1

At 0°C, 3-bromopyridine-4-methanol (2.44 g, 12.98 mmol) was dissolved in DMF (30 mL), NaH (2.08 g, 51.91 mmol, purity:60%) was slowly added, and the materials were reacted for 0.5 hours; then, Compound 4-7 (4.99 g, 12.98 mmol) was added at 0°C, and the mixture was stirred at 0°C for 1 hour. At 0°C, the reaction solution was quenched with an aqueous saturated ammonium chloride solution (100 mL), then diluted with water (50 mL) and extracted with dichloromethane (150 mL). The obtained organic layer was washed with a saturated brine solution (200 mL), dried over anhydrous sodium sulfate, filtered and concentrated under reduced pressure to obtain a crude product. The crude product was purified through preparative HPLC (chromatographic column: Waters Xbridge 250 × 80 mm × 15 µm; mobile phase: 10 mmol/L aqueous trifluoroacetic acid solution and acetonitrile; gradient: acetonitrile 40%-70%) to obtain trifluoroacetate of Compound 11-1. ¹H NMR (400 MHz, DMSO-*d*₆) δ ppm 1.13 - 1.25 (m, 7 H) 4.39 (s, 2 H) 4.46 (br s, 1 H) 4.54 (s, 2 H) 5.46 (s, 2 H) 7.55 (d, J=8.63 Hz, 1 H) 7.62 (d, J=8.63 Hz, 1 H) 8.11 (dd, J=8.57, 5.19 Hz, 2 H) 8.60 (s, 1 H) 8.77 (s, 1 H).

### Step 2: Preparation of trifluoroacetate of Compound 11-2

The trifluoroacetate of Compound 11-1 (100 mg, 203.35 µmol), bis(pinacolato) diboron (103.28 mg, 406.70 µmol), potassium pivalate (57.03 mg, 406.70 µmol), and triphenylphosphine palladium (15.00 mg, 40.67 µmol) were mixed in DMF (1 mL), the reaction was degassed and replaced with N₂ three times, and then the mixture was stirred in a nitrogen atmosphere at 120°C for 16 hours. The reaction solution was filtered, and the filtrate was purified through preparative HPLC (chromatographic column: 3_Phenomenex Luna 75 × 30 mm × 3 µm; mobile phase: 0.05% aqueous trifluoroacetic acid solution-acetonitrile; gradient: acetonitrile: 16%-36%) to obtain trifluoroacetate of Compound 11-2. LCMS (ESI) m/z: 377.1 (M+1).

### Step 3: Preparation of Compound 11

At room temperature, a solution of sodium hydroxide (10.63 mg, 265.67 µmol) in water (0.3 mL) was added to a solution of trifluoroacetate of Compound 11-2 (10 mg, 26.57 µmol) in anhydrous methanol (0.6 mL). The mixture was stirred at 20°C for 3 hours. The reaction solution was filtered, the filtrate was purified through preparative HPLC (chromatographic column: 3_Phenomenex Luna 75 × 30 mm × 3 µm; mobile phase: 0.05% aqueous trifluoroacetic acid solution-acetonitrile; gradient: acetonitrile: 15%-35%) to obtain trifluoroacetate of Compound 11. LCMS (ESI) m/z: 349.0 (M+1). ¹H NMR (400 MHz, CDCl₃) δ ppm 0.98 - 1.11 (m, 2 H) 1.22 - 1.27 (m, 2 H) 4.22 (dt, J=7.21, 3.48 Hz, 1 H) 4.59 (s, 2 H) 4.75 (br s, 2 H) 7.48 (d, *J=*5.14 Hz, 1 H) 7.83 (d, J=8.31 Hz, 1 H) 8.70 (d, J=8.19 Hz, 1 H) 8.83 (d, J=4.89 Hz, 1 H) 9.00 (d, J=5.87 Hz, 2 H) 14.54 (br d. *J*=3.55 Hz, 1 H).

### Example 12

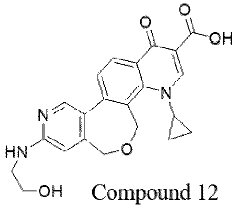

### Synthetic route:

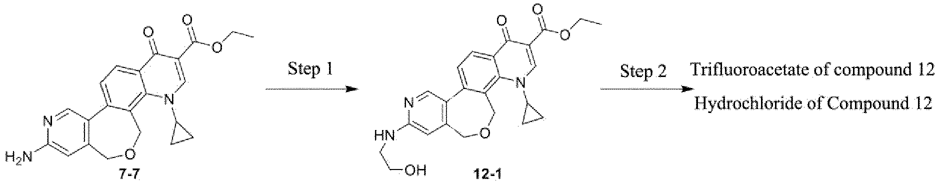

### Step 1: Preparation of trifluoroacetate of Compound 12-1 and Compound 12-1

At room temperature, acetic acid (27.61 mg, 459.87 µmol) was added to a solution of trifluoroacetate of Compound 7-7 (0.12 g, 306.58 µmol), (tert-butyldimethylsilyloxy) acetaldehyde (106.88 mg, 613.15 µmol) in dichloromethane (3 mL) and ethanol (1.5 mL), the mixture was warmed to 40°C and stirred for 1 hour and then cooled to room temperature, sodium cyanoborohydride (57.80 mg, 919.73 µmol) was added, the materials continued to react at 40°C for 1 hour. Multi-batch post-processing, Method 1: the reaction solution was concentrated under reduced pressure to obtain a crude product, and the crude product was purified through preparative HPLC (chromatographic column: 3_Phenomenex Luna C18 70*30mm*3µm; mobile phase: 0.05% aqueous trifluoroacetic acid solution and acetonitrile; gradient: acetonitrile 37%-57%) to obtain trifluoroacetate of Compound 12-1; Method 2: the reaction solution was concentrated under reduced pressure to obtain a crude product, the obtained crude product was adjusted to pH=8 with a saturated sodium carbonate solution, and extracted with dichloromethane, and organic phases were combined, and concentrated under reduced pressure to obtain Compound 12-1. LCMS (ESI) m/z: 436.2 (M+1).

### Step 2: Preparation of trifluoroacetate of Compound 12 and hydrochloride of Compound 12

Method 1: at room temperature, a solution of sodium hydroxide (55.11 mg, 1.38 mmol) in water (0.3 mL) was added to Compound 12-1 (0.06 g, 137.78 µmol) in methanol (0.6 mL), and then the mixture was stirred at room temperature for 3 hours. The reaction solution was filtered, and the filtrate was purified through preparative HPLC (chromatographic column: Phenomenex Synergi Polar-RP 100*25mm*4µm; mobile phase: 0.05% aqueous trifluoroacetic acid solution-acetonitrile; gradient: acetonitrile: 20%-40%) to obtain trifluoroacetate of Compound 12. LCMS (ESI) m/z: 408.2 (M+1).

Method 2: at room temperature, a solution of sodium hydroxide (918.54 mg, 22.96 mmol) in water (10 mL) was added to a solution of Compound 12-1 (1 g, 2.30 mmol) in methanol (20 mL), and then the mixture was stirred at 20-50°C for 36 hours and filtered, the filter cake was collected to obtain a crude product. Hydrochloric acid (12 M, 2 mL) was added to a solution of the crude product (100 mg, 245.45 µmol) in water (4 mL), and then the mixture was stirred for 16 hours. The reaction solution was filtered, and then the filter cake was collected and dried to obtain hydrochloride of Compound 12. LCMS (ESI) m/z: 408.1 (M+1). ¹H NMR (400 MHz, CD₃OD) δ ppm 9.08 (s, 1 H), 8.63 (d, J=8.44 Hz, 1 H), 8.31 (s, 1 H), 7.85 (d, J=8.31 Hz, 1 H), 7.18 (s, 1 H), 4.96 (br s, 2 H), 4.64 (s, 2 H), 4.41 - 4.48 (m, 1 H), 3.87 (t, J=5.20 Hz, 2 H), 3.63 (t, J=5.32 Hz, 2 H), 1.44 - 1.51 (m, 2 H), 1.05 - 1.11 (m, 2 H).

### Example 13

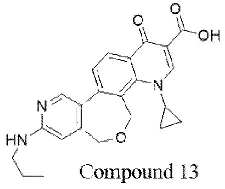

### Synthetic route:

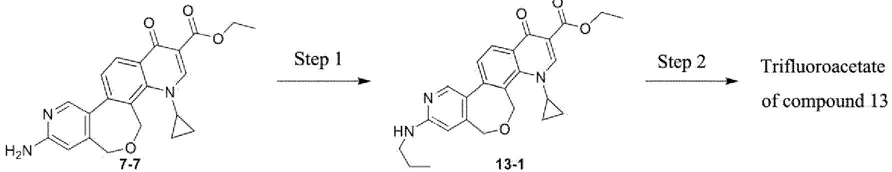

### Step 1: Preparation of trifluoroacetate of Compound 13-1

At room temperature, acetic acid (4.60 mg, 76.64 µmol) was added to a solution of compound 7-7 (0.02 g, 51.10 µmol) and propionaldehyde (4.45 mg, 76.64 µmol) in dichloromethane (1 mL) and ethanol (0.5 mL), the mixture was warmed to 40°C and stirred for 1 hour and then cooled to room temperature, sodium cyanoborohydride (9.63 mg, 153.29 µmol) was added, and the materials continued to react at 40°C for 1 hour. The reaction solution was concentrated under reduced pressure, then purified through preparative HPLC (chromatographic column: Phenomenex Synergi Polar-RP 100*25mm*4µm; mobile phase: 0.05% aqueous trifluoroacetic acid solution-acetonitrile; gradient: acetonitrile: 28%-48%) to obtain trifluoroacetate of Compound 13-1. LCMS (ESI) m/z: 434.2 (M+1).

### Step 2: Preparation of trifluoroacetate of Compound 13

At room temperature, a solution of sodium hydroxide (5.07 mg, 126.87 µmol) in water (0.25 mL) was added to a solution of trifluoroacetate of Compound 13-1 (11 mg, 25.37 µmol) in methanol (0.5 mL), and then the mixture was stirred at room temperature for 16 hours. The reaction solution was filtered, and the filtrate was purified through preparative HPLC (chromatographic column: Phenomenex Synergi Polar-RP 100*25mm*4µm; mobile phase: 0.05% aqueous trifluoroacetic acid solution-acetonitrile; gradient: acetonitrile: 20%-40%) to obtain trifluoroacetate of Compound 13. LCMS (ESI) m/z: 406.2 (M+1).

### Example 14

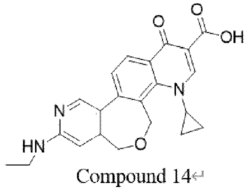

### Synthetic route:

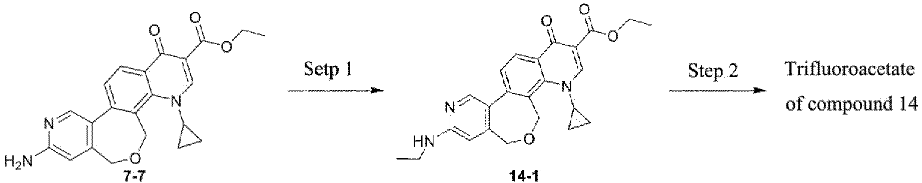

### Step 1: Preparation of Compound 14-1

At room temperature, acetic acid (3.38 mg, 76.65 µmol) was added to a solution of trifluoroacetate of Compound 7-7 (0.02 g, 51.10 µmol) and acetaldehyde (3.38 mg, 76.65 µmol) in dichloromethane (1 mL) and ethanol (0.5 mL), the mixture was warmed to 30°C and stirred for 1 hour and then cooled to room temperature, sodium cyanoborohydride (9.63 mg, 153.29 µmol) was added, and the materials continued to react at 30°C for 1 hour. The reaction solution was concentrated under reduced pressure to obtain Compound 3-9. LCMS (ESI) m/z: 420.1 (M+1).

### Step 2: Preparation of trifluoroacetate of Compound 14

At room temperature, a solution of sodium hydroxide (10.22 mg, 255.56 µmol) in water (0.5 mL) was added to a solution of Compound 14-1 (21.44 mg, 51.11 µmol) in methanol (1 mL), and then the mixture was stirred at 30°C for 16 hours. The reaction solution was filtered, and the filtrate was purified through preparative HPLC (chromatographic column: Phenomenex Synergi Polar-RP 100*25mm*4µm; mobile phase: 0.05% aqueous trifluoroacetic acid solution-acetonitrile; gradient: acetonitrile: 23%-43%) to obtain trifluoroacetate of Compound 14. LCMS (ESI) m/z: 392.1 (M+1).

### Example 15

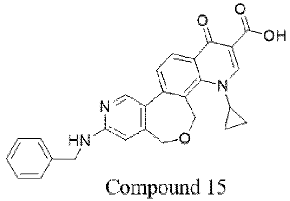

### Synthetic route:

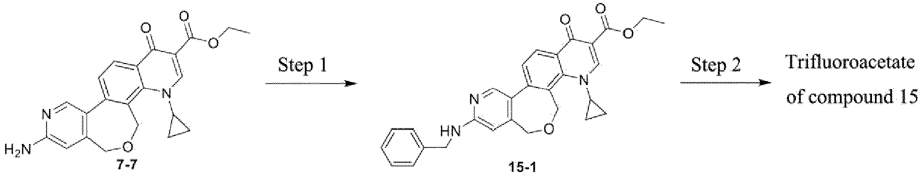

### Step 1: Preparation of Compound 15-1

At room temperature, acetic acid (6.90 mg, 114.97 µmol) was added to a solution of trifluoroacetate of Compound 7-7 (0.03 g, 76.64 µmol) and benzaldehyde (16.27 mg, 153.29 µmol) in dichloromethane (1 mL) and ethanol (0.5 mL), the mixture was warmed to 30°C and stirred for 1 hour and then cooled to room temperature, sodium cyanoborohydride (9.63 mg, 153.29 µmol) was added, and the materials continued to react at 50°C for 1 hour. The reaction solution was concentrated under reduced pressure to obtain crude Compound 15-1. LCMS (ESI) m/z: 482.3 (M+1).

### Step 2: Preparation of trifluoroacetate of Compound 15

At room temperature, a solution of sodium hydroxide (24.92 mg, 623.00 µmol) in water (1 mL) was added to a solution of Compound 15-1 (60 mg, 124.60 µmol) in methanol (2 mL), and then the mixture was stirred at 30°C for 15 hours. The reaction solution was filtered, and the filtrate was purified through preparative HPLC (chromatographic column: Phenomenex Synergi Polar-RP 100*25mm*4µm; mobile phase: 0.05% aqueous trifluoroacetic acid solution-acetonitrile; gradient: acetonitrile: 32%-52%) to obtain trifluoroacetate of Compound 15. LCMS (ESI) m/z: 454.2(M+1). 1H NMR (400 MHz, CD₃OD) δ = 9.08 (s, 1H), 8.64 (d, J = 8.3 Hz, 1H), 8.31 (s, 1H), 7.85 (d, J = 8.3 Hz, 1H), 7.54 - 7.34 (m, 6H), 7.26 (s, 1H), 4.98 (s, 2H), 4.72 (s, 2H), 4.65 (s, 2H), 4.44 (td, J = 3.5, 7.2 Hz, 1H), 1.47 (br d, J = 6.5 Hz, 2H), 1.08 (br d, J = 2.6 Hz, 2H).

### Example 16

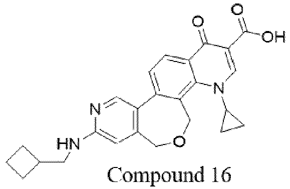

### Synthetic route:

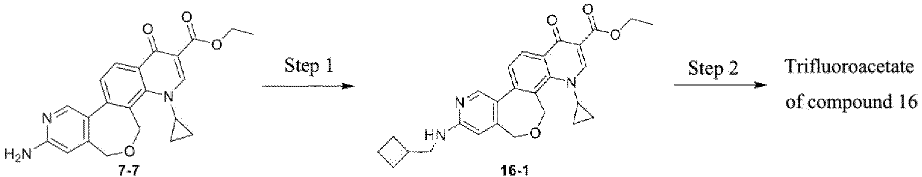

### Step 1: Preparation of Compound 16-1

At room temperature, acetic acid (23.01 mg, 383.22 µmol) was added to a solution of trifluoroacetate of Compound 7-7 (0.1 g, 255.48 µmol) and cyclobutane carboxaldehyde (42.98 mg, 510.96 µmol) in dichloromethane (10 mL) and methanol (5 mL), the mixture was warmed to 40 °C and stirred for 1 hour and then cooled to room temperature, pyridine-borane (71.23 mg, 766.44 µmol) was added, and the materials continued to react at 40°C for 40 hours. The reaction solution was quenched with a saturated ammonium chloride solution (10 mL) and then extracted with dichloromethane (10 mL). The obtained organic layer was washed with a saturated brine solution (10 mL), dried over anhydrous sodium sulfate, filtered and concentrated under reduced pressure to obtain crude compound 16-1. LCMS (ESI) m/z: 460.2 (M+1).

### Step 2: Preparation of trifluoroacetate of Compound 16

At room temperature, a solution of sodium hydroxide (24.92 mg, 623.00 µmol) in water (0.6 mL) was added to a solution of Compound 16-1 (120 mg, 250.69 µmol) in methanol (1.2 mL), and then the mixture was stirred at 40°C for 16 hours. The reaction solution was filtered, and the filtrate was purified through preparative HPLC (chromatographic column: 3_Phenomenex Luna C18 75*30mm*3µm; mobile phase: 0.05% aqueous trifluoroacetic acid solution-acetonitrile; gradient: acetonitrile: 29%-49%) to obtain trifluoroacetate of Compound 16. LCMS (ESI) m/z: 432.2(M+1). 1H NMR (400 MHz, DMSO-*d*₆) δ = 8.89 (s, 1H), 8.45 (d, J = 8.3 Hz, 1H), 8.37 (s, 1H), 7.88 (d, J = 8.5 Hz, 1H), 6.97 (br s, 1H), 4.77 (br s, 2H), 4.51 (s, 2H), 4.48 (br d, J = 3.6 Hz, 1H), 3.43 (br d, J = 7.1 Hz, 2H), 2.68 - 2.59 (m, 1H), 2.18 - 2.00 (m, 2H), 1.95 - 1.84 (m, 2H), 1.83 - 1.68 (m, 2H), 1.32 (br d, J = 6.6 Hz, 2H), 1.02 (br s, 2H).

### Example 17

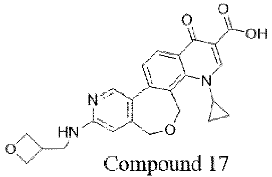

### Synthetic route:

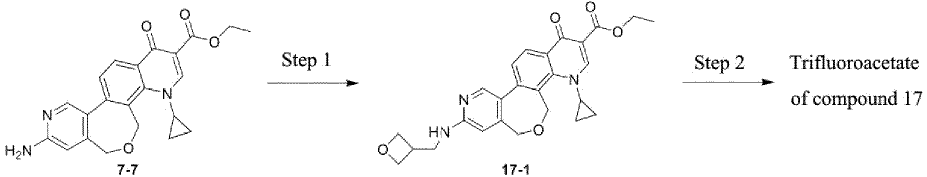

### Step 1: Preparation of compound 17-1

At room temperature, acetic acid (23.01 mg, 383.22 µmol) was added to a solution of trifluoroacetate of Compound 7-7 (0.1 g, 255.48 µmol) and oxa-3-cyclobutanecarboxaldehyde (43.99 mg, 510.96 µmol) in dichloromethane (10 mL) and methanol (5 mL), the mixture was warmed to 40°C and stirred for 1 hour and then cooled to room temperature, pyridine-borane (71.23 mg, 766.44 µmol) was added, and the materials continued to react at 40°C for 40 hours. The reaction solution was quenched with a saturated ammonium chloride solution (10 mL) and then extracted with dichloromethane (10 mL). The obtained organic layer was washed with a saturated brine solution (10 mL), dried over anhydrous sodium sulfate, filtered and concentrated under reduced pressure to obtain Compound 17-1. LCMS (ESI) m/z: 462.2 (M+1).

### Step 2: Preparation of trifluoroacetate of Compound 17

At room temperature, a solution of sodium hydroxide (8.67 mg, 216.68 µmol) in water (0.5 mL) was added to a solution of Compound 17-1 (10 mg, 21.67 µmol) in methanol (1 mL), and then the mixture was stirred at 40°C for 16 hours. The reaction solution was filtered, and the filtrate was purified through preparative HPLC (chromatographic column: 3_Phenomenex Luna C18 75*30mm*3µm; mobile phase: 0.05% aqueous trifluoroacetic acid solution-acetonitrile; gradient: acetonitrile: 11%-31%) to obtain trifluoroacetate of Compound 17. LCMS (ESI) m/z: 434.1(M+1). 1H NMR (400 MHz, DMSO-*d*₆) δ = 9.68 (br d, J = 1.1 Hz, 1H), 8.93 (s, 1H), 8.54 (d, J = 2.2 Hz, 1H), 7.92 - 7.83 (m, 1H), 7.16 (s, 1H), 4.91 - 4.81 (m, 2H), 4.58 (br d, J = 1.3 Hz, 3H), 4.53 - 4.43 (m, 2H), 4.22 - 4.19 (m, 1H), 3.58 (br dd, J = 3.2, 6.9 Hz, 2H), 3.54 - 3.46 (m, 1H), 1.33 - 1.33 (m, 1H), 1.31 (br d, J = 7.6 Hz, 1H), 1.03 (br s, 2H).

### Example 18

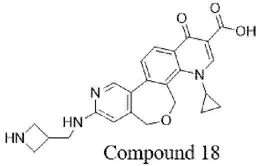

### Synthetic route:

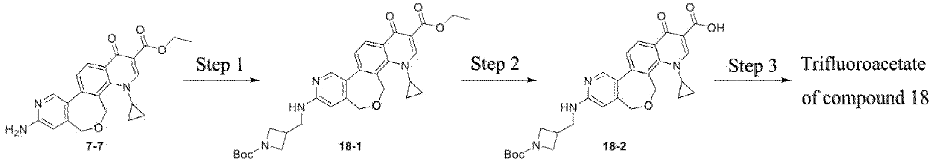

### Step 1: Preparation of Compound 18-1

At 20°C, trifluoroacetate of Compound 7-7 (20 mg, 51.10 µmol) and N-Boc-3-formylazetidine (14.20 mg, 76.64 µmol) were dissolved in dichloromethane (1 mL) and water (0.5 mL), then glacial acetic acid (4.60 mg, 76.64 µmol) was added, and the mixture was stirred at 50°C for 16 hours. At room temperature, sodium cyanoborohydride (9.63 mg, 153.29 µmol) was added, and the mixture was stirred at 20°C for 5 hours, then warmed to 50°C and stirred for 16 hours. The mixture was filtered and concentrated under reduced pressure to obtain Compound 18-1. LCMS (ESI) m/z:561.4 (M+1).

### Step 2: Preparation of trifluoroacetate of Compound 18-2

At 20°C, Compound 18-1 (80 mg, 142.69 µmol) was dissolved into methanol (2 mL), a sodium hydroxide solution (28.54 mg of sodium hydroxide was dissolved in 1 mL of water) was added and the mixture was stirred at 20°C for 16 hours. The mixture was concentrated under reduced pressure to obtain the residue. The residue was purified through preparative HPLC (chromatographic column: Phenomenex Synergi Polar-RP 100*25mm*4µm; mobile phase: 0.05% aqueous trifluoroacetic acid solution-acetonitrile; gradient: acetonitrile: 32%-52%) to obtain trifluoroacetate of Compound 18-2. LCMS (ESI) m/z: 533.3 (M+1).

### Step 3: Preparation of trifluoroacetate of Compound 18

At 20°C, trifluoroacetate of Compound 18-2 (42 mg, 78.86 µmol) was dissolved in dichloromethane (1 mL), trifluoroacetic acid (616.00 mg, 5.40 mmol) was slowly added, and then the mixture was stirred at 20°C for 2 hours. The mixture was concentrated under reduced pressure to obtain the residue. The residue was purified through preparative HPLC (chromatographic column: 3_Phenomenex Luna C18, 70*30mm*3µm; mobile phase: 0.05% aqueous trifluoroacetic acid solution-acetonitrile; gradient: acetonitrile: 5%-25%) to obtain trifluoroacetate of Compound 18. LCMS (ESI) m/z: 433.2 (M+1). ¹H NMR (400 MHz, CD₃OD) δ ppm 1.02 - 1.15 (m, 2 H) 1.23 - 1.40 (m, 2 H) 1.42 - 1.54 (m, 2 H) 3.17 - 3.28 (m, 2 H) 3.51 (br dd, J=13.27, 8.25 Hz, 2 H) 3.70 - 4.07 (m, 2 H) 4.43 (td, J=7.27, 3.91 Hz, 1 H) 4.62 - 4.69 (m, 2 H) 5.01 (br s, 2 H) 7.20 (s, 1 H) 7.84 (br d, J=8.44 Hz, 1 H) 8.33 (br s, 1 H) 8.54 - 8.72 (m, 1 H) 9.07 (br d, J=5.99 Hz, 1 H).

### Example 19

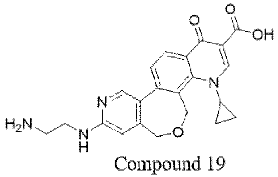

### Synthetic route:

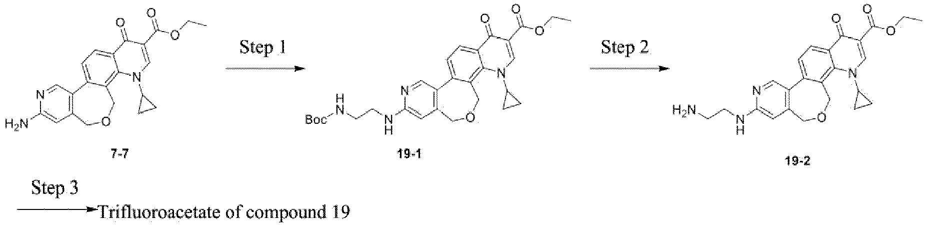

### Step 1: Preparation of trifluoroacetate of Compound 19-1

At 20°C, trifluoroacetate of Compound 7-7 (55 mg, 140.51 µmol) and tert-butyl N-(2- ethoxycarbonyl) carbamate (22.37 mg, 140.51 µmol) were dissolved in dichloromethane (1 mL) and methanol (0.5 mL), then glacial acetic acid (16.88 mg, 281.03 µmol) was added, and the mixture was stirred at 50°C for 3 hours. At 20°C, sodium cyanoborohydride (26.49 mg, 421.54 µmol) was added, and the mixture was stirred at 20°C for 1 hour, then warmed to 50°C and stirred for 16 hours. The mixture was concentrated under reduced pressure to obtain the residue. The residue was purified through preparative HPLC (chromatographic column: 3_Phenomenex Luna C18, 75 × 30 mm × 3 µm; mobile phase: 0.05% aqueous trifluoroacetic acid solution and acetonitrile; the proportion of acetonitrile in the mobile phase ranged from 25% to 45%) to obtain trifluoroacetate of Compound 19-1. LCMS (ESI) m/z: 535.3(M+1).

### Step 2: Preparation of trifluoroacetate of Compound 19-2

At room temperature, trifluoroacetate of Compound 19-1 (22 mg, 41.15 µmol) was dissolved in dichloromethane (1 mL), then trifluoroacetic acid (469.22 mg, 4.12 mmol) was added, and the mixture was stirred at 20°C for 2 hours. The mixture was concentrated under reduced pressure to obtain trifluoroacetate of Compound 19-2. LCMS (ESI) m/z:435.1(M+1).

### Step 3: Preparation of trifluoroacetate of Compound 19

At room temperature, trifluoroacetate of Compound 19-2 (17.8 mg, 40.97 µmol) was dissolved in methanol (1mL), then a sodium hydroxide solution (8.19 mg sodium hydroxide was dissolved in 0.5 mL of water) was added, and the mixture was stirred at 20°C for 16 hours. The mixture was concentrated under reduced pressure to obtain the residue. The residue was purified through preparative HPLC (chromatographic column: 3_Phenomenex Luna C18, 75 × 30 mm × 3 µm; mobile phase: 0.05% aqueous trifluoroacetic acid solution- acetonitrile; gradient: acetonitrile: 10%-30%) to obtain trifluoroacetate of Compound 19. LCMS (ESI) m/z: 407.1 (M+1). ¹H NMR (400 MHz, CD₃OD) δ ppm 0.90 - 1.01 (m, 2 H) 1.36 (br d, J=6.97 Hz, 2 H) 3.13 (t, J=5.69 Hz, 2 H) 3.64 (t, J=5.69 Hz, 2 H) 4.27 - 4.36 (m, 1 H) 4.44 (s, 2 H) 4.72 - 4.75 (m, 2 H) 6.69 (s, 1 H) 7.73 (d, J=8.44 Hz, 1 H) 8.37 (s, 1 H) 8.46 (d, J=8.44 Hz, 1 H) 8.94 (s, 1 H).

### Example 20

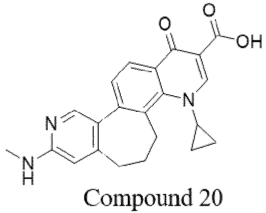

### Synthetic route:

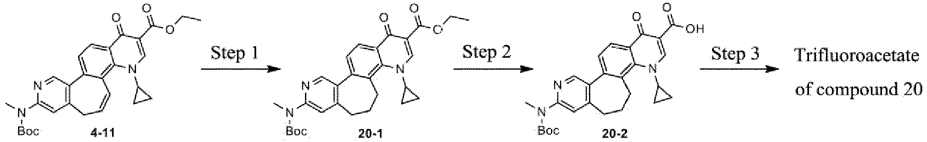

### Step 1: Preparation of Compound 20-1

At room temperature, Compound 4-11 (15 mg, 29.91 µmol) was dissolved in methanol (2 mL), wet Pd/C (10 mg, 50% of water content) was added, the mixture was stirred in hydrogen (15 Psi) atmosphere at 10-15°C for 2 hours, filtered and concentrated under reduced pressure to obtain Compound 20-1. LCMS (ESI) m/z: 504.2 (M+1).

### Step 2: Preparation of hydrochloride of Compound 20-2

At room temperature, Compound 20-1 (16 mg, 28.59 µmol) was dissolved in methanol (1 mL) and water (0.5 mL), then sodium hydroxide (6.86 mg, 171.57 µmol) was added, and the mixture was stirred at 10-15°C for 5 hours. The reaction solution was adjusted to pH=1-2 with 1M of aqueous hydrochloric acid solution, and then concentrated to obtain hydrochloride of Compound 20-2. LCMS (ESI) m/z: 476.1 (M+1).

### Step 3: Preparation of trifluoroacetate of Compound 20

At room temperature, hydrochloride (14 mg, 29.44 µmol) of Compound 20-2 was dissolved in dichloromethane (1 mL), trifluoroacetic acid (0.5 mL) was added and the mixture was stirred at 25-30°C for 1 hour. The reaction solution was concentrated, and the residue was purified through preparative HPLC (chromatographic column: 3_Phenomenex Luna C18 75*30mm*3µm; mobile phase: 0.05% aqueous trifluoroacetic acid solution- acetonitrile; gradient: acetonitrile: 18%-38%) to obtain trifluoroacetate of Compound 20. LCMS (ESI) m/z: 376.0 (M+1). ¹H NMR (400 MHz, CDCl₃) δ = 14.51 (s, 1H), 10.22 (s, 1H), 9.01 (s, 1H), 8.51 (s, 1H), 7.85 (s, 1H), 7.43 (m, 1H), 6.68 (s, 1H), 3.13 (s, 3H), 2.74 (s, 2H), 2.39 (s, 2H), 1.36 - 1.31 (m, 2H), 1.14 (s, 2H).

### Example 21

### Synthetic route:

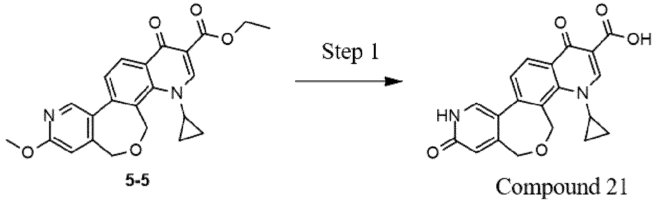

### Step 1: Preparation of Compound 21

Compound 5-5 (105 mg, 258.35 µmol) was added into hydrobromic acid (12 mL, 40% aqueous solution), and the mixture was stirred at 110°C for 8 hours. The reaction solution was concentrated, and the residue was purified through preparative HPLC (chromatographic column: Phenomenex Synergi Polar-RP 100*25mm*4µm; mobile phase: 0.05% aqueous trifluoroacetic acid solution-acetonitrile; gradient: acetonitrile: 29%-49%) to obtain Compound 21. LCMS (ESI) m/z: 365.3 (M+1). ¹H NMR (400 MHz, DMSO-*d*₆) δ = 8.88 (s, 1H), 8.42-8.40 (m, 1H), 7.92 (s, 1H), 7.84-7.82 (m, 1H), 6.60 (s, 1H), 4.84 (s, 1H), 4.44 (s, 1H), 1.32 - 1.30 (m, 2H), 1.00 (s, 2H).

### Example 22

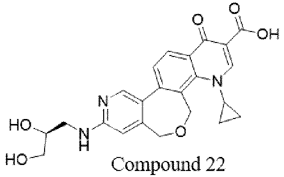

### Synthetic route:

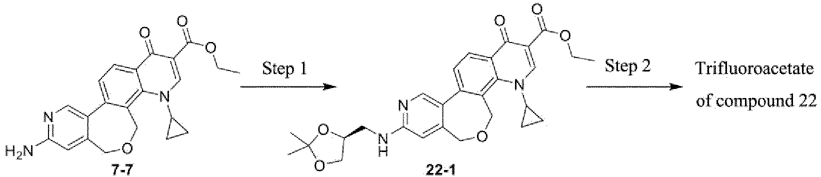

### Step 1: Preparation of Compound 22-1

At room temperature, Compound 7-7 (70.00 mg, 178.84 µmol) and (R)-(+)-2,2-dimethyl-1,3-dioxolane-4-carboxaldehyde (46.55 mg, 357.67 µmol) were dissolved in anhydrous dichloromethane (4 mL) and anhydrous methanol (2 mL), then acetic acid (16.11 mg, 268.25 µmol) was added at 20-30°C, the reaction solution was warmed to 40°C and stirred for1 hour and then cooled to 20-30°C, pyridine-borane (49.86 mg, 536.51 µmol) was added, the reaction solution was warmed to 40°Cand stirred for 16 hours, the reaction was concentrated, 10 mL of dichloromethane was added, the reaction solution was washed with saturated sodium bicarbonate (10 mL*2) and brine (10 mL) successively, dried, and concentrated to obtain Compound 22-1. LCMS (ESI) m/z: 506.1 (M+1).

### Step 2: Preparation of trifluoroacetate of Compound 22

At room temperature, concentrated hydrochloric acid (0.5 mL) was added to a solution of Compound 22-1 (35.00 mg, 69.23 µmol) in ethanol (1.5 mL) and water (1.0 mL), and the reaction solution was stirred at 40°C for 22 hours. The reaction solution was concentrated under reduced pressure to obtain a crude product. The crude product was purified through preparative HPLC (chromatographic column: YMC Triart C18 150*25mm*5µm; mobile phase: 0.05% aqueous trifluoroacetic acid solution and acetonitrile; the proportion of acetonitrile in the mobile phase ranged from 11% to 31%, 10 min) to obtain trifluoroacetate of Compound 22.

### Example 23

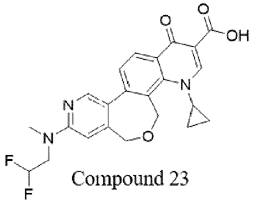

### Synthetic route:

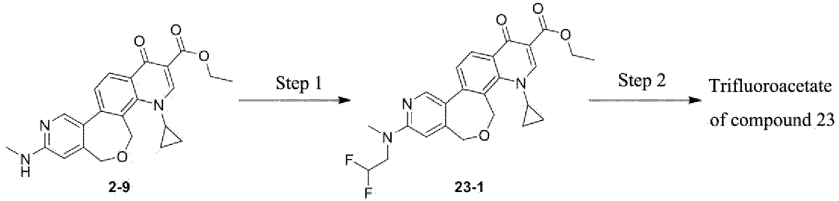

### Step 1: Preparation of Compound 2-9

At room temperature, Compound 2-9 (50 mg, 123.32 µmol) was dissolved in anhydrous N,N-dimethylformamide (3 mL), the reaction solution was cooled to 0°C, and then sodium hydride (5.43 mg, 135.65 µmol) and difluoroethyl trifluoromethanesulfonate (29.04 mg, 135.65 µmol) were added successively under the protection of nitrogen. The reaction solution was stirred at 25°C for 1 hour. Compound 23-1 was obtained without the posttreatment of the reaction solution. LCMS (ESI) m/z: 470.2 (M+1).

### Step 2: Preparation of trifluoroacetate of Compound 23

At room temperature, a solution of sodium hydroxide (4.86 mg, 121.41 µmol) in water (1 mL) was added to a solution of Compound 23-1 (57 mg, 121.41 µmol) in anhydrous N,N-dimethylformamide (3 mL), and the reaction solution was stirred at 25°C for 3 hours. The reaction solution was concentrated under reduced pressure to obtain a crude product. The crude product was purified through preparative HPLC (chromatographic column: YMC Triart C18 150*25mm*5µm; mobile phase: 0.05% aqueous trifluoroacetic acid solution and acetonitrile; the proportion of acetonitrile in the mobile phase ranged from 39% to 59%, 10 min) to obtain trifluoroacetate of Compound 23. LCMS (ESI) m/z: 442.1 (M+1). ¹H NMR (400 MHz, DMSO-*d*₆) δ ppm 14.83 - 15.13 (m, 1 H), 8.85 - 8.94 (m, 1 H), 8.54 - 8.62 (m, 1 H), 8.41 - 8.48 (m, 1 H), 7.88 - 7.99 (m, 1 H). 6.99 - 7.07 (m, 1 H), 6.06 - 6.48 (m, 1 H), 4.65 - 4.74 (m, 2 H), 4.52 - 4.56 (m, 2 H), 4.45 - 4.50 (m, 1 H), 4.05 - 4.18 (m, 2 H), 3.13 - 3.24 (m, 3 H), 1.28 - 1.38 (m, 2 H), 0.96 - 1.07 (m, 2 H).

### Example 24

### Synthetic route:

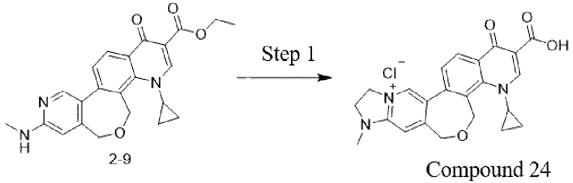

### Step 1: Preparation of Compound 24

At 0°C, sodium hydride (39.46 mg, 986.57 µmol) was added to a solution of Compound 2-9 (0.10 g, 246.64.5µmol) in DMF (4 mL), 10 minutes later, 1-fluoro-2-iodoethane (85.81 mg, 493.28 µmol) was added to the reaction solution and stirred for 1 hour. The reaction solution was quenched with a saturated ammonium chloride solution (10 mL) and then extracted with dichloromethane (10 mL*2). The obtained organic layer was washed with a saturated brine solution (10 mL*2), dried over anhydrous sodium sulfate, filtered and concentrated under reduced pressure to obtain a crude product. The crude product was purified through preparative HPLC (chromatographic column: YMC Triart 30*150mm*7µm; mobile phase: aqueous hydrochloric acid solution and acetonitrile; the proportion of acetonitrile in the mobile phase ranged from 13% to 33%, 10min) to obtain Compound 24. LCMS (ESI) m/z: 404.2(M+1). 1H NMR (400 MHz, DMSO-d₆) δ = 8.93 (s, 1H), 8.83 (s, 1H), 8.54 (d, J = 8.4 Hz, 1H), 7.84 (d, J = 8.4 Hz, 1H), 7.57 (s, 1H), 4.83 (br s, 2H), 4.80 - 4.72 (m, 2H), 4.60 (s, 2H), 4.51 - 4.44 (m, 1H), 4.09 - 4.01 (m, 2H), 3.20 - 3.18 (m, 3H), 1.32 (br d, J = 6.5 Hz, 2H), 1.04 (br s, 2H).

### Example 25

### Synthetic route:

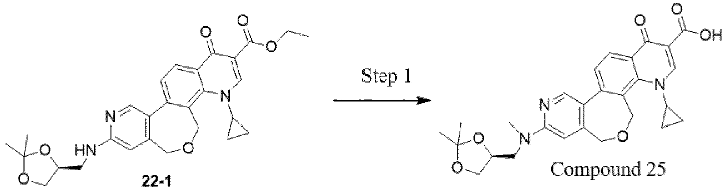

### Step 1: Preparation of Compound 25

At 0°C, sodium hydride (1.58 mg, 39.56 µmol) was added to a solution of Compound 22-1 (0.02 g, 39.56 mmol) in DMF (2 mL), 10 minutes later, the iodomethane (39.56 µmol, 2.46 µL) was added to the reaction solution and stirred for 1 hour. The reaction solution was quenched with a saturated ammonium chloride solution (10 mL) and then extracted with dichloromethane (10 mL*2). The obtained organic layer was washed with a saturated brine solution (5 mL), dried over anhydrous sodium sulfate, filtered and concentrated under reduced pressure to obtain a crude product. The crude product was purified through preparative HPLC (chromatographic column: Waters Xbridge 150*25mm* 5µm; mobile phase: 0.05% aqueous ammonium bicarbonate solution and acetonitrile; the proportion of acetonitrile in the mobile phase ranged from 29% to 59%, 9 min) to obtain Compound 25. LCMS (ESI) m/z: 492.2(M+1). ¹H NMR (400 MHz, DMSO-d₆) δ = 8.88 (s, 1H), 8.54 (s, 1H), 8.44 (d, *J* = 8.8 Hz, 1H), 7.91 (d, *J=* 8.0 Hz, 1H), 6.93 (s, 1H), 4.70 (br d, *J* = 1.5 Hz, 2H), 4.52 (s, 2H), 4.48 (br dd, *J* = 2.9, 5.8 Hz, 1H), 4.42 - 4.32 (m, 2H), 3.92 - 3.84 (m, 1H), 3.79 - 3.65 (m, 2H), 3.18 (s, 3H), 1.39 (s, 3H), 1.36 - 1.31 (m, 2H), 1.28 (s, 3H), 1.26 - 1.22 (m, 2H).

### Example 26

### Synthetic route:

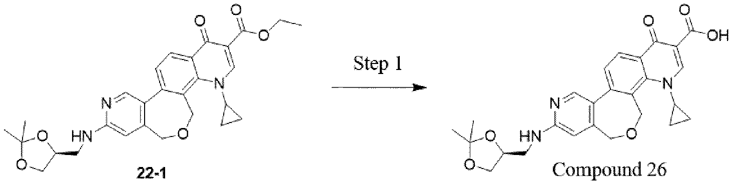

### Step 1: Preparation of Compound 26

At room temperature, a solution (0.5 mL) of sodium hydroxide (15.82 mg, 395.60 µmol) in water (0.5 mL) was added to Compound 22-1 (20.00 mg, 39.56 µmol) in methanol (1 mL), and then the mixture was stirred at 40°C for 16 hours. The reaction solution was filtered, the filtrate was purified through preparative HPLC (chromatographic column: Waters Xbridge 150*25mm* 5µm; mobile phase: 0.05% aqueous ammonium bicarbonate solution and acetonitrile; the proportion of acetonitrile in the mobile phase ranged from 25% to 55%, 10 min) to obtain Compound 26. LCMS (ESI) m/z: 478.3(M+1). ¹H NMR (400 MHz, CD₃OD) δ ppm 8.83 (br s, 1 H), 8.51 (br d, J=8.31 Hz, 1 H), 8.37 (m, 1 H), 7.69 (m, 1 H), 6.72 (s, 1 H), 4.56 - 4.68 (m, 2 H), 4.51 (s, 2 H), 4.36 - 4.44 (m, 1 H), 4.25 - 4.34 (m, 1 H), 4.13 (dd, J=8.25, 6.30 Hz, 1 H), 3.79 (dd, J=8.38, 6.30 Hz, 1 H), 3.60 (dd, J=8.19, 5.62 Hz, 2 H), 1.45 (s, 3 H), 1.41 (br d, J=6.72 Hz, 2 H), 1.37 (s, 3 H), 1.01 (br s, 2 H).

### Example 27

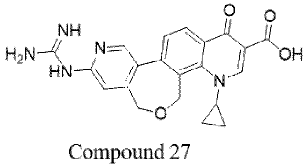

### Synthetic route:

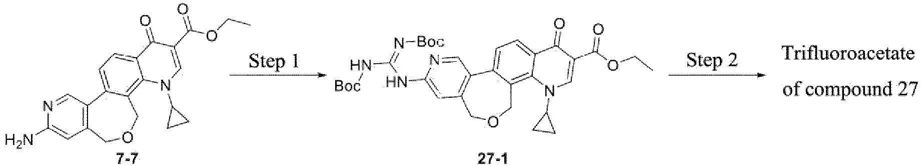

### Step 1: Preparation of Compound 27-1

At room temperature, Compound 7-7 (50 mg, 127.74 µmol) was dissolved in anhydrous dichloromethane (5 mL), N, N-di-tert-butyloxycarbonyl thiourea (42.36 mg, 153.29 µmol), triethylamine (38.78 mg, 383.22 µmol) and copper chloride (20.61 mg, 153.29 µmol) were successively added, and the reaction solution was stirred at 20°C for 12 hours. The reaction solution was filtered, concentrated under reduced pressure to obtain Compound 27-1. LCMS (ESI) m/z: 634.3 (M+1).

### Step 2: Preparation of trifluoroacetate of Compound 27

At room temperature, Compound 27-1 (80 mg, 126.24 µmol) was dissolved in a solution of ethanol (3 mL) and water (2 mL), 12M of hydrochloric acid (12 M, 2 mL) was added to the reaction solution, and the reaction solution was stirred at 80°C for 1 hour. The reaction solution was concentrated under reduced pressure to obtain a crude product. The crude product was purified through preparative HPLC (chromatographic column: Phenomenex Synergi Polar-RP 100*25mm*4µm; mobile phase: 0.05% aqueous trifluoroacetic acid solution and acetonitrile; the proportion of acetonitrile in the mobile phase ranged from 18% to 48%, 9 min), to obtain trifluoroacetate of Compound 27. LCMS (ESI) m/z: 406.2 (M+1).

### Example 28

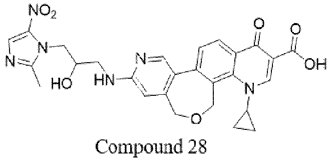

### Synthetic route:

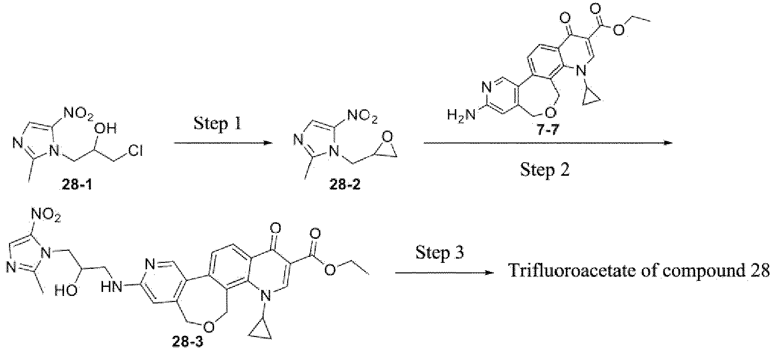

### Step 1: Preparation of Compound 28-2

At 0°C, Compound 28-1 (1 g, 4.55 mmol) was dissolve in DCM (10 mL), then a solution of sodium hydroxide (182.12 mg, 4.55 mmol) in water (10 mL) was slowly added, and the materials were reacted at a temperature of 0-25°C for 5 hours. The reaction solution was diluted with water (20 mL) and extracted with dichloromethane (60 mL). The obtained organic layer was washed with a saturated brine solution (30 mL), dried over anhydrous sodium sulfate, filtered and concentrated under reduced pressure to obtain Compound 28-2. LCMS (ESI) m/z: 184.1(M+1).

### Step 2: Preparation of Compound 28-3

Compound 28-2 (19.50 mg, 106.45 µmol), and Compound 7-7 (50 mg, 127.74 µmol) were dissolved in acetonitrile (1 mL), then calcium trifluoromethanesulfonate (15.00 mg, 40.67 µmol) was added, the mixture was stirred at 45°C for 16 hours, the raw materials were not reacted completely upon monitoring, and then the reaction solution was warmed to 60°C and continuously reacted for 16 hours. The reaction solution was filtered, and the filtrate was purified through preparative HPLC (chromatographic column: YMC Triart C18 150*25mm*5µm; mobile phase: [0.01% aqueous hydrochloric acid solution and acetonitrile; the proportion of acetonitrile in the mobile phase ranged from 16% to 46%, 8.5 min) to obtain Compound 28-3. LCMS (ESI) m/z: 575.3 (M+1).

### Step 3: Preparation of trifluoroacetate of Compound 28

At room temperature, a solution of sodium hydroxide (6.96 mg, 174.04 µmol) in water (0.5 mL) was added to a solution of Compound 28-3 (20 mg, 34.81 µmol) in anhydrous methanol (1 mL). The mixture was stirred at 25°C for 2 hours. The reaction solution was filtered, and the filtrate was purified through preparative HPLC (chromatographic column: Phenomenex Synergi Polar-RP 100*25mm*4µm; mobile phase [0.05% aqueous trifluoroacetic acid solution and acetonitrile; the proportion of acetonitrile in the mobile phase ranged from 15% to 45%, 9 min) to obtain trifluoroacetate of Compound 28. LCMS (ESI) m/z: 547.3 (M+1).

### Example 29

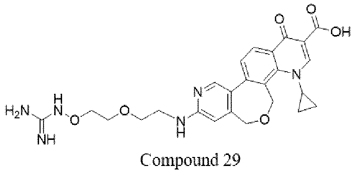

### Synthetic route:

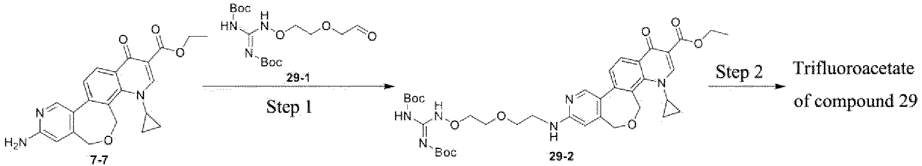

### Step 1: Preparation of Compound 29-2

At room temperature, Compound 7-7 (50 mg,127.74 µmol) and Compound 29-1 (92.33 mg, 255.48 µmol) were dissolved in methanol (2 mL) and dichloromethane (4 mL), then acetic acid (11.5 mg, 191.61 µmol) was added, and the mixture was stirred at 40°C for 1 hour. The reaction solution was cooled to 10-20°C, then pyridine-borane (35.61 mg, 383.22 µmol) was added, and the mixture was stirred at 40°C for 16 hours. The reaction solution was concentrated under reduced pressure to obtain Compound 29-2. LCMS (ESI) m/z: 737.4 (M+1).

### Step 2: Preparation of hydrochloride of Compound 29

At room temperature, Compound 29-2 (94 mg,127.58 µmol) was dissolved in water (2 mL) and methanol (3 mL), then concentrated hydrochloric acid (1.02 g, 10 mmol) was added, and the mixture was stirred at 50-55°C for 16 hours. The reaction solution was purified through preparative HPLC (chromatographic column: YMC Triart C18 150*25mm*5µm; mobile phase: 0.01% aqueous hydrochloric acid solution and acetonitrile; the proportion of acetonitrile in the mobile phase ranged from 9% to 29%, 10 min) to obtain hydrochloride of Compound 29. LCMS (ESI) m/z: 509.2 (M+1).

### Biological evaluation:

### Experimental example 1: Minimum inhibitory concentration (MIC) assay

### 1) Preparation of compound motherplate

On the day of the experiment, the compound in the flask was dissolved in 100% DMSO until the concentration of mother liquor was 0.4 mg/mL. If the mother liquor was not used on the same day, it was stored at -80°C for future use.

The compound mother liquor and standard antibiotics were subjected to 2-fold gradient dilution with corresponding solvents on a 96-microwell plate (V-bottom) to obtain 100 × working solutions (Well 1 to Well 7) in sequence. Among them, the concentrations of the compound were 400 µg/mL, 200 µg/mL, 100 µg/mL, 50 µg/mL, 25 µg/mL, 12.5 µg/mL, 6.25 µg/mL, and 3.125 µg/mL. 100% DMSO was used as growth control (Well 8). As such, the compound motherplate was obtained.

### 2) Preparation of inoculating solution

One day before the experiment, aerobic bacteria *(Staphylococcus epidermidis)* stored in a cryogenic tube at -80°C were scribed on an MHA (Cation-adjusted Mueller-Hinton agar) plate. The aerobic bacteria were placed in a 35+/-2°C incubator for 18-24 hours of aerobic cultivation.

Two days before the experiment, anaerobic bacteria *(Propionibacterium acnes)* stored in a cryogenic tube at - 80°C were scribed on an MBA (Brucella Agar+5% (v/v) defibrillated sheep blood+5 µg/Ml Hemin+ 1 µg/mL Vitamin K₁) plate. The anaerobic bacteria were placed in a 35+/-2°C anaerobic incubator for 42-48 hours of cultivation.

On the day of the experiment, the plate was taken out, clones were picked from the plate and suspended in physiological saline. Then, the turbidity of the bacterial suspension (containing 1.0-2.0×10⁸ CFU/mL) was adjusted to OD600=0.2 by a turbidity meter. Then, the adjusted bacterial suspension was diluted to an appropriate concentration with the assay culture medium: about 5×10⁵ CFU/mL for aerobic bacteria, and about 1×10⁶ CFU/mL for anaerobic bacteria. As such, the inoculating solution was obtained.

### 3) Minimum inhibitory concentration (MIC) assay

2 µl of 2-fold series dilution of 100× high-concentration working solution was transferred from a compound mother plate (prepared in step 1) to a round-bottom 96-well plate, and then 198 µL of bacterial inoculating solution (prepared in step 2) was added to each well to obtain an MIC assay plate. Therefore, the final assay concentrations of the compound were 4 µg/mL, 2 µg/mL, 1 µg/mL, 0.5 µg/mL, 0.25 µg/mL, 0.125 µg/mL, 0.06 µg/mL and 0.03 µg/mL. The total volume of each well on the assay plate was 200 µL: containing 1% DMSO, about 5×10⁵ CFU/mL of aerobic bacteria and about 1×10⁶ CFU/mL of anaerobic bacteria.

All assay plates for aerobic bacteria were aerobically cultured at 35+/-2°C for 16 to 20 hours, and all assay plates for anaerobic bacteria were anaerobically cultured in an anaerobic incubator at 35+/-2°C for 48 hours.

Note: for anaerobic bacteria, both the preparation of bacterial solution in step 2 and the inoculation of bacterial solution in step 3 were operated in an anaerobic workstation.

### 4) Reading of Minimum inhibitory concentration (MIC) assay

After cultivation, by observing the assay plates with naked eyes, the concentration of drug contained in the wells in which the bacterial growth was completely or significantly inhibited was the minimum inhibitory concentration.

### 5) The experimental results are shown in Table 1.

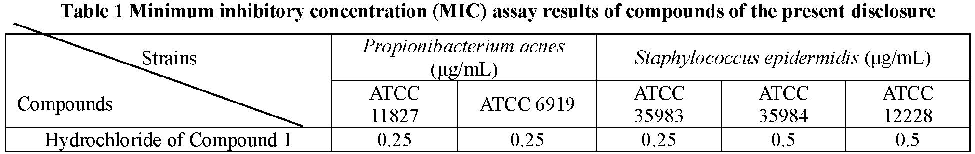

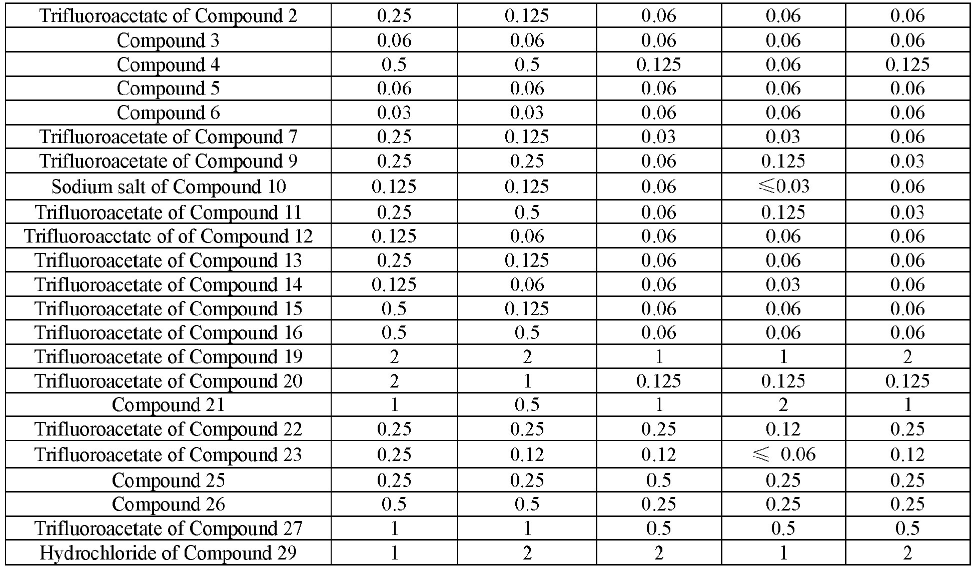

**Experimental conclusions:** the compound of the present disclosure has good in vitro bacteriostatic activity.

### Experiment example 2: Pharmacokinetic evaluation of compounds

### 1) Experimental materials:

CD-1 mice (male, Beijing Vital River Laboratory Animal Technology Co., Ltd.)

### 2) Experimental Operations:

The pharmacokinetic characteristics of rodents after intravenous and oral administration of the compounds were tested using a standard protocol. In the experiment, the candidate compounds were prepared into clear solutions and administered to mice by single intravenous injection and oral administration. The intravenous and oral vehicles were mixed vehicles of 10%DMSO+90% (20%SBE-β-CD). This project included four male CD-1 mice, two of which were administered intravenously at a dosage of 1 mg/kg, and plasma samples were collected at 0.083 hours, 0.25 hours, 0.5 hours, 1 hour, 2 hours, 4 hours, 8 hours, and 24 hours after administration; the other two mice were orally administered by gavage at a dosage of 2 mg/kg, and plasma samples were collected at 0.25 hours, 0.5 hours, 1 hour, 2 hours, 4 hours, 8 hours, and 24 hours after administration. Blood samples were placed on ice after collection, and plasma was separated by centrifugation within 1 hour (centrifugation conditions: 6000g, 3 minutes, 2-8°C). Plasma samples were stored in a refrigerator at -80°C before analysis. Plasma concentrations were quantitatively analyzed using LC-MS/MS analysis method, and pharmacokinetic parameters were calculated such as peak concentration (Cₘₐₓ), clearance rate (CL), half-life (T_{1/2}), tissue distribution (V_{dss}), area under concentration-time curve (AUC₀₋ₗₐₛₜ), and bioavailability (F), etc.

### 3) The experimental results are shown in Table 4.

**Table 4 Pharmacokinetic test results of the compounds of the present disclosure**

| Compounds | Peak concentration Cₘₐₓ (nM) | Clearance rate CL (mL/min/kg) | Tissue distribution Vass (L/kg) | Half-life T_{1/2} (IV, h) | Area under concentration-time curve AUC₀₋ₗₐₛₜ PO (nM.hr) | Bioavailability F (%) |
|---|---|---|---|---|---|---|
| Hydrochloride of Compound 7 | 683 | 132 | 3.04 | 0.341 | 924 | 132 |

**Experimental conclusions:** the compounds of the present disclosure have good pharmacokinetic properties, including good oral bioavailability and oral exposure.

### Experiment example 3: in-vivo pharmacodynamic study of compounds

### 1) Experimental materials

**Experimental strain:** *propionibacterium acnes ATCC 6919* (Beijing Biobw Biotechnology Co., Ltd.)
**Experimental animals:** Balb/C mice (Zhejiang Vital River Laboratory Animal Technology Co., Ltd.)

### 2) Experimental methods

### 1. Establishment of Acne Model:

Balb/C mice were grouped and numbered, and adaptively fed for one week. On the day of modeling, *Propionibacterium acnes* was taken, and OD600 was measured using a microplate reader. BHI culture medium was diluted to OD600 = 1 (about 5×10⁸ CFU/mL), centrifuged to discard supernatant, and the remaining culture was diluted to 1×10⁸ CFU/mL with PBS. Once the molding began, the mice were anesthetized with a small animal anesthesia machine, the air flow and isoflurane content of the anesthesia machine were adjusted, and the mice were placed in an anesthesia induction chamber for anesthesia induction. After there were no obvious signs of activity, the mice were taken out and their toes were clamped with tweezers or hemostatic forceps for toe pinching reaction testing, if there was no retraction pain response, the surgery could be performed. The anesthetized mice were placed on a mice plate and fixed, the air flow or isoflurane content was adjusted to maintain anesthesia (isoflurane anesthesia induction content of 3% to 4%, anesthesia maintenance content of 1% to 2%, and air flow rate of 300 mL/min to 500 mL/min). Blank control group: 20 µL of PBS was intracutaneously injected into the left ear of each mouse. Model group, drug group to be tested: 20 µL suspension of *propionibacterium acnes* in PBS was intracutaneously injected into the left ear of each mouse. After the injection of *Propionibacterium acnes,* the mice in each group were administered according to the methods of administration and dosages described in Table 5 at 4 hours, 20 hours, 28 hours, and 44 hours.

After *Propionibacterium acnes* was injected for 48 hours, photographs of ears of mice were taken in each group at the endpoint of the experiment, the left auricular thicknesses were measured with a vernier caliper, the left ear was taken for tissue homogenate, and the ear bacterial load and IL-6, IL-8 levels (mice do not express IL-8, so a functional homolog CXCL1/KC was detected) were measured.

### 2. Formulation of drugs:

The drugs to be tested included Compound 6, Compound 7, and Compound 12, all of which were in a form of hydrochloride. In this experiment, Compound 6, Compound 7, and Compound 12 all referred to the corresponding hydrochloride drugs with a solvent of 40% DMSO+60% glycerol, the drugs were added into DMSO, heated to 100°C in a water bath, and then glycerol was added thereto to obtain a bright yellow viscous solution.

3. The methods of administration and dosages are shown in Table 5 below.

**Table 5 Methods of Administration and Dosages**

| Groups | Administration Area | Tested Drugs | Administration Do sage | Administration Frequency | Administration Method |
|---|---|---|---|---|---|
| Blank control group | Molding Ear | - | - | - | - |
| Compound 6 group | Molding Ear | Compound 6 | 15 µL | 4 hours, 20 hours, 28 hours, and 44 hours after injection of *Propionibacterium acnes* | Administration by applying with a pipette gun |
| Compound 7 group | Modeling Ear | Compound 7 | 15 µL | 4 hours, 20 hours, 28 hours, and 44 hours after injection of *Propionibacterium acnes* | Administration by applying with a pipette gun |
| Compound 12 group | Modeling Ear | Compound 12 | 15 µL | 4 hours, 20 hours, 28 hours, and 44 hours after injection of *Propionibacterium acnes* | Administration by applying with a pipette gun |
| Model group | Modeling Ear | Blank solvent | 15 µL | 4 hours, 20 hours, 28 hours, and 44 hours after injection of *Propionibacterium acnes* | Administration by applying with a pipette gun |

Administration area: *Propionibacterium acnes* was injected into the left ear.

Administration time: the drugs were administrated at 4 hours, 20 hours, 28 hours, and 44 hours post-injection of *Propionibacterium acnes,* a total of 4 times.

### 4. Modeling method:

Balb/C mice were anesthetized with a small animal anesthesia machine and isoflurane in Compound 6 group, Compound 7 group, Compound 12 group, and model group. After the anesthesia was maintained in plateau, 20 µL suspension of 1×10⁸ CFU/mL *Propionibacterium acnes* in PBS was injected into the left ears of mice in the model group and the drug group to be tested using a 1 mL sterile syringe, while blank control group was injected with an equal volume of 1× PBS.

### 5. Detection indexes:

Macroscopic photography: after *Propionibacterium acnes* was injected for 48 hours, the ears of mice were roughly photographed.

Auricular thickness measurement: after *Propionibacterium acnes* was injected for 48 hours, the auricular thicknesses of mice were measured with a vernier caliper.

Ear bacterial load detection: after *Propionibacterium acnes* was injected for 48 hours, the left ear of the mouse was homogenized with 1 mL of PBS, and 3 portions of 200 µL homogenate were taken, to which 800 µL of PBS was added for dilution. 100 µL of the diluted homogenate was subjected to 10-fold dilution, and the above operation was repeated to dilute a total of 5 gradients. The homogenates of each gradient were coated onto BHI solid culture medium, and incubated in an anaerobic environment at 37°C for 48 hours before calculating the number of colonies.

Mouse BCA protein detection: after *Propionibacterium acnes* was injected for 48 hours, the BCA protein detection was detected by using the BCA protein concentration assay kit. The left ear of mouse was homogenized with 1 mL of PBS and the dilution factor equivalent to a mass volume ratio of 1:9 was calculated, then centrifuged at 1500 g for 10 minutes. Each group of samples was diluted 10 times with PBS. 20 µL of sample was taken and added to a 96-well plate. 200 µL of BCA working solution was added to each well, and placed at 37°C for 15-30 minutes. A562 was measured with a microplate reader, the standard curves were plotted, and the BCA concentrations were calculated. As such, the IL-6 level and the CXCL1 level were obtained.

### 3) Experimental results are shown in Table 6, Table 7 and Table 8.

**Table 6: Effect of tested drugs on auricular thicknesses of mice in acne model**

| Groups | Auricular thickness (mm) Mean ± SD | Auricular thickness (% of Blank) Mean ± SD |
|---|---|---|
| Blank control group | 0.45 ± 0.02 | 100.00 ± 3.77 |
| Compound 6 group | 0.51 ± 0.08^{##} | 112.71 ± 17.19^{##} |
| Compound 7 group | 0.39 ± 0.03^{##} | 85.31 ± 7.43^{##} |
| Compound 12 group | 0.44 ± 0.04^{###} | 97.24 ± 8.70^{###} |
| Model group | 0.61 ± 0.03*** | 135.36 ± 7.30*** |

After *Propionibacterium acnes* was injected for 48 hours, the auricular thicknesses of mice in each group were measured using a digital vernier caliper. The results are expressed in Mean ± SD. Compared with the blank control group, *p<0.05, **p<0.01, ***p<0.001; compared with the model group, ^{#}p<0.05, ^{##}p<0.01, ^{###}p<0.001.

**Table 7: Effect of tested drugs on ear bacterial loads of mice in acne model**

| Groups | Log₁₀ CFU/Ear Mean ± SD |
|---|---|
| Blank control group | - |
| Compound 6 group | 5.53 ± 0.10*** |
| Compound 7 group | 5.36 ± 0.03*** |
| Compound 12 group | 5.35 ± 0.10*** |
| Model group | 6.29±0.06 |

After *Propionibacterium acnes* was injected for 48 hours, the tissues of the left ear were taken for bacterial load testing. The results are expressed in Mean ± SD. Compared with the model group, *p<0.05, **p<0.01, ***p<0.001.

There were no change in the left ears of mice in blank control group, and there were obvious redness and swelling on the left ears of mice in acne model group. After applying Compound 6, Compound 7 and Compound 12 for treatment, the redness and swelling on the left ears of mice were reduced to varying degrees, and the situation was improved.

It can be seen that from Table 6 that compared with the mice in blank control group, the auricular thicknesses of the left ears of mice in acne model group are significantly increased (p<0.001), however after applying Compound 6, Compound 7 and Compound 12 for treatment, the auricular thicknesses of the left ears of mice are significantly reduced (p<0.05), wherein after treatment with Compound 7, an optimal efficacy is achieved.

It can be seen that from Table 7 that after applying Compound 6, Compound 7 and Compound 12 for treatment, the bacterial loads in the left ears of mice are significantly decreased (p<0.001), wherein after treatment with Compound 7, the bacterial loads in the left ears of mice are most significantly reduced.

**Table 8: Effect of tested drugs on IL-6 and CXCL1 levels of mice in acne model**

| Groups | IL-6 (pg/mg) Mean ± SD | CXCL1 (pg/mg) Mean ± SD |
|---|---|---|
| Blank control group | 215.62 ± 41.74 | 161.46 ± 16.68 |
| Compound 6 group | 322.4 ± 43.33^{###} | 191.5 ± 32.71^{###} |
| Compound 7 group | 261.67 ± 40.82^{###} | 185.95 ± 19.42^{###} |
| Compound 12 group | 266.06 ± 55.11^{###} | 181.08 ± 34.97^{###} |
| Model group | 757.95 ± 60.00*** | 426.02 ± 34.31*** |

After *Propionibacterium acnes* was injected for 48 hours, the auricular thicknesses of mice in each group were measured using a digital vernier caliper. The results are expressed in Mean ± SD. Compared with blank control group, *p<0.05, **p<0.01, ***p<0.001; compared with model group, ^{#}p<0.05, ^{##}p<0.01, ^{###}p<0.001.

It can be seen that from Table 8 that compared with blank control group, the IL-6 and CXCL1 contents in the left ear tissues of mice in the acne model group are significantly increased (p<0.001), however after applying Compound 6, Compound 7 and Compound 12 for treatment, the IL-6 and CXCL1 contents in the left ear tissues of mice are both significantly reduced (p<0.001), wherein after treatment with Compound 7, and optimal efficacy is achieved.

### 4) Experimental conclusions

Animal experiments were conducted on the effects of different drugs on the disorders of acne model mice herein, wherein acne models and immunosuppressive mice acne models were established, respectively to prove that the tested drugs can improve the disorders of acne model mice and reduce the IL-6 and CXCL-1 levels in the focal tissues. The main study results are as follows: all the Compound 6, Compound 7, and Compound 12 can improve the thicknesses of the infected ears of mice in the acne model and alleviate redness and swelling symptoms, and can reduce the bacterial loads in the infected ears of mice in the acne model and inhibit the growth of *Propionibacterium acnes,* as well as can reduce the contents of inflammatory factors IL-6 and CXCL1 in the infected ears of mice in the acne model.

## Claims

1. A compound represented by formula (II), a stereoisomer, or a pharmaceutically acceptable salt thereof, 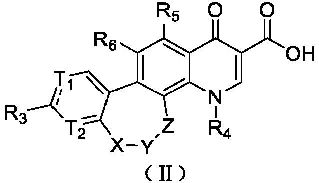 wherein, 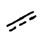
is selected from a single bond and a double bond;
T₁ is selected from the group consisting of N, NH, CR₁ and N⁺R₁(A⁻);
T₂ is selected from the group consisting of N and CR₂;
A⁻ is selected from the group consisting of F⁻, Cl⁻, Br⁻, I⁻, OH⁻ and HCO₃⁻;
X is selected from the group consisting of -C(R₇R₈)- and -C(R₇R₈)-C(R₇R₈)-;
Y is selected from the group consisting of -O-, -S-, -NH-, and -C(R₇R₈)-;
Z is -C(R₇R₈)-;
alternatively, -Y-Z- is selected from the group consisting of -C(R₉)=C(R₉)-;
R₁ is selected from the group consisting of H, F, Cl, Br, I, -OH, -NH₂, -CN and -CH₃;
R₂ is selected from the group consisting of H, F, Cl, Br, I, -OH, -NH₂, -CN and C₁₋₃ alkyl, wherein the C₁₋₃ alkyl is optionally substituted by 1, 2, or 3 Rₐ;
R₃ is selected from the group consisting of H, F, Cl. Br, I, =O, -OH, -NH₂, -CN, -NHC(=NH)NH₂, C₁₋₃ alkyl, C₁₋₃ alkylamino and C₁₋₃ alkoxy, wherein the C₁₋₃ alkyl, C₁₋₃ alkylamino and C₁₋₃ alkoxy are independently optionally substituted by 1, 2, or 3 R_{b}, respectively;
alternatively, R₁ and R₃ together with the atoms to which they are attached form 5-6 membered heterocycloalkyl, 5-6 membered heterocycloalkenyl, or 5-6 membered heteroaryl, wherein the 5-6 membered heterocycloalkyl, 5-6 membered heterocycloalkenyl and 5-6 membered heteroaryl are independently optionally substituted by 1, 2, 3 or 4 R_{c}, respectively;
R₄ is selected from the group consisting of H, C₁₋₃ alkyl, C₃₋₆ cycloalkyl, phenyl and 5-6 membered heteroaryl, wherein the C₁₋₃ alkyl, C₃₋₆ cycloalkyl, phenyl and 5-6 membered heteroaryl are independently optionally substituted by 1, 2, 3 or 4 R_{d}, respectively;
R₅ is selected from the group consisting of H, F, Cl, Br, I, -OH, -NH₂ and -CN;
R₆ is selected from the group consisting of H, F, Cl, Br, I, -OH, -NH₂ and -CN;
R₇, R₈ and R₉ are each independently selected from the group consisting of H, F, Cl, Br, I, -OH, -NH₂ and - CN, respectively;
each Rₐ is independently selected from the group consisting of F, Cl, Br, I, -OH, -NH₂ and -CN, respectively;
each R_{b} is independently selected from the group consisting of F, Cl, Br, I, -OH, -NH₂, -CN, C₁₋₃ alkoxy, C₃₋₆ cycloalkyl, 3-6 membered heterocycloalkyl, 5-6 membered heteroaryl and phenyl, respectively, wherein the C₁₋₃ alkoxy, C₃₋₆ cycloalkyl, 3-6 membered heterocycloalkyl, 5-6 membered heteroaryl and phenyl are independently optionally substituted by 1, 2, 3 or 4 R, respectively;
each R_{c} is independently selected from the group consisting of F, Cl, Br, I, -OH, -NH₂, -CN and -CH₃, respectively;
each R_{d} is independently selected from the group consisting of F, Cl, Br, I, -OH, -NH₂ and -CN, respectively; each R is independently selected from the group consisting of F, Cl, Br, I, -OH, -NH₂, -NO₂, -CN, - ONHC(=NH)NH₂ and C₁₋₃ alkyl, respectively; and
the "hetero" in the 5-6 membered heterocycloalkyl, 3-6 membered heterocycloalkyl, 5-6 membered heterocycloalkenyl and 5-6 membered heteroaryl means 1, 2, 3 or 4 heteroatoms or heteroatom groups independently selected from the group consisting of O, NH, S and N, respectively.

2. The compound, the stereoisomer, or the pharmaceutically acceptable salt thereof of claim 1, wherein each R is independently selected from the group consisting of -NO₂, -ONHC(=NH)NH₂ and -CH₃, respectively.

3. The compound, the stereoisomer, or the pharmaceutically acceptable salt thereof of claim 1, wherein each R_{b} is independently selected from the group consisting of F, Cl, Br, -OH, -NH₂, -OCH₂CH₃, cyclobutyl, oxetanyl, oxacyclopentyl, azetidinyl, 5-membered heteroaryl and phenyl, wherein the -OCH₂CH₃, cyclobutyl, oxetanyl, oxacyclopentyl, azetidinyl, 5-membered heteroaryl and phenyl are independently optionally substituted by 1, 2, 3 or 4 R, respectively; preferably, each R_{b} is independently selected from the group consisting of F, -OH, -NH₂,

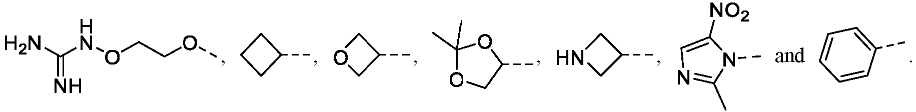

4. The compound, the stereoisomer, or the pharmaceutically acceptable salt thereof of claim 1, wherein each R_{c} is independently -CH₃, respectively.

5. The compound, the stereoisomer, or the pharmaceutically acceptable salt thereof of claim 1, wherein each R_{d} is independently selected from the group consisting of F, Cl, Br and -NH₂, respectively.

6. The compound, the stereoisomer, or the pharmaceutically acceptable salt thereof of claim 1, wherein R₁ is H;
and/or, R₂ is selected from the group consisting of H and -CH₃.

7. The compound, the stereoisomer, or the pharmaceutically acceptable salt thereof of claim 1, wherein R₃ is selected from the group consisting of H, F, Cl, Br, =O, -OH, -NH₂, -CN, -NHC(=NH)NH₂, -CH₃, -NHCH₃, - N(CH₃)₂, -NHCH₂CH₃, -NHCH₂CH₂CH₃, -N(CH₃)CH₂CH₃ and -OCH₃, wherein the -NHC(=NH)NH₂, -CH₃, - NHCH₃, -N(CH₃)₂, -NHCH₂CH₃, -NHCH₂CH₂CH₃, -N(CH₃)CH₂CH₃ and -OCH₃ are independently optionally substituted by 1, 2, or 3 R_{b}, respectively; preferably, R₃ is selected from the group consisting of H, =O, -NH₂, - NHC(=NH)NH₂, -CH₃, -NHCH₃, -N(CH₃)₂, -NHCH₂CH₃, -NHCH₂CH₂CH₃, -NHCH₂CH₂OH, -NHCH₂CH₂NH₂, -OCH₃,

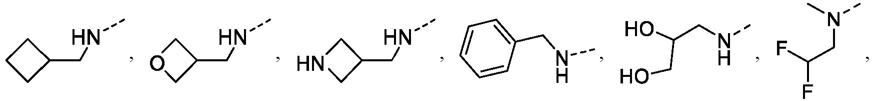

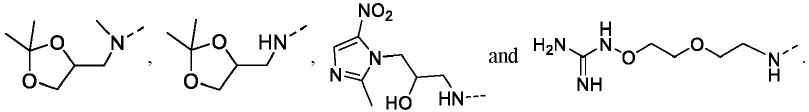

8. The compound, the stereoisomer, or the pharmaceutically acceptable salt thereof of claim 1, wherein R₄ is selected from the group consisting of H, -CH₂CH₂F,

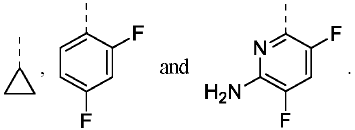

9. The compound, the stereoisomer, or the pharmaceutically acceptable salt thereof of claim 1, wherein R₅ and R₆ are independently selected from the group consisting of H and F.

10. The compound, the stereoisomer, or the pharmaceutically acceptable salt thereof of claim 1, wherein R₇, R₈ and R₉ are each independently H, respectively.

11. The compound, the stereoisomer, or the pharmaceutically acceptable salt thereof of claim 1, wherein R₁ and R₃ together with the atoms to which they are attached form 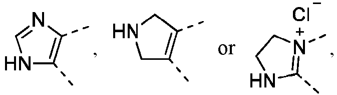 wherein the 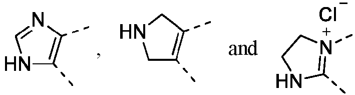 are independently optionally substituted by 1, 2, 3 or 4 R_{c}, respectively; preferably, R₁ and R₃ together with the atoms to which they are attached form

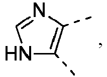

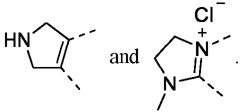

12. The compound, the stereoisomer, or the pharmaceutically acceptable salt thereof of claim 1, wherein the compound has a structure represented by formula (II-2): 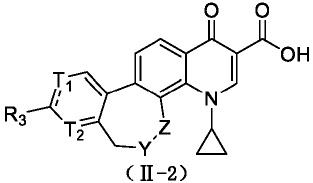 wherein, X, Y, Z, T₁, T₂, R₃, R₅ and R₆ are as defined in claim 1.

13. The compound, the stereoisomer, or the pharmaceutically acceptable salt thereof according to claim 1, wherein the compound is selected from:

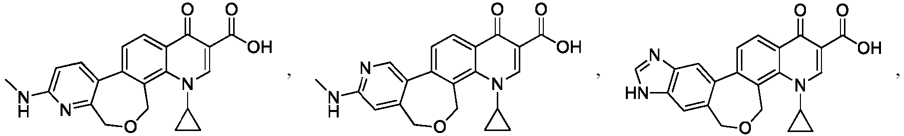

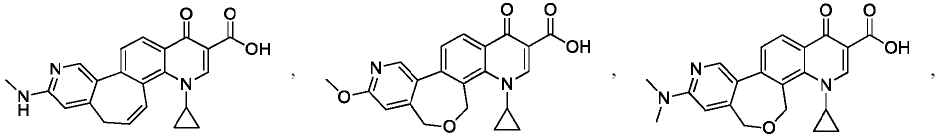

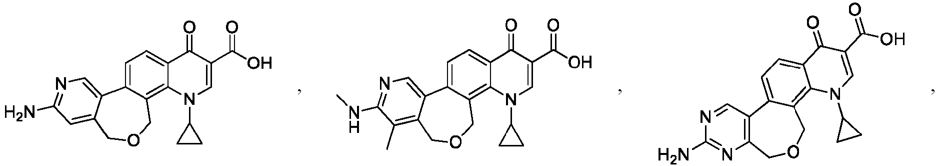

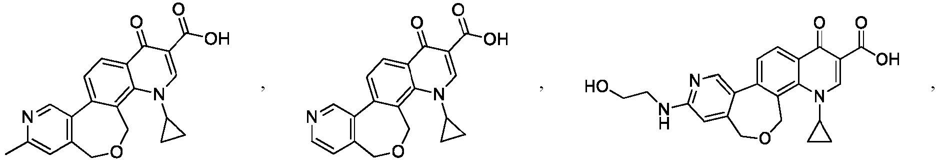

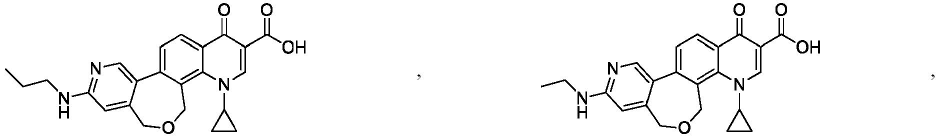

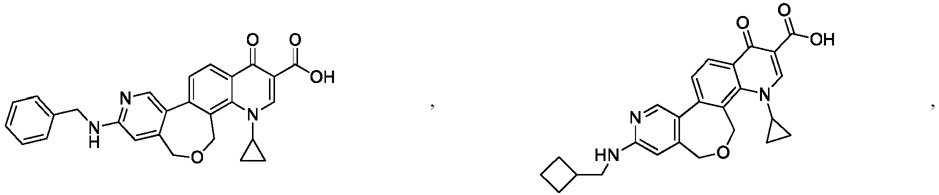

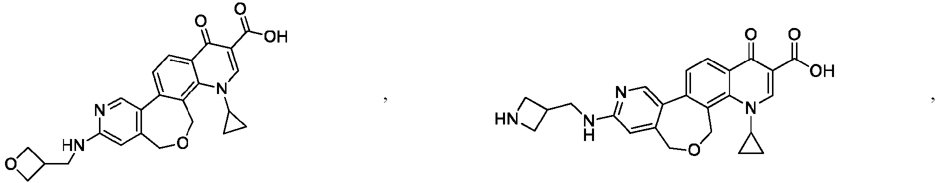

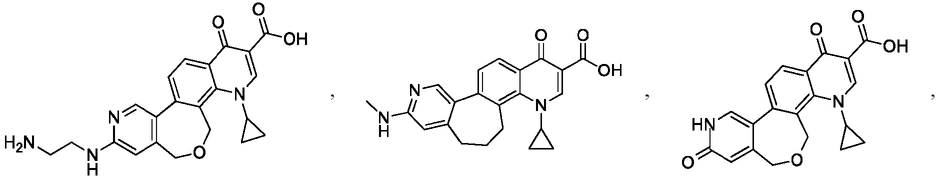

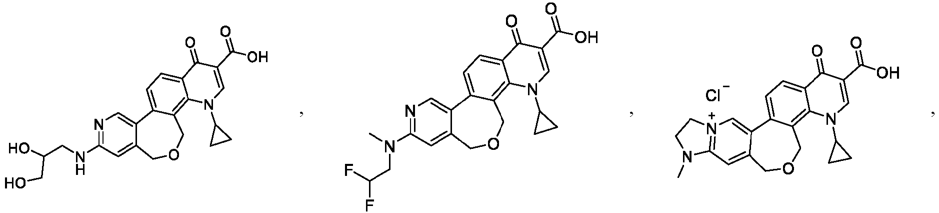

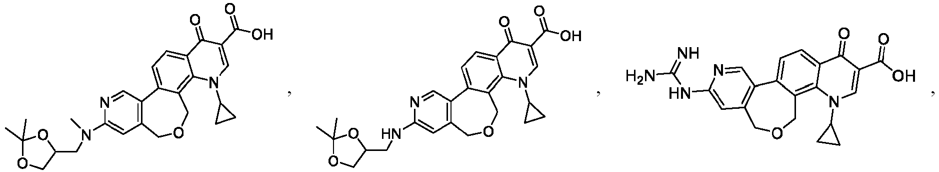

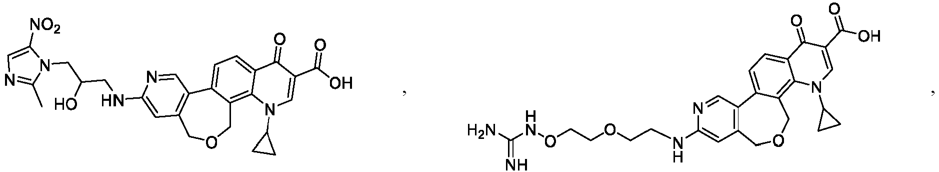

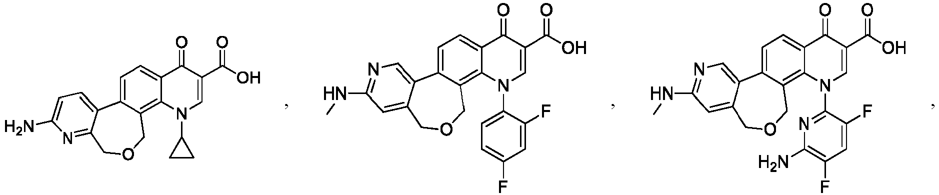

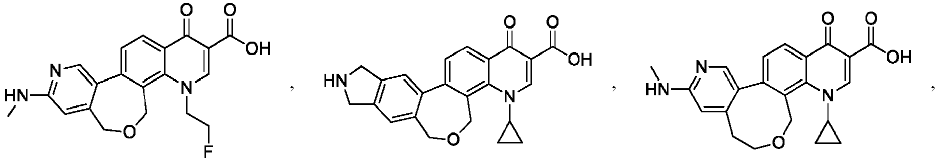

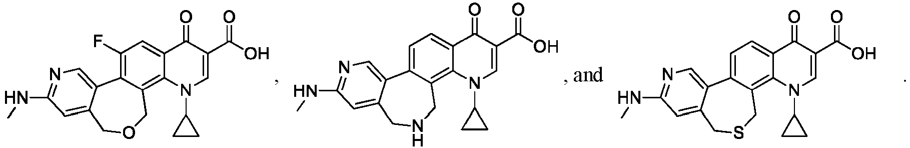

14. The compound, the stereoisomer, or the pharmaceutically acceptable salt thereof according to claim 13, wherein the compound is selected from:

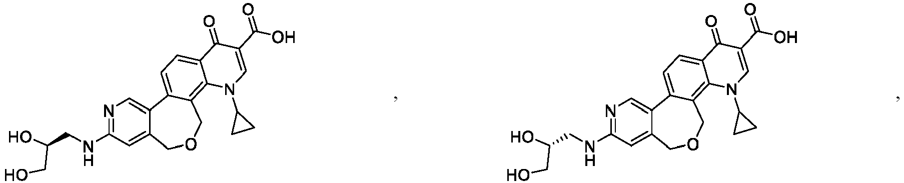

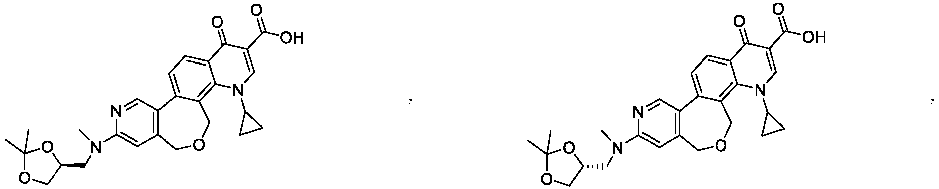

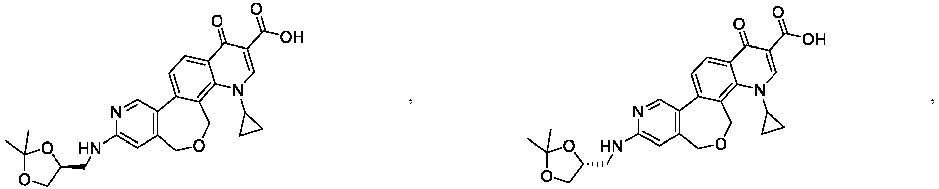

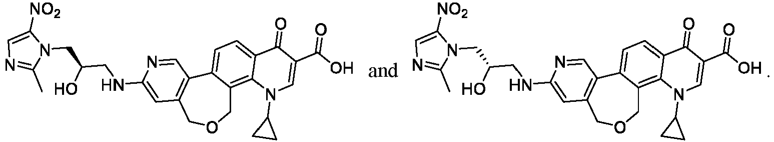

15. The compound, the stereoisomer, or the pharmaceutically acceptable salt thereof of any one of claims 1 to 14 for use as antibacterial and anti-inflammatory drugs preferably, for use in the treatment of acne.

## Patentansprüche

1. Eine Verbindung, dargestellt durch Formel (II), ein Stereoisomer oder ein pharmazeutisch akzeptables Salz davon, 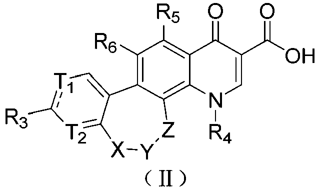 wobei, 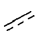
ausgewählt ist aus einer Einfachbindung und einer Doppelbindung;
T₁ ausgewählt ist aus der Gruppe bestehend aus N, NH, CR₁ und N⁺R₁(A⁻);
T₂ ausgewählt ist aus der Gruppe bestehend aus N und CR₂;
A⁻ ausgewählt ist aus der Gruppe bestehend aus F⁻, Cl⁻, Br⁻, I⁻, OH⁻ und HCO₃⁻;
X ausgewählt ist aus der Gruppe bestehend aus -C(R₇R₈)- und -C(R₇R₈)-C(R₇R₈)-;
Y ausgewählt ist aus der Gruppe bestehend aus -O-, -S-, -NH- und -C(R₇R₈)-;
Z ist: -C(R₇R₈)-;
ersatzweise -Y-Z- ausgewählt aus der Gruppe bestehend aus -C(R₉)=C(R₉)- ist;
R₁ ausgewählt aus der Gruppe bestehend aus H, F, Cl, Br, I, -OH, -NH₂, -CN und -CH₃ ist;
R₂ ausgewählt aus der Gruppe bestehend aus H, F, Cl, Br, I, -OH, -NH₂, -CN und C₁₋₃-Alkyl ist, wobei das C₁₋₃-Alkyl wahlweise durch 1, 2 oder 3 Rₐ substituiert ist;
R₃ ausgewählt aus der Gruppe bestehend aus H, F, Cl, Br, I, =O, -OH, -NH₂, -CN, -NHC(=NH)NH₂, C₁₋₃-Alkyl, C₁₋₃-Alkylamino und C₁₋₃-Alkoxy ist, wobei das C₁₋₃-Alkyl, das C₁₋₃-Alkylamino und das C₁₋₃-Alkoxy wahlweise unabhängig voneinander durch 1, 2 bzw. 3 R_{b} substituiert sind;
ersatzweise R₁ und R₃ zusammen mit den Atomen, an die sie gebunden sind, ein 5-6-gliedriges Heterocycloalkyl, ein 5-6-gliedriges Heterocycloalkenyl oder ein 5-6-gliedriges Heteroaryl bilden, wobei das 5-6-gliedrige Heterocycloalkyl, das 5-6-gliedrige Heterocycloalkenyl und das 5-6-gliedrige Heteroaryl unabhängig voneinander jeweils wahlweise durch 1, 2, 3 bzw. 4 R_{c} substituiert sind;
R₄ ausgewählt aus der Gruppe bestehend aus H, C₁₋₃-Alkyl, C₃₋₆-Cycloalkyl, Phenyl und 5-6-gliedrigem Heteroaryl ist, wobei das C₁₋₃-Alkyl, das C₃₋₆-Cycloalkyl, das Phenyl und das 5-6-gliedrige Heteroaryl unabhängig voneinander jeweils wahlweise durch 1, 2, 3 bzw. 4 R_{d} substituiert sind;
R₅ ausgewählt aus der Gruppe bestehend aus H, F, Cl, Br, I, -OH, -NH₂ und -CN ist;
R₆ ausgewählt aus der Gruppe bestehend aus H, F, Cl, Br, I, -OH, -NH₂ und -CN ist;
R₇, R₈ und R₉ jeweils unabhängig voneinander aus der Gruppe ausgewählt sind, die jeweils aus H, F, Cl, Br, I, -OH, -NH₂ und -CN besteht;
jedes Rₐ unabhängig voneinander ausgewählt ist aus der Gruppe bestehend jeweils aus F, Cl, Br, I, -OH, -NH₂ und -CN;
jedes R_{b} unabhängig voneinander aus der Gruppe ausgewählt ist, die jeweils aus F, Cl, Br, I, -OH, -NH₂, - CN, C₁₋₃-Alkoxy, C₃₋₆-Cycloalkyl, 3-6-gliedrigem Heterocycloalkyl, 5-6-gliedrigem Heteroaryl und Phenyl besteht, wobei das C₁₋₃-Alkoxy, das C₃₋₆-Cycloalkyl, das 3-6-gliedrige Heterocycloalkyl, das 5-6 -gliedrige Heteroaryl und das Phenyl jeweils unabhängig voneinander wahlweise durch 1, 2, 3 bzw. 4 R substituiert sind;
jedes R_{c} unabhängig voneinander ausgewählt ist aus der Gruppe bestehend aus F, Cl, Br, I, -OH, -NH₂, -CN bzw. -CH₃;
jedes R_{d} unabhängig voneinander ausgewählt ist aus der Gruppe bestehend aus F, Cl, Br, I, -OH, -NH₂ bzw. -CN;
jedes R unabhängig voneinander ausgewählt ist aus der Gruppe bestehend aus F, Cl, Br, I, -OH, -NH₂, -NO₂, -CN, -ONHC(=NH)NH₂ bzw. C₁₋₃-Alkyl; und
das "Hetero" in dem 5-6-gliedrigen Heterocycloalkyl, 3-6-gliedrigen Heterocycloalkyl, 5-6-gliedrigen Heterocycloalkenyl und 5-6-gliedrigen Heteroaryl 1, 2, 3 oder 4 Heteroatome oder Heteroatomgruppen bedeutet, die jeweils unabhängig voneinander aus der Gruppe ausgewählt sind, die jeweils aus O, NH, S und N besteht.

2. Die Verbindung, das Stereoisomer oder das pharmazeutisch akzeptable Salz davon von Anspruch 1, wobei jedes R unabhängig voneinander aus der Gruppe ausgewählt ist, die aus -NO₂, -ONHC(=NH)NH₂ bzw. -CH₃ besteht.

3. Die Verbindung, das Stereoisomer oder das pharmazeutisch akzeptable Salz davon von Anspruch 1, wobei jedes R_{b} unabhängig voneinander aus der Gruppe ausgewählt ist, die aus F, Cl, Br, -OH, -NH₂, -OCH₂CH₃, Cyclobutyl, Oxetanyl, Oxacyclopentyl, Azetidinyl, 5-gliedrigem Heteroaryl und Phenyl besteht, wobei das - OCH₂CH₃, Cyclobutyl, Oxetanyl, Oxacyclopentyl, Azetidinyl, das 5-gliedrige Heteroaryl und das Phenyl unabhängig voneinander wahlweise durch 1, 2, 3 bzw. 4 R substituiert sind; vorzugsweise ist jedes R_{b} unabhängig voneinander aus der Gruppe ausgewählt, die aus F, -OH, -NH₂, 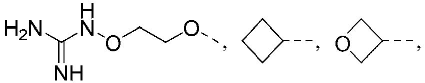 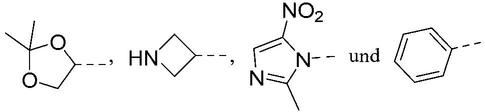 besteht.

4. Die Verbindung, das Stereoisomer oder das pharmazeutisch akzeptable Salz davon von Anspruch 1, wobei jedes R_{c} jeweils unabhängig voneinander -CH₃ ist.

5. Die Verbindung, das Stereoisomer oder das pharmazeutisch akzeptable Salz davon von Anspruch 1, wobei jedes R_{d} unabhängig voneinander aus der Gruppe ausgewählt ist, die aus F, Cl, Br bzw. -NH₂ besteht.

6. Die Verbindung, das Stereoisomer oder das pharmazeutisch akzeptable Salz davon von Anspruch 1, wobei R₁ = H ist;
und/oder R₂ ausgewählt ist aus der Gruppe bestehend aus H und -CH₃.

7. Die Verbindung, das Stereoisomer oder das pharmazeutisch akzeptable Salz davon von Anspruch 1, wobei R₃ ausgewählt ist aus der Gruppe bestehend aus H, F, Cl, Br, =O, -OH, -NH₂, -CN, -NHC(=NH)NH₂, -CH₃, - NHCH₃, -N(CH₃)₂, -NHCH₂CH₃, -NHCH₂CH₂CH₃, -N(CH₃)CH₂CH₃ und -OCH₃, wobei das -NHC(=NH)NH₂, - CH₃, -NHCH₃, -N(CH₃)₂, -NHCH₂CH₃, -NHCH₂CH₂CH₃, -N(CH₃)CH₂CH₃ und -OCH₃ unabhängig voneinander wahlweise durch 1, 2 bzw. 3 R_{b} substituiert sind; vorzugsweise ist R₃ ausgewählt aus der Gruppe bestehend aus H, =O, -NH₂, -NHC(=NH)NH₂, -CH₃, -NHCH₃, -N(CH₃)₂, -NHCH₂CH₃, -NHCH₂CH₂CH₃, -NHCH₂CH₂OH, - NHCH₂CH₂NH₂, -OCH₃ 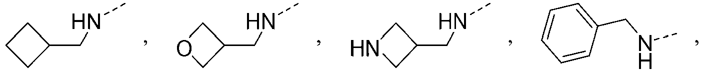 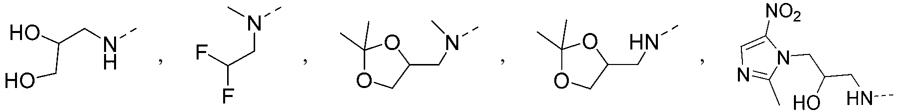 und

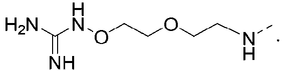

8. Die Verbindung, das Stereoisomer oder das pharmazeutisch akzeptable Salz davon von Anspruch 1, wobei R₄ ausgewählt ist aus der Gruppe bestehend aus H, -CH₂CH₂F

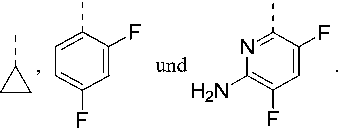

9. Die Verbindung, das Stereoisomer oder das pharmazeutisch akzeptable Salz davon von Anspruch 1, wobei R₅ und R₆ unabhängig voneinander aus der Gruppe ausgewählt sind, die aus H und F besteht.

10. Die Verbindung, das Stereoisomer oder das pharmazeutisch akzeptable Salz davon von Anspruch 1, wobei R₇, R₈ und R₉ jeweils unabhängig voneinander H sind.

11. Die Verbindung, das Stereoisomer oder das pharmazeutisch akzeptable Salz davon von Anspruch 1, wobei R₁ und R₃ zusammen mit den Atomen, an die sie gebunden sind, 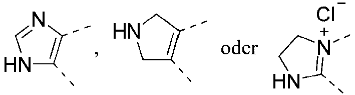 bilden, wobei 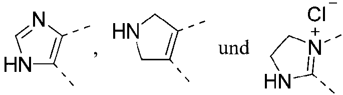 unabhängig voneinander wahlweise durch 1, 2, 3 bzw. 4 R_{c}, substituiert sind; vorzugsweise bilden R₁ und R₃ zusammen mit den Atomen, an den sie gebunden sind

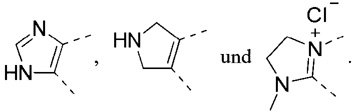

12. Die Verbindung, das Stereoisomer oder das pharmazeutisch akzeptable Salz davon von Anspruch 1, wobei die Verbindung eine Struktur hat, die durch die Formel (II-2) dargestellt wird: 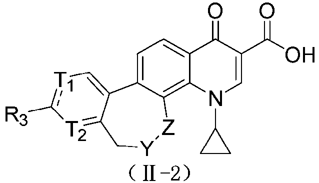 wobei X, Y, Z, T₁, T₂, R₃, R₅ und R₆ wie in Anspruch 1 definiert sind.

13. Die Verbindung, das Stereoisomer oder das pharmazeutisch akzeptable Salz davon nach Anspruch 1, wobei die Verbindung ausgewählt ist aus:

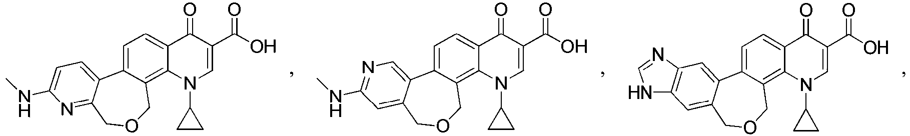

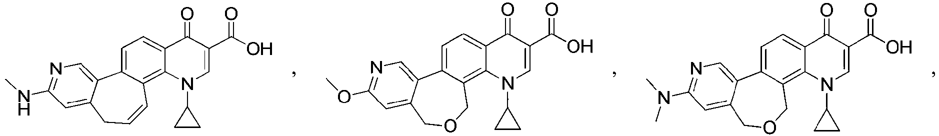

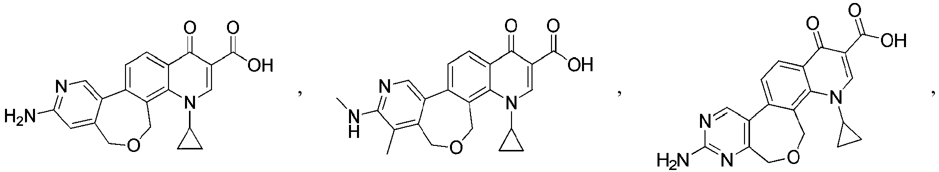

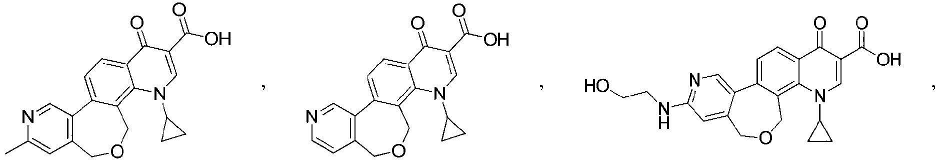

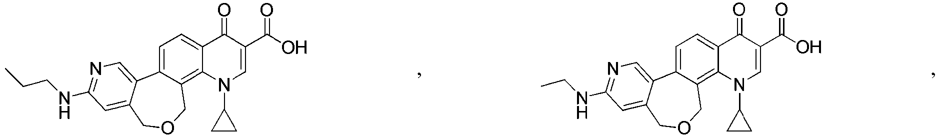

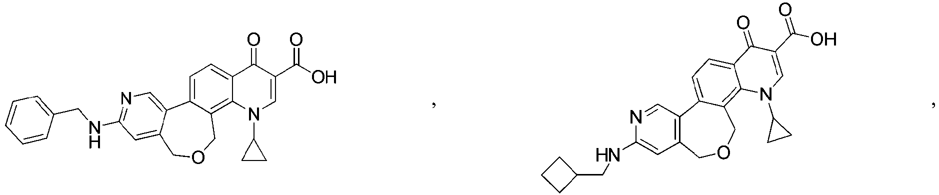

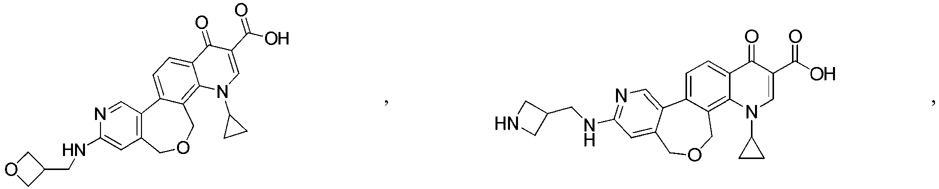

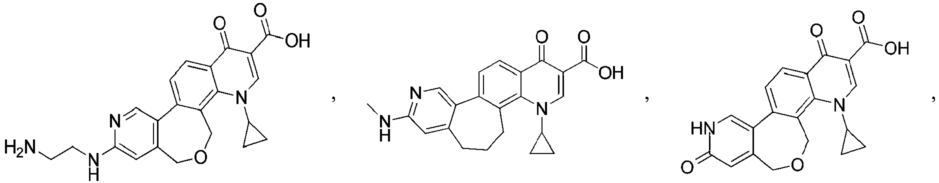

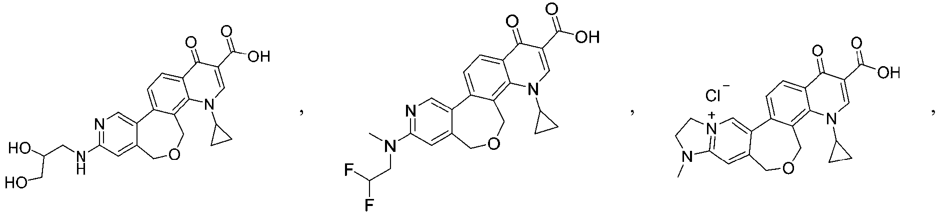

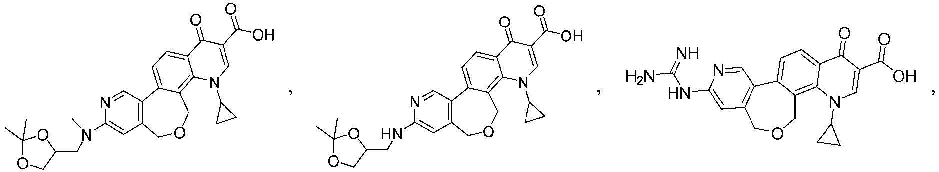

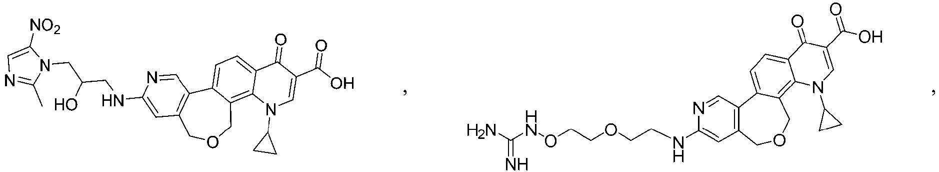

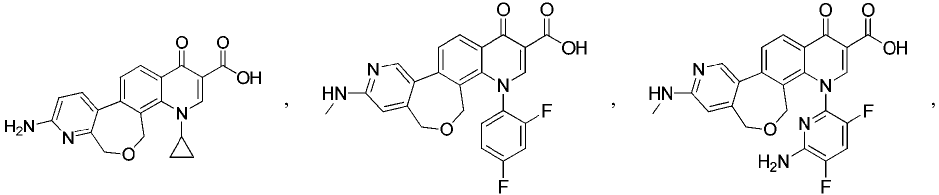

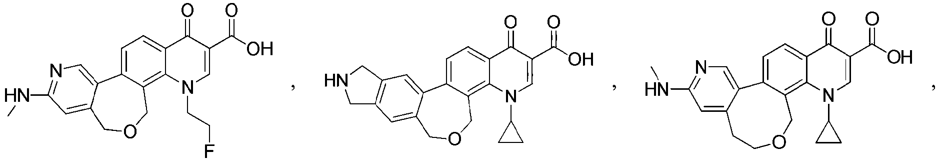

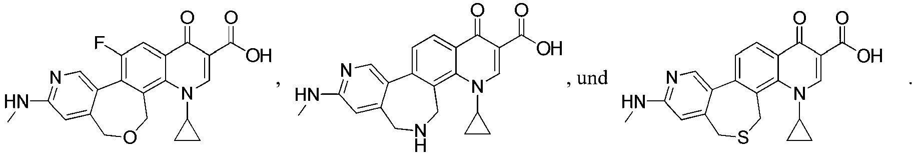

14. Die Verbindung, das Stereoisomer oder das pharmazeutisch akzeptable Salz davon nach Anspruch 13, wobei die Verbindung ausgewählt ist aus: 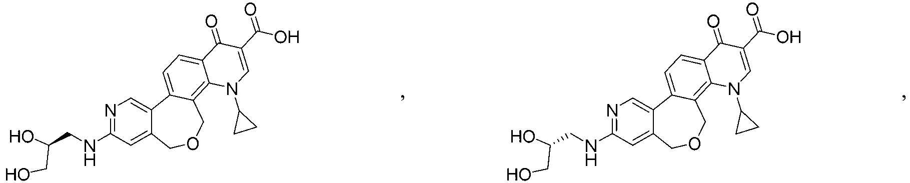 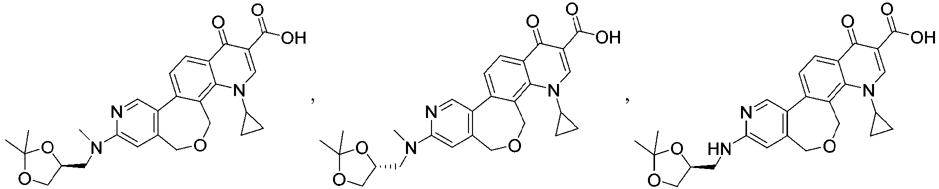 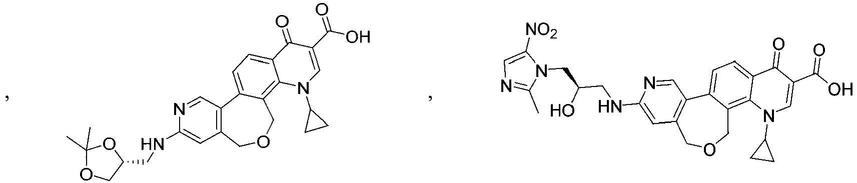 und

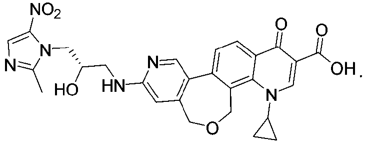

15. Die Verbindung, das Stereoisomer oder das pharmazeutisch akzeptable Salz davon nach einem der Ansprüche 1 bis 14 zur Verwendung als antibakterielle und entzündungshemmende Arzneimittel, vorzugsweise zur Verwendung bei der Behandlung von Akne.

## Revendications

1. Un composé représenté par la formule (II), un stéréoisomère, ou un sel pharmaceutiquement acceptable de celui-ci, 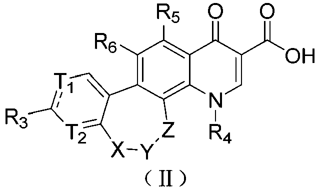 dans lequel, 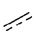
est choisi parmi une liaison simple et une liaison double ;
T₁ est choisi dans le groupe constitué par N, NH, CR₁ et N⁺R₁(A⁻) ;
T₂ est choisi dans le groupe constitué par N et CR₂ ;
A⁻ est choisi dans le groupe constitué par F⁻, Cl⁻, Br⁻, I⁻, OH⁻ et HCO₃⁻ ;
X est choisi dans le groupe constitué par -C(R₇R₈)- et -C(R₇R₈)-C(R₇R₈)- ;
Y est choisi dans le groupe constitué par -O-, -S-, -NH- et -C(R₇R₈)- ;
Z est -C(R₇R₈)- ;
alternativement, -Y-Z- est choisi dans le groupe constitué par -C(R₉)=C(R₉)- ;
R₁ est choisi dans le groupe constitué par H, F, Cl, Br, I, -OH, -NH₂, -CN et -CH₃ ;
R₂ est choisi dans le groupe constitué par H, F, Cl, Br, I, -OH, -NH₂, -CN et alkyle en C₁₋₃, dans lequel l'alkyle en C₁₋₃ est facultativement substitué par 1, 2 ou 3 Rₐ ;
R₃ est choisi dans le groupe constitué par H, F, Cl, Br, I, =O, -OH, -NH₂, -CN, -NHC(=NH)NH₂, alkyle en C₁₋₃, alkylamino en C₁₋₃ et alcoxy en C₁₋₃, dans lequel l'alkyle en C₁₋₃, l'alkylamino en C₁₋₃ et l'alcoxy en C₁₋₃ sont indépendamment facultativement substitués par 1, 2 ou 3 R_{b}, respectivement ;
en variante, R₁ et R₃ ensemble avec les atomes auxquels ils sont attachés forment un hétérocycloalkyle à 5-6 chaînons, un hétérocycloalcényle à 5-6 chaînons ou un hétéroaryle à 5-6 chaînons, dans lequel l'hétérocycloalkyle à 5-6 chaînons, l'hétérocycloalcényle à 5-6 chaînons et l'hétéroaryle à 5-6 chaînons sont indépendamment facultativement substitués par 1, 2, 3 ou 4 R_{c}, respectivement ;
R₄ est choisi dans le groupe constitué par H, alkyle en C₁₋₃, cycloalkyle en C₃₋₆, phényle et hétéroaryle à 5-6 chaînons, dans lequel l'alkyle en C₁₋₃, le cycloalkyle en C₃₋₆, le phényle et l'hétéroaryle à 5-6 chaînons sont indépendamment facultativement substitués par 1, 2, 3 ou 4 R_{d}, respectivement ;
R₅ est choisi dans le groupe constitué par H, F, Cl, Br, I, -OH, -NH₂ et -CN ;
R₆ est choisi dans le groupe constitué par H, F, Cl, Br, I, -OH, -NH₂ et -CN ;
R₇, R₈ et R₉ sont chacun indépendamment choisis dans le groupe constitué par H, F, Cl, Br, I, -OH, -NH₂ et - CN, respectivement ;
chaque Rₐ est indépendamment choisi dans le groupe constitué par F, Cl, Br, I, -OH, -NH₂ et -CN, respectivement ;
chaque R_{b} est indépendamment choisi dans le groupe constitué par F, Cl, Br, I, -OH, -NH₂, -CN, alcoxy en C₁₋₃, cycloalkyle en C₃₋₆, hétérocycloalkyle à 3-6 chaînons, hétéroaryle à 5-6 chaînons et phényle, respectivement, dans lequel l'alcoxy en C₁₋₃, le cycloalkyle en C₃₋₆, l'hétérocycloalkyle à 3-6 chaînons, l'hétéroaryle à 5-6 chaînons et le phényle sont indépendamment facultativement substitués par 1, 2, 3 ou 4 R, respectivement ;
chaque R_{c} est indépendamment choisi dans le groupe constitué par F, Cl, Br, I, -OH, -NH₂, -CN et -CH₃, respectivement ;
chaque est indépendamment choisi dans le groupe constitué par F, Cl, Br, I, -OH, -NH₂ et -CN, respectivement ;
chaque R est indépendamment choisi dans le groupe constitué par F, Cl, Br, I, -OH, -NH₂, -NO₂, -CN, - ONHC(=NH)NH₂ et alkyle en C₁₋₃, respectivement ; et
« hétéro » dans l'hétérocycloalkyle à 5-6 chaînons, l'hétérocycloalkyle à 3-6 chaînons, l'hétérocycloalcényle à 5-6 chaînons et l'hétéroaryle à 5-6 chaînons signifie 1, 2, 3 ou 4 hétéroatomes ou groupes d'hétéroatomes indépendamment choisis dans le groupe constitué par O, NH, S et N, respectivement.

2. Le composé, le stéréoisomère ou le sel pharmaceutiquement acceptable de celui-ci de la revendication 1, dans lequel chaque R est indépendamment choisi dans le groupe constitué par -NO₂, -ONHC(=NH)NH₂ et -CH₃, respectivement.

3. Le composé, le stéréoisomère ou le sel pharmaceutiquement acceptable de celui-ci de la revendication 1, dans lequel chaque R_{b} est indépendamment choisi dans le groupe constitué par F, Cl, Br, -OH, -NH₂, -OCH₂CH₃, cyclobutyle, oxétanyle, oxacyclopentyle, azétidinyle, hétéroaryle à 5 chaînons et phényle, dans lequel le - OCH₂CH₃, le cyclobutyle, l'oxétanyle, l'oxacyclopentyle, l'azétidinyle, l'hétéroaryle à 5 chaînons et le phényle sont indépendamment facultativement substitués par 1, 2, 3 ou 4 R, respectivement ; de préférence, chaque R_{b} est indépendamment choisi dans le groupe constitué par F, -OH, -NH₂,

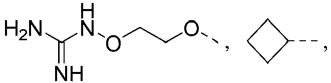

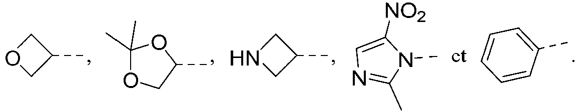

4. Le composé, le stéréoisomère ou le sel pharmaceutiquement acceptable de celui-ci de la revendication 1, dans lequel chaque R_{c} est indépendamment -CH₃, respectivement.

5. Le composé, le stéréoisomère ou le sel pharmaceutiquement acceptable de celui-ci de la revendication 1, dans lequel chaque est indépendamment choisi dans le groupe constitué par F, Cl, Br et -NH₂, respectivement.

6. Le composé, le stéréoisomère ou le sel pharmaceutiquement acceptable de celui-ci de la revendication 1, dans lequel R₁ est H ;
et/ou, R₂ est choisi dans le groupe constitué par H et -CH₃.

7. Le composé, le stéréoisomère ou le sel pharmaceutiquement acceptable de celui-ci selon la revendication 1, dans lequel R₃ est choisi dans le groupe constitué par H, F, Cl, Br, =O, -OH, -NH₂, -CN, -NHC(=NH)NH₂, - CH₃, -NHCH₃, -N(CH₃)₂, -NHCH₂CH₃, -NHCH₂CH₂CH₃, -N(CH₃)CH₂CH₃ et -OCH₃, où facultativement - NHC(=NH)NH₂, -CH₃, -NHCH₃, -N(CH₃)₂, -NHCH₂CH₃, -NHCH₂CH₂CH₃, -N(CH₃)CH₂CH₃ et -OCH₃ sont indépendamment substitués par 1, 2, ou 3 R_{b}, respectivement; de préférence, R₃ est choisi dans le groupe constitué de H, =O, -NH₂, -NHC(=NH)NH₂, -CH₃, -NHCH₃, -N(CH₃)₂, -NHCH₂CH₃, -NHCH₂CH₂CH₃, -NHCH₂CH₂OH, -NHCH₂CH₂NH₂, -OCH₃, 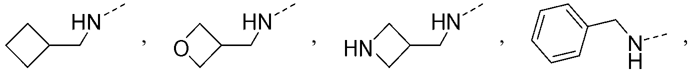 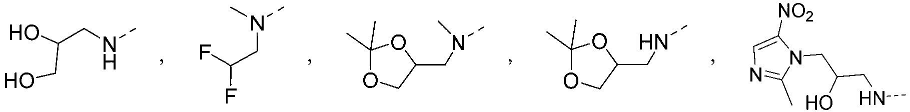 et

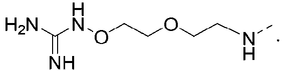

8. Le composé, le stéréoisomère ou le sel pharmaceutiquement acceptable de celui-ci de la revendication 1, dans lequel R₄ est choisi dans le groupe constitué par H, -CH₂CH₂F,

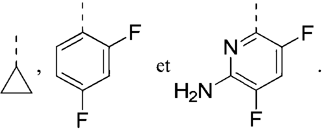

9. Le composé, le stéréoisomère ou le sel pharmaceutiquement acceptable de celui-ci de la revendication 1, dans lequel R₅ et R₆ sont indépendamment choisis dans le groupe constitué par H et F.

10. Le composé, le stéréoisomère ou le sel pharmaceutiquement acceptable de celui-ci de la revendication 1, dans lequel R₇, R₈ et R₉ sont chacun indépendamment H, respectivement.

11. Le composé, le stéréoisomère ou le sel pharmaceutiquement acceptable de celui-ci de la revendication 1, dans lequel R₁ et R₃ ensemble avec les atomes auxquels ils sont attachés forment 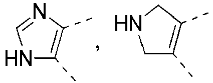 ou 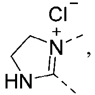 dans lequel 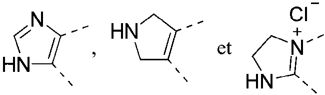 sont indépendamment facultativement substitués par 1, 2, 3 ou 4 R_{c}, respectivement ; de préférence, R₁ et R₃ ensemble avec les atomes auxquels ils sont attachés forment

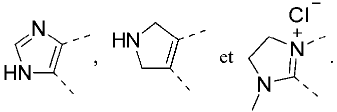

12. Le composé, le stéréoisomère ou le sel pharmaceutiquement acceptable de celui-ci de la revendication 1, dans lequel le composé a une structure représentée par la formule (II-2) : 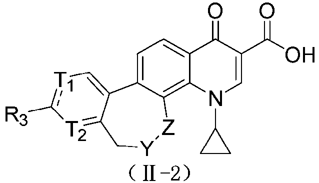 dans laquelle X, Y, Z, T₁, T₂, R₃, R₅ et R₆ sont tels que définis dans la revendication 1.

13. Le composé, le stéréoisomère ou le sel pharmaceutiquement acceptable de celui-ci selon la revendication 1, dans lequel le composé est choisi parmi :

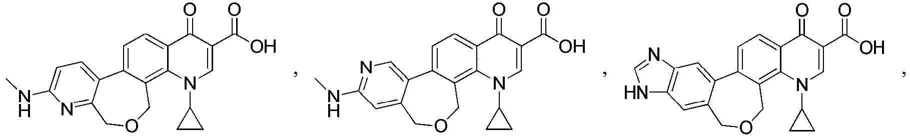

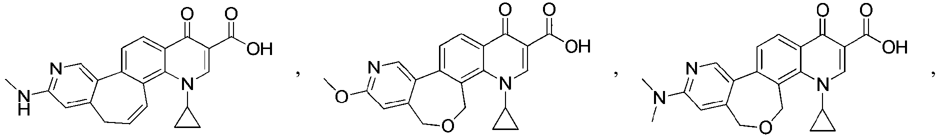

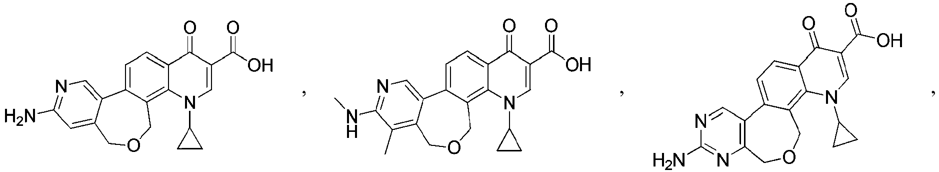

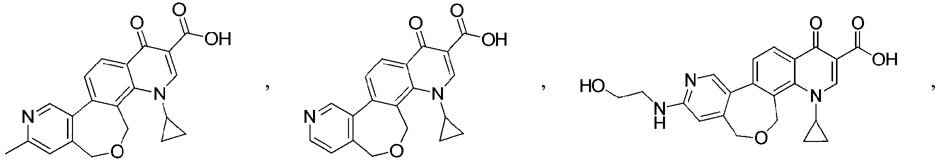

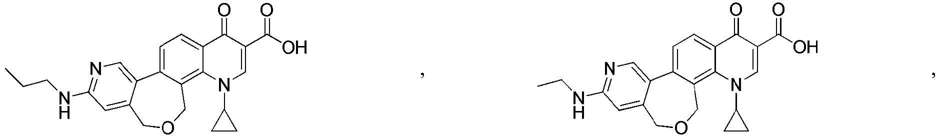

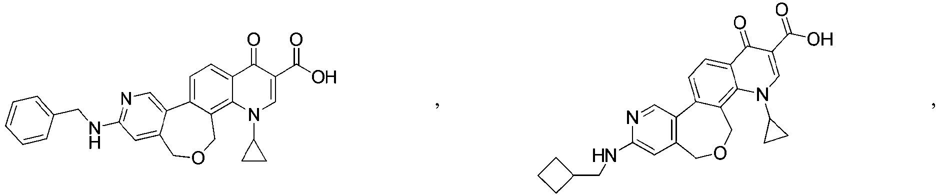

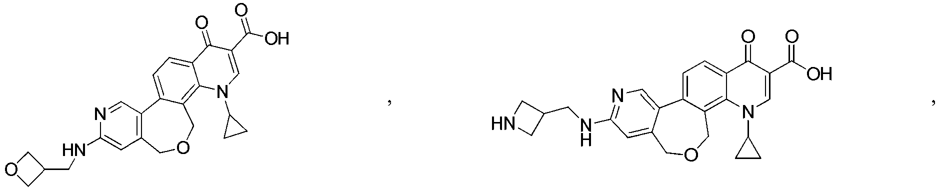

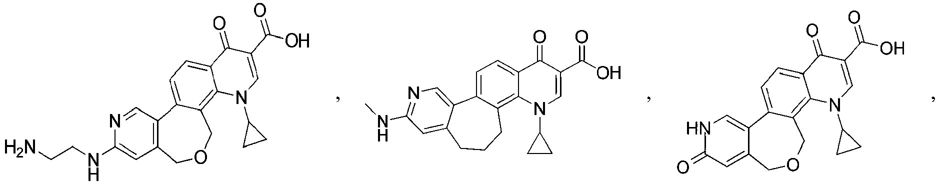

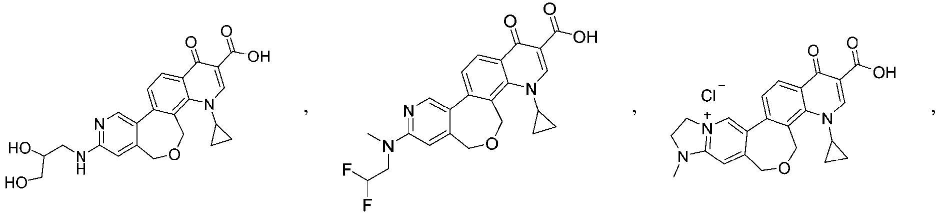

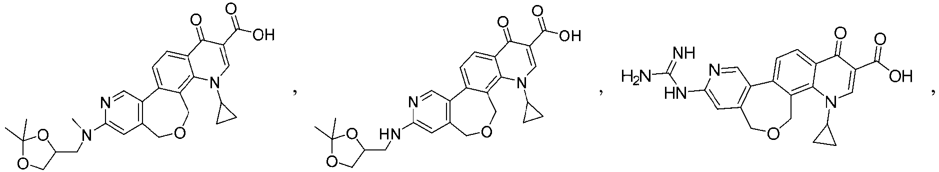

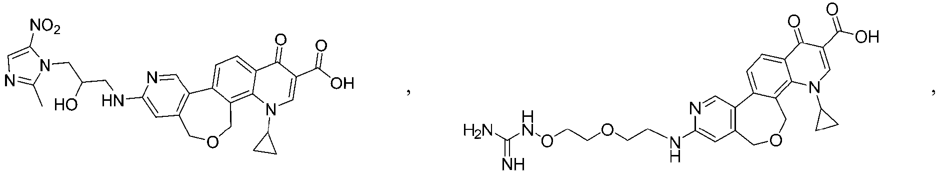

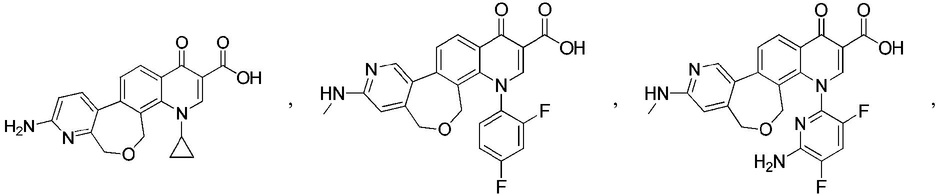

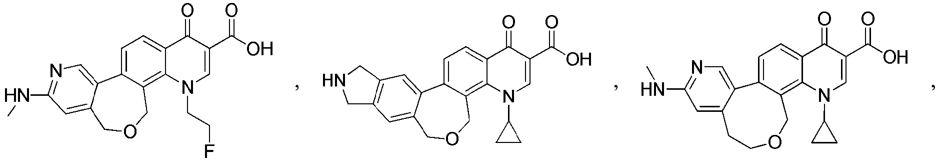

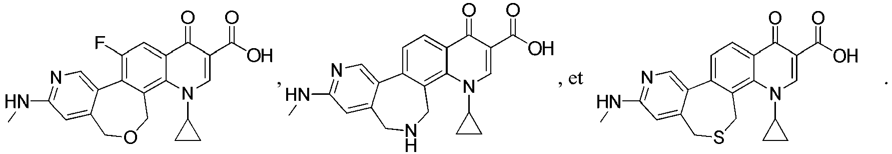

14. Le composé, le stéréoisomère ou le sel pharmaceutiquement acceptable de celui-ci selon la revendication 13, dans lequel le composé est choisi parmi :

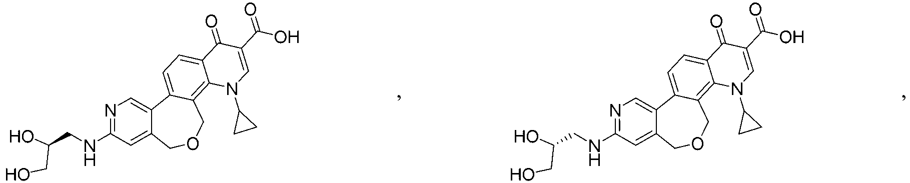

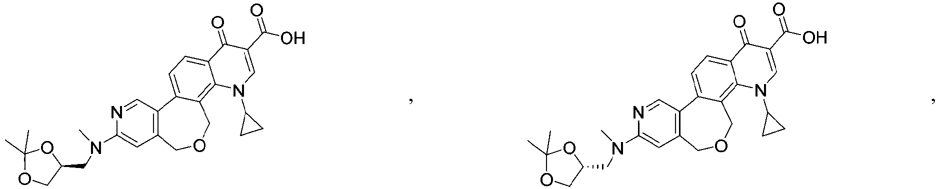

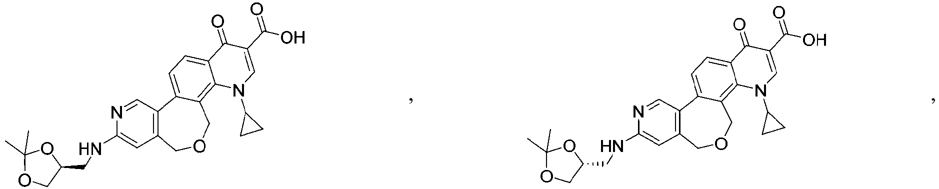

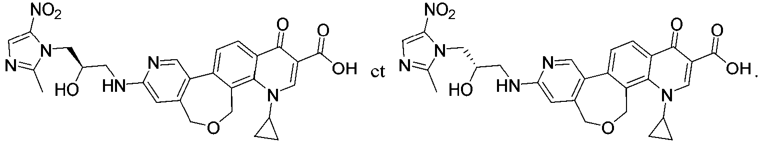

15. Le composé, le stéréoisomère ou le sel pharmaceutiquement acceptable de celui-ci de l'une quelconque des revendications 1 à 14 pour une utilisation en tant que médicaments antibactériens et anti-inflammatoires, de préférence, pour une utilisation dans le traitement de l'acné.
